# EUROPEAN PATENT APPLICATION

(11) **EP 4 620 482 A1**
(43) Date of publication of application: **24.09.2025**
(21) Application number: 24164184.4
(22) Date of filing: 18.03.2024
(51) Int. Cl.: A61K 39/08, A61P 35/00, C07K 14/00

(54) **FLAGELLIN-RELATED PEPTIDES AND USES THEREOF**

(71) Applicant: Istituto Europeo di Oncologia S.r.l., 20121 Milano (MI) (IT); Istituto Nazionale Tumori IRCCS - Fondazione G. Pascale, 80131 Napoli (NA) (IT)
(72) Inventor: NEZI, Luigi, 20121 Istituto Europeo di Oncologia S.r.l., Via Filodrammatici 10 (IT); MANZO, Teresa, 20121 Istituto Europeo di Oncologia S.r.l., Via Filodrammatici 10 (IT); MACANDOG, Angeli Dominique, 20121 Istituto Europeo di Oncologia S.r.l., Via Filodrammatici 10 (IT); ASCIERTO, Paolo Antonio, 80131 Istituto Nazionale Tumori IRCCS - Fondazione G. Pascale, Via Mariano Semmola (IT); BUONAGURO, Luigi, 80131 Istituto Nazionale Tumori IRCCS - Fondazione G. Pascale, Via Mariano Semmola (IT)
(74) Representative: Pace Napoleone, Maria

(57) **Abstract**

The present invention relates to peptides coded by bacteria flagellin-related genes and to their use in tumor vaccination therapies. In particular, it relates to a composition comprising at least one peptide for use for the prevention and/or treatment of a cancer, wherein said at least one peptide is coded by a flagellin-related gene.

## Description

### TECHNICAL FIELD

The present invention relates to peptides and their use in cancer immunotherapy. In particular, the present invention relates to peptides coded by flagellin-related genes of Lachnospiraceae and to their use in tumor vaccination therapies.

### BACKGROUND

The introduction of immune checkpoint inhibitor (ICI) therapy in the last decade set a precedent for melanoma treatment, significantly improving patient outcome^{1,2} in an otherwise therapy resistant cancer type².

However, a proportion of melanoma remains non-responsive to ICI (nR).

In this regard, the influence of the gut microbiome has been widely reported, with independent studies demonstrating that the gut microbiome of patients with melanoma responding to ICI (R) is compositionally and functionally different from those with nR³⁻⁷.

More importantly, recent clinical studies show that fecal microbiota transplant (FMT) from either R or healthy donors can improve the response in a subset of patients with ICI-refractory melanoma⁸⁻¹⁰, suggesting that a targeted modulation of the gut microbiome has still unexplored therapeutic potential.

Consolidation of these microbiome studies could contribute to rationally design therapeutically active consortia of bacteria, but results of a large-scale meta-analysis point to high variability across cohorts as a major limitation^{7,11}.

Recent studies have shown the success of ICI on mismatch repair (MMR)-deficient (dMMR) patients that is linked, at least in part, to a higher immune "visibility" of their tumors¹¹⁻¹⁶. These results led to trials combining immunotherapy with a personalized cancer vaccine that encodes for patient-specific tumor neoantigens (NCT03897881)¹⁷.

It is therefore still highly desired a cancer vaccine which can improve the outcome of cancer therapies, such as ICI, by boosting immune response against the cancer. In particular, it is desired a vaccine which boosts immune response against cancer but not against endogenous antigens.

### SUMMARY OF THE INVENTION

The present invention relates to the identification of peptides derived from flagellin-related proteins of Lachnospiraceae which can be useful to enhance anti-tumor immunity, in particular in patients undergoing immunotherapy. Indeed, it was found that patients with melanoma responsive to ICI can specifically mount a CD8⁺ T cell-mediated response directed against tumor-mimicking, flagellin-related peptides. The use of non-endogenous peptides to stimulate the immune system against a tumor is advantageous because it lowers the risk of an auto-immune response.

It is an object of the invention a composition comprising at least one peptide for use for the prevention and/or treatment of a cancer, wherein said at least one peptide is coded by at least one flagellin-related gene, said gene being selected from the group consisting of A0A0M6WP36, C0FQ31, A0A127SWS0, C0FQ36, A0A396AI63, A0A0M6WMV3, A0A0M6WD36, D4KYR7, R6VY20, A0A351R0D5, A0A0M6WBR8, A0A0M6WYS8, R6EJ07, R6VQ94, G2SZK1, C7G5T8, A5Z847, A0A174K7I7, A0A1Q6SAN9, D4KYS1, D4KW84, R6EJJ6, R6EC52, A0A395V8C5, R6EJJ3, R5SPI8, R5T8V3, D4RW24, R5T617, A0A373K755, R6X2U9, G2T3F2, G2SZJ5, D4KYS2, R6NV68, R5T127,A0A395V4R6, C4Z282, A0A395VCZ3, A0A174BIC3, R5SNW4, A0A395VAJ9, A0A174ZTA0, A0A396ABL6, A0A3E4X9M7, R6NRZ0, R5SHJ2, R5E9Z9, R7C6Q7, A0A3D5WGL2, D7GSU0, D7GSR3, D7GSR2, A0A173WCI3, A0A1C5P5U9, R6EJS1, R5WFA2, R9M386, A0A174N4P2, D7GST1, R6P646, A0A174LQE7, A0A174YXY8, D7GSS4, R7CCH1, D7GSS8, A0A1C5WBB9, A0A1C5KUA4, D4RY26, J5GJA7, R6NM46, A0A1C5RKN2, R7NFE3, A0A174YYK7, R5E067, A2V703, R5DRV5, A0A0K4GI28, A0A174MPU4, A0A376JIF4, R5DRV9, R6NJG6, A0A285PW02, C0FTT4, A0A2X1MZ58, R6VWT3, A0A174DCE4, A0A174I7J5, C4ZAM7, C4Z9R8, A0A2U2EKC6, R6EL64, A0A395UYP9, A0A2U2MF49, R6EHH0, A0A396FMF8, A0A0M6WBY2, A0A174Q4T5, B7ART7, A0A396FGM9, A0A396FKV9, R6ESB9, R5TD65, R6BIK6, C7GHZ7, A0A174AGF2, R5SHJ0, P0AEM4, A0A174YHS0 and A0A3E5AME9, wherein said peptide is at least 9 amino acids long, preferably it is between 9 and 20 amino acids, more preferably it consists of 9 amino acids.

Preferably, said flagellin-related gene is from a bacteria of Clostridia class, more preferably of the Lachnospiraceae family, even more preferably of Roseburia genere, in particular Roseburia inulinivorans.

Preferably, said peptide is coded by a flagellin-related gene selected from the group consisting of: A0A0M6WP36, C0FQ31, A0A127SWS0, C0FQ36, A0A396AI63, A0A0M6WMV3, A0A0M6WD36, D4KYR7, R6VY20, A0A351R0D5, A0A0M6WBR8, A0A0M6WYS8, R6EJ07 and R6VQ94, more preferably selected from the group consisting of A0A0M6WD36, A0A0M6WMV3, A0A0M6WYS8, A0A351R0D5, R6EJ07, R6VY20, A0A396AI63, A0A0M6WP36 and R6VQ94. Said numbers correspond to identification numbers of respective genes in the UniProt or in the UniParc database, January 2024 release. The name of the genes may vary in subsequent or different databases releases, the skilled person is able to identify the corresponding name of the genes in different database releases according to common general knowledge in the field.

More preferably, said at least one peptide is selected from the group consisting of peptides comprising or consisting of a sequence of any of SEQ ID Ns. 1-143, more preferably selected from the group consisting of peptides comprising or consisting of a sequence of SEQ ID Ns. 1, 2, 3, 4, 5, 6, 7, 8, 9, or 10.

Preferably, the composition comprises at least two, three, four, five, six, seven, eight, nine or ten, peptides selected from the group consisting of peptides comprising or consisting of a sequence of any of SEQ ID Ns. 1-143, more preferably selected from the group consisting of peptides comprising or consisting of a sequence of SEQ ID Ns. 1, 2, 3, 4, 5, 6, 7, 8, 9, or 10.

Preferably the composition comprises at least one peptide comprising or consisting of a sequence selected from the group consisting of:
- KMSYEDIEL (SEQ ID N. 1),
- MPKDGAAFI (SEQ ID N.2),
- ALNETSAIL (SEQ ID N.3),
- ILAQAGQSM (SEQ ID N.4),
- II,AQAGTSM (SEQ ID N.5),
- NEFNADLLM (SEQ ID N.6),
- TEFNGQKLL (SEQ ID N.7),
- SVRGRLGAF (SEQ ID N.8),
- FPELKHFTM (SEQ ID N.9), and
- GLDALNNLL (SEQ ID N. 10)
or variants thereof,
preferably in combination with at least one peptide of SEQ ID Ns. 1-143.

Said peptides of SEQ ID Ns. 1-143 and combinations thereof are also objects of the invention. In particular, it is an object of the invention a peptide comprising or consisting of a sequence of any of SEQ ID Ns. 1-143.

In particular, it is an object of the invention a composition comprising at least one peptide coded by a flagellin-related gene selected from the group consisting of:
A0A0M6WP36, A0A127SWS0, C0FQ36, A0A396AI63, A0A0M6WMV3, A0A0M6WD36, D4KYR7, R6VY20, A0A351R0D5, A0A0M6WBR8, A0A0M6WYS8, R6EJ07 and R6VQ94.

It is also an object of the invention a composition comprising at least one peptide comprising or consisting of a sequence of SEQ ID Ns. 1-143, preferably at least one peptide selected from the group consisting of peptides comprising or consisting of a sequence of SEQ ID Ns. 1, 2, 3, 4, 5, 6, 7, 8, 9, or 10. More preferably, it comprises at least two, three, four, five, six, seven, eight, nine or ten peptides comprising or consisting of a sequence of SEQ ID Ns. 1, 2, 3, 4, 5, 6, 7, 8, 9, or 10, optionally in combination with any other peptide of SEQ ID Ns. 1-143. In an embodiment, said composition comprises a peptide of SEQ ID N.3, a peptide of SEQ ID N.4, a peptide of SEQ ID N.5, a peptide of SEQ ID N.6, a peptide of SEQ ID N.7, a peptide of SEQ ID N.8 and a peptide of SEQ ID N.9. In another embodiment, said composition comprises a peptide of SEQ ID N. 1, a peptide of SEQ ID N.2 and a peptide of SEQ ID N. 10.

Preferably, said flagellin-related gene is from a bacterium of Chlostridia class, more preferably of the Lachnospiraceae family, even more preferably of Roseburia genere, in particular Roseburia inulinivorans.

Said cancer may be characterized by increased expression of any known cancer antigen, such as any cancer antigen which can be found in the database Cancer Antigenic Peptide Database (https://caped.icp.ucl.ac.be/).

Preferably, the cancer is characterized by increased expression of at least one of the following antigens: MAGE-A1, N-ras, CEA, alpha-actinin-4, NY-ESO-1/LAGE-2, MAGE-A3, N-ras, PAP, Preferentially Expressed Antigen in Melanoma (PRAME), Melanoma Antigen Recognized by T Cells 1 (MART-1/Melan-A), and secernin 1 (SCRN1).

Preferably the cancer is selected from melanoma, lung cancers, bladder cancers, esophageal and head and neck cancers, glioblastoma, sarcomas, colorectal cancers, gastric cancers, skin cancers, cutaneous squamous cell carcinomas, thyroid cancers, undifferentiated pleomorphic sarcoma/myxofibrosarcoma, non-small cell lung cancer, small cell lung cancer, multiple myeloma, cerebral cancers, renal cancers, leukemias, lymphomas, hematopoietic and lymphoid cancers, diffuse large B-cell lymphoma, hepatocellular carcinomas, breast cancers, pancreatic cancers, and gynecology cancers.

A further object of the invention is a pharmaceutical composition comprising the peptides above defined and a pharmaceutically acceptable excipient and/or vehicle.

It is a further object of the invention a vaccine or immunogenic composition comprising the peptides as above defined and a pharmaceutically acceptable vehicle or excipient and preferably an adjuvant.

Preferably, said pharmaceutical composition or vaccine or immunogenic composition is for use in the treatment and/or prevention of cancer, preferably for use in the treatment and/or prevention of cancer characterized by increased expression of at least one of the following antigens: MAGE-A1, N-ras, CEA, alpha-actinin-4, NY-ESO-1/LAGE-2, MAGE-A3, N-ras, PAP, PRAME, MART1/Melan-A and SCRN1. Even more preferably, said pharmaceutical composition or vaccine or immunogenic composition is for use in the treatment and/or prevention of a cancer selected from the group consisting of melanoma, lung cancers, bladder cancers, esophageal and head and neck cancers, glioblastoma, sarcomas, colorectal cancers, gastric cancers, skin cancers, cutaneous squamous cell carcinomas, thyroid cancers, undifferentiated pleomorphic sarcoma/myxofibrosarcoma, non-small cell lung cancer, small cell lung cancer, multiple myeloma, cerebral cancers, renal cancers, leukemias, lymphomas, hematopoietic and lymphoid cancers, diffuse large B-cell lymphoma, hepatocellular carcinomas, breast cancers, pancreatic cancers, and gynecology cancers.

In an embodiment, said pharmaceutical composition may comprise more than one composition according to the invention, i.e. it may comprise two or more compositions according to the invention, each one comprising at least one peptide of SEQ ID Ns 1-143.

In an embodiment, the peptide according to the invention is able to bind to a MHC, in particular of class I. As used herein, MHC of class I indicates HLA of class I, preferably HLA-A and/or HLA-B. Said peptide may be able to bind any MHC I, in particular a HLA selected from the group consisting of HLA-A01:01, HLA-A02:01, HLA-A03:01, HLA-A24:02, HLA-A26:01, HLA-B07:02, HLA-B08:01, HLA-B27:05, HLA-B39:01, HLA-B40:01, HLA-B58:01, and HLA-B15:01. Preferably, said MHC I is chosen from the group consisting of HLA-A*02:01, HLA-B*07:02, HLA-B*15:01, HLA-B*40:01 and HLA-B*08:01. Preferably, said peptide binds a MHC class I HLA allele with a binding affinity lower than or equal to 100 nM.

In some embodiments, said peptide binds to the same MHC-I of a tumor-associated antigen (TAA) or an epitope thereof. Preferably, said TAA is selected from MAGE-A1, N-ras, CEA, alpha-actinin-4, NY-ESO-1/LAGE-2, MAGE-A3, N-ras, PAP, PRAME, MART1/Melan-A and SCRN1.

It is also an object of the invention a composition comprising at least one peptide as above defined for use as a vaccine against cancer.

It is a further object of the invention a dietary supplement or replacement comprising the peptides as above defined or combinations thereof.

It is a further object of the invention the non therapeutic use of the dietary supplement as a nutraceutic. It is also within the scope of the invention said dietary supplement or replacement for use for the prevention and/or treatment of a cancer and/or as a vaccine against cancer.

It is also an object of the invention a composition comprising at least one peptide as above defined for use for increasing effectiveness of an immune checkpoint inhibitor wherein said peptide is administered to a subject in need thereof before, simultaneously or after said immune checkpoint inhibitor. "Increasing effectiveness" means that the ICI is more effective than when administered without the peptide(s).

Peptides can be administered in alternative forms, such as for example nucleic acids coding for said peptides, or vectors comprising said nucleic acids or directly to cells, for example antigen presenting cells (APC), which express the peptides.

According to the invention, an expression vector that induces the expression of a peptide comprises a nucleic acid, DNA or RNA, coding for the peptide. The vector may be a RNA vector, a DNA vector, a viral vector or a bacterial vector.

Furthermore, according to the present invention, the peptides can advantageously be used to stimulate T cells ex vivo and then re-infuse said T cells in the patient either after the administration of a further therapeutic agent or in the absence of any specific pre-treatment.

The invention further relates to an engineered cell comprising a recombinant protein, or a polynucleotide encoding a recombinant protein, preferably said recombinant protein being a recombinant receptor, more preferably a receptor expressed on the surface of the immune cell receptor preferably, wherein the recombinant receptor specifically binds to at least one peptide of the composition as described above, preferably wherein the recombinant receptor is a recombinant T cell receptor (TCR) or a chimeric antigen receptor (CAR). Therefore, the present invention also relates to a T lymphocyte receptor capable of binding one or more peptides as previously defined in which the receptor optionally comprises one or more co-stimulatory domains. The receptor is, for example, a chimeric receptor for the antigen (CAR).

In another embodiment, the present invention relates to a polynucleotide comprising a nucleotide sequence (DNA or RNA) which codes for a peptide as described above.

In a further embodiment, the present invention relates to an expression vector comprising a nucleotide sequence as previously defined.

Such polynucleotide and expression vector can also be included in a pharmaceutical composition. Such polynucleotide and expression vector can also be advantageously used as a medicament, preferably in the treatment and/or prevention of cancer, preferably in the treatment and/or prevention of cancer characterized by increased expression of at least one of the following antigens: MAGE-A1, N-ras, CEA, alpha-actinin-4, NY-ESO-1/LAGE-2, MAGE-A3, N-ras, PAP, PRAME, MART1/Melan-A and SCRN1.

It is specified that in the context of this discussion, the expression pharmaceutical composition or composition also refers indiscriminately to an immunogenic composition or to a vaccine composition.

Preferably the composition of the invention can be administered in combination with further therapeutic regimen in a patient in need thereof. Still preferably, the composition of the invention is administered to a subject prior to, simultaneously or sequentially with other therapeutic regimens or co-agents useful for treating, and/or stabilizing cancer and/or preventing cancer relapsing (e.g. multiple drug regimens), in a therapeutically effective amount. The composition according to the present invention can be administered in the same or different composition(s) and by the same or different route(s) of administration as said co- agents.

Said other therapeutic regimens or co-agents may be selected from the group consisting of radiation therapy, chemotherapy, surgery, targeted therapy (including small molecules, peptides and monoclonal antibodies), adoptive cell therapy and anti-angiogenic therapy. Anti-angiogenic therapy is defined herein as the administration of an agent that directly or indirectly targets tumor-associated vasculature. Preferred anti-cancer agents include a chemotherapeutic agent, a targeted drug and an immunotherapeutic agent, such as an immune checkpoint modulator. Preferably, said co-agent is an immune checkpoint inhibitor (ICI). Said ICI can be selected from an antibody anti-PD-1, an antibody anti-PD-L1 and an antibody anti-CTLA-4.

In an embodiment, the composition of the invention is administered for the uses of the invention in combination with a second composition according to the invention. Accordingly, said second composition comprises at least one peptide of SEQ ID Ns 1-143, preferably it comprises at least two, three, four, five, six, seven, eight, nine or ten peptides selected from the group consisting of peptides comprising or consisting of a sequence of SEQ ID Ns. 1, 2, 3, 4, 5, 6, 7, 8, 9, or 10. In some embodiments, also more than two compositions according to the invention may be administered for the uses of the invention. The two or more compositions may comprise different peptides selected from peptides of SEQ ID Ns 1-143. The compositions of the invention may be administered to a subject simultaneously or sequentially. In an embodiment, a first composition according to the invention is administered in combination with a second composition according to the invention for use for increasing effectiveness of an immune checkpoint inhibitor.

The composition of the invention can also be used as a dietary supplement or replacement, meaning for example a food product or drinking product comprising the composition as defined herein.

### DETAILED DESCRIPTION OF THE INVENTION

### Figures

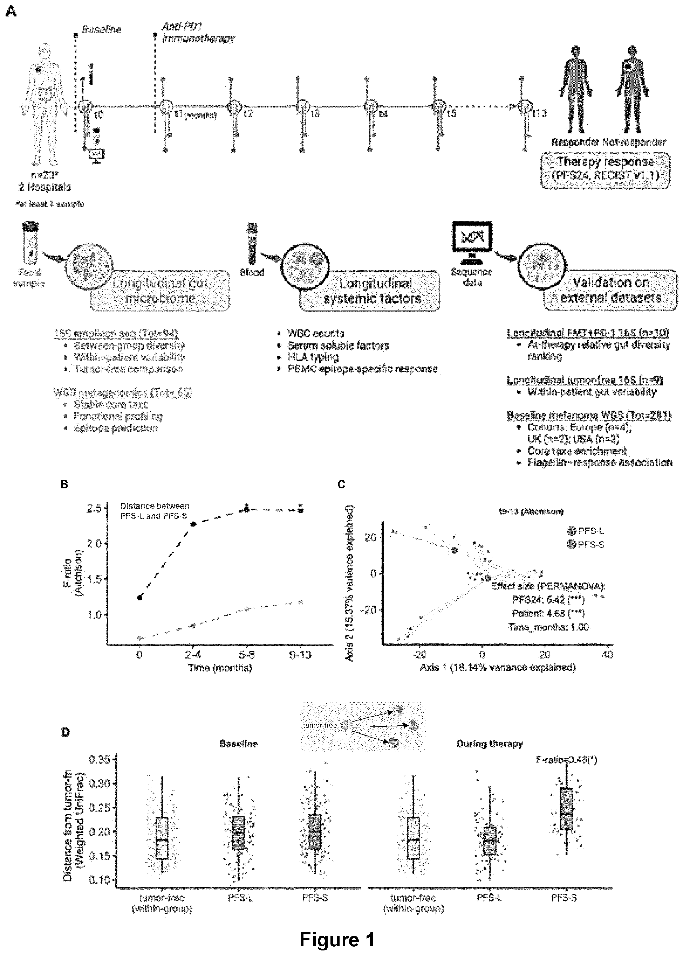

**Figure 1****. Gut microbiota differences between response groups are reinforced during therapy. (A)** Overview of study design. **(B)** Aitchison gut composition distance (PERMANOVA pseudo F-ratio) between PFS-L (PFS ≥ 24 months) and PFS-S (PFS < 24 months) groups compared across time (0, 2-4, 5-8, and 9-13 months of therapy). Asterisk indicates PERMANOVA p-adj (distance-Response) at the given timepoint group. Gray line indicates F-ratio distance by sequence batch, for reference. **(C)** Corresponding PCA plot at t9-13 using all available samples. Statistics are from multi-variate PERMANOVA (distance~Response+Patient+Timepoint) at t9-13. **(D)** Sample distances (Weighted UniFrac) from tumor-free group, using all unique patients at baseline, and all unique patients at 5-8 and 9-13 months to represent therapy. Within-group distances of tumor-free reference are shown for comparison. Statistics are from PERMANOVA (distance-group) of inter-sample distances. P-adj<0.001 (***), p-adj<0.01 (**), p-adj<0.05 (*), ns (unannotated).

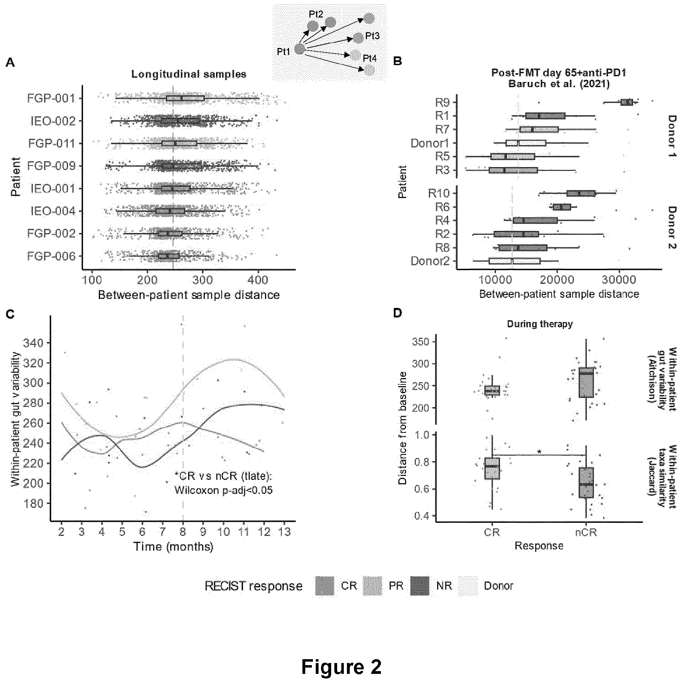

**Figure 2****. Gut variability over therapy teases out differences among RECIST responders. (A)** Ranking of patients by gut diversity variability, based on "between-patient" distances, which are the inter-sample distances excluding distances between samples from the same patient, and sorting by the median per patient. Dashed line indicates cut-off at d=246. **(B)** Ranking of between-patient distances in melanoma patients that received fecal microbiota transplant (FMT) in the indicated study. Data reflect gut composition similarity across patients at day 65 post-FMT, with three cycles of anti-PD1 immunotherapy. The plot includes between-patient measurements of the two FMT donors in the study, whose respective medians are indicated with a dashed line. **(C)** Within-patient gut variability over time in complete (CR), partial (PR), and not-responders (nR). Dashed line indicates cut-off timepoint for early/late grouping (tearly: < 8 months; date: ≥8 months). Statistics are from Wilcoxon rank sum test. **(D)** Comparison of within-patient measures in CR and nCR by gut variability (top) and taxa similarity (bottom). Gut composition distances are measured by Aitchison unless otherwise stated. FGP-006, FGP-002, IEO-004, IEO-002: CR, FGP-009, IEO002: nR, FGP-011, FGP-001: PR; R3: CR, R5, 7: PR, R1,2, 4, 6, 8-10: nR. P-adj<0.001 (***), p-adj<0.01 (**), p-adj<0.05 (*), ns (unannotated).

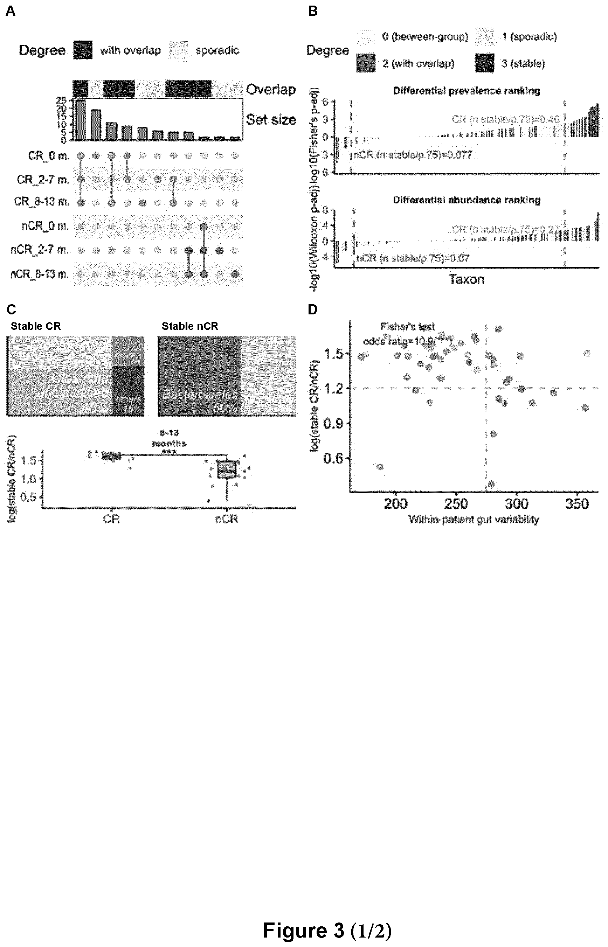

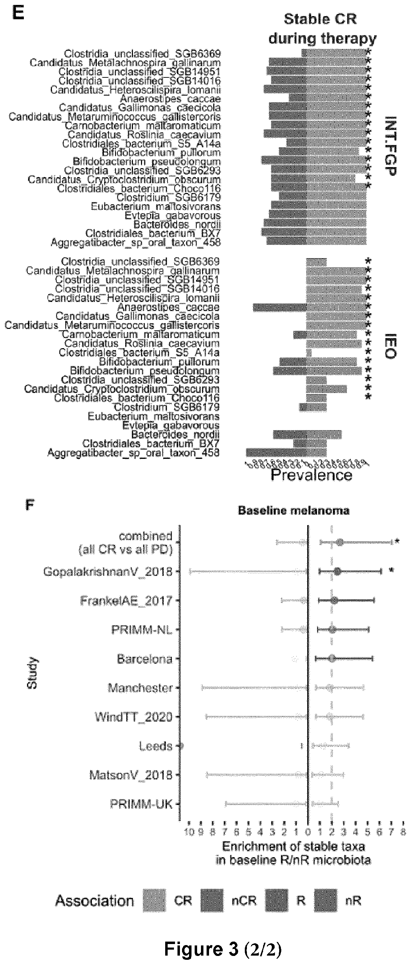

**Figure 3****. Complete responder gut comprises a stable, Clostridia-dominated community. (A)** UpSet plot showing overlaps of prevalent taxa (present in >80% of samples) found in CR and nCR groups at t0, tearly, and date. Connected-dots indicate prevalent taxa shared between timepoint groups ("with overlap"), whereas non-connected dots indicate prevalent taxa detected only in one timepoint group ("sporadic"). Plot is filtered to exclude taxa that have overlaps across CR and nCR (not group-specific). With overlap (dark gray), spurious (ligh gray). **(B)** Ranking of prevalent taxa by differential prevalence (top) and differential abundance (bottom). Gray scale indicate uncreasing degree of group-specific overlap, from not group-specific (light) to overlap across all three timepoints (dark). Annotations indicate the proportion of taxa that are stable (i.e., prevalent across timepoints) among those in the 75^{th} percentile of differential ranking. **(C)** Order-level composition of stable CR and nCR taxa (top) and the corresponding "stable taxa index" in CR and nCR (ie., log-ratio of their abundances) (bottom). Asterisks indicate significance by Wilcoxon rank sum test. Stable CR is defined as prevalent taxa that are present across t0, tearly, and date, whereas stable nCR is defined as prevalent taxa that are present in at least two timepoint groups. **(D)** Spearman correlation of within-patient gut variability with stable taxa index (log(CR/nCR)). Dashed lines indicate arbitrary cut-offs for gut stability and stable CR mark. Asterisks indicate significance by Fisher's exact test. **(E)** Prevalence of stable CR taxa across CR and nCR samples at therapy. Asterisks indicate significance in differential prevalence by Fisher's exact test (-log10(p-adj) > 1.3 (*)). **(F)** Baseline enrichment of stable CR and stable nCR taxa among R- and nR-prevalent taxa, respectively, across the indicated nine external melanoma cohorts. Dashed line indicates cut-off for enrichment (Fisher's exact test |OR|=2); lines in dark grey indicate enrichment; lines in light gray indicate no enrichment; asterisks indicate significant enrichment at unadjusted p<0.05 (Fisher's exact test); whiskers depict the interval for 95% confidence. P-adj<0.001 (***), p-adj<0.01 (**), p-adj<0.05 (*), ns (unannotated).

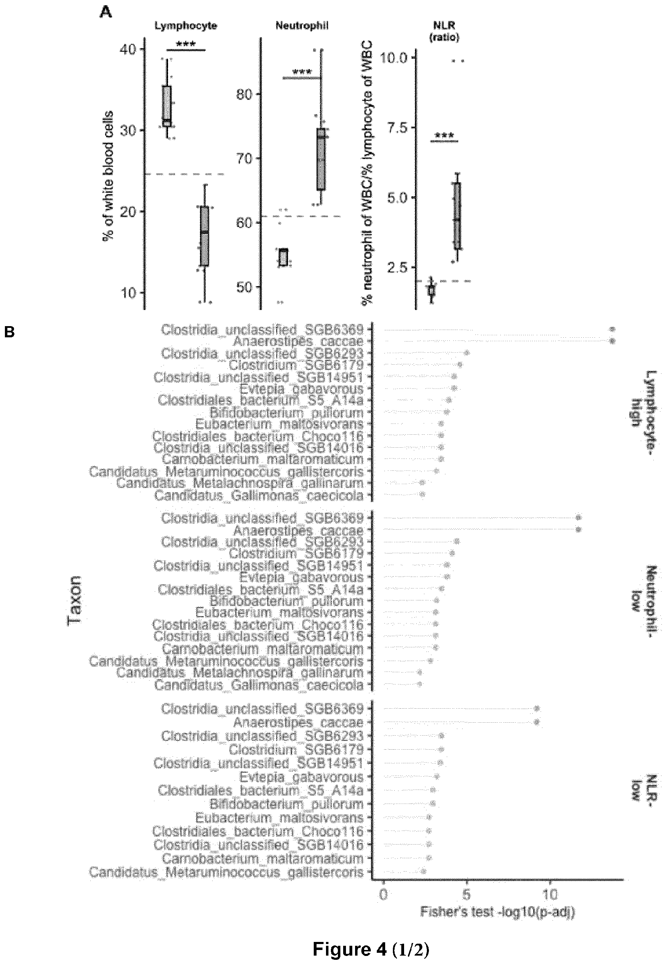

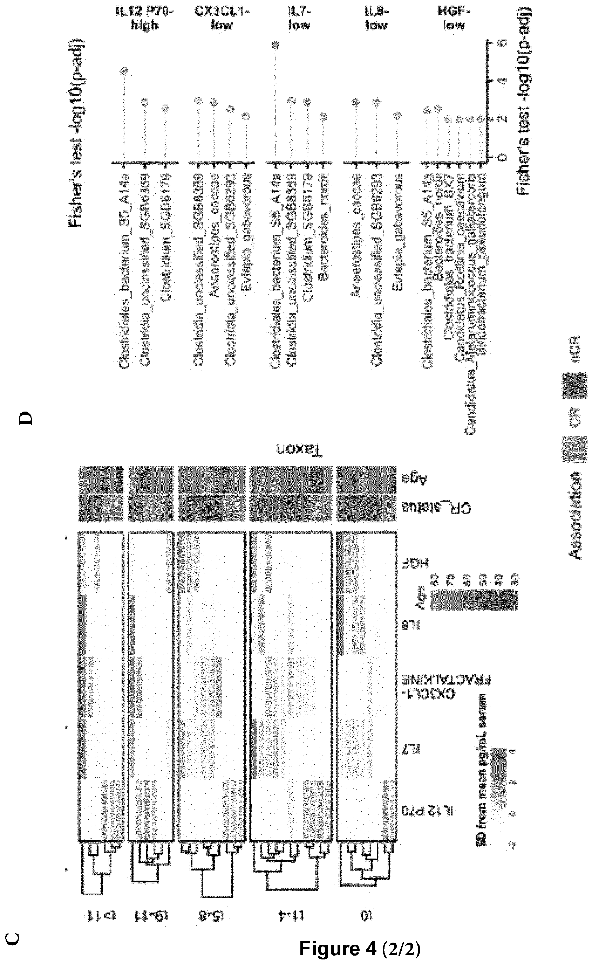

**Figure 4****. Stable taxa in complete responders associate with favorable blood and immune markers. (A)** Comparison of peripheral blood factors (percent of total) between CR and nCR samples at therapy timepoints. Dashed red lines indicate cuts-off for "high" or "low" based on distribution (low-lymphocyte <0.246; low-neutrophil < 0.61; low-NLR cut-off <2). **(B)** Fisher's test of association between stable CR taxa presence and "lymphocyte-high", "neutrophil-low", and "NLR-low" cases among therapy samples. Taxa are filtered to include only significantly associated taxa at p-adj<0.01. **(C)** Heatmap of quantities in serum (SD of mean pg/mL) of top cytokines, at 0, 1-4, 5-8, 9-11, and >11 months. **(D)** Fisher's test of association between stable CR taxa presence and "IL-12P70-high", "CX3CL1/FRACTALKINE-low", "IL7-low", "IL8-low", and "HGF-low" cases among therapy samples. Features are filtered to include only significantly associated taxa at p-adj<0.01. CR (light blue), nCR (red). P-adj<0.001 (***), p-adj<0.01 (**), p-adj<0.05 (*), ns (unannotated).

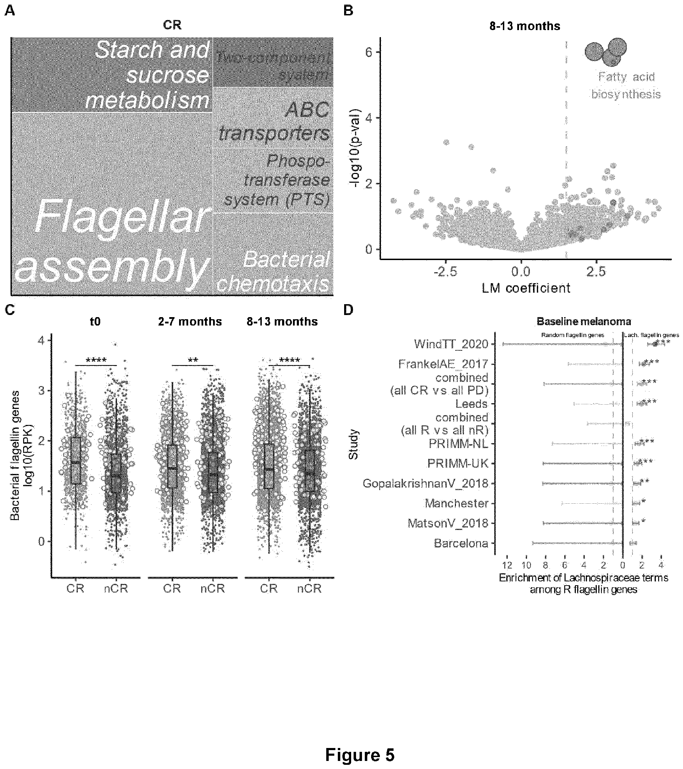

**Figure 5****. CR gut microbiome is functionally distinct and stably carries genes for fatty acid metabolism and flagellin structure. (A)** Treemap of over-represented pathways in CR at p-adj < 0.05, based on KOs associated with CR across all samples (|LM coefficient| > 1.5). Terms in white indicate stable pathways, defined as over-represented pathways appearing at t0, tearly, and tlate (p-adj < 0.05) within CR, whereas terms in gray indicate non-stable pathways. **(B)** Differentially abundant KOs in CR and nCR at late therapy timepoints. Blue points outlined in gray indicate significant KOs at p-adj < 0.005; positive coefficient refers to CR-associated terms whereas negative coefficient refers to nCR-associated terms. Dashed line indicates cut-off for testing over-represented KOs. Non-outlined blue points indicate KOs that map to over-represented pathways in (B) by the same color. **(C)** Gene family abundances (log10(RPK)) of bacterial flagellin-related terms in CR and nCR, compared at t0, tearly, and tlate. Points outlined in blue indicate top CR-associated flagellin genes by log2(fold-change). **(D)** Baseline enrichment of Lachnospiraceae-associated terms among R-associated flagellin gene families across nine melanoma cohorts; dashed lines indicate cut-off for enrichment in R (Fisher's exact test |OR|>1); lines to the left indicate enrichment of non-specific bacterial flagellin terms subsampled to the same size; lines in gray indicate no enrichment; whiskers depict the interval for 95% confidence; asterisks indicate significant enrichment at p-adj<0.05 (Fisher's exact test). CR (light blue), nCR (red). P-adj<0.001 (***), p-adj<0.01 (**), p-adj<0.05 (*), ns (unannotated).

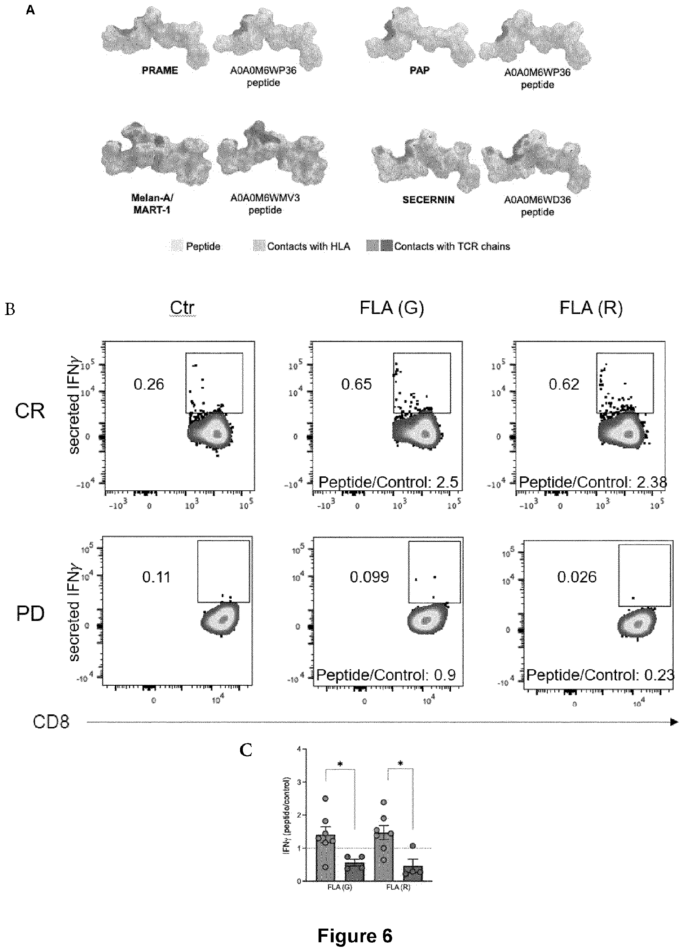

**Figure 6****. Peripheral CD8⁺ T cells of patients with melanoma responsive to ICI show higher activation upon stimulation with tumor mimicking, flagellin-related purified peptides. (A)** Structural simulation of three candidate epitopes (right of pair) predicted from CR-associated flagellin proteins, determined to have sequence and structural homology to a tumor-associated antigen (TAA) (left of pair) and strong affinity to the same HLA allele as that of the TAA (<100 nM). **(B-C)** Representative flow cytometry contour plots **(B)** and MFI measurements of IFN- γ activation markers **(C)** in the CD8⁺ T cell population of peripheral blood monocytic cells (PBMC) from patients with melanoma grouped by response to ICI (responsive=4, non-responsive= 2).

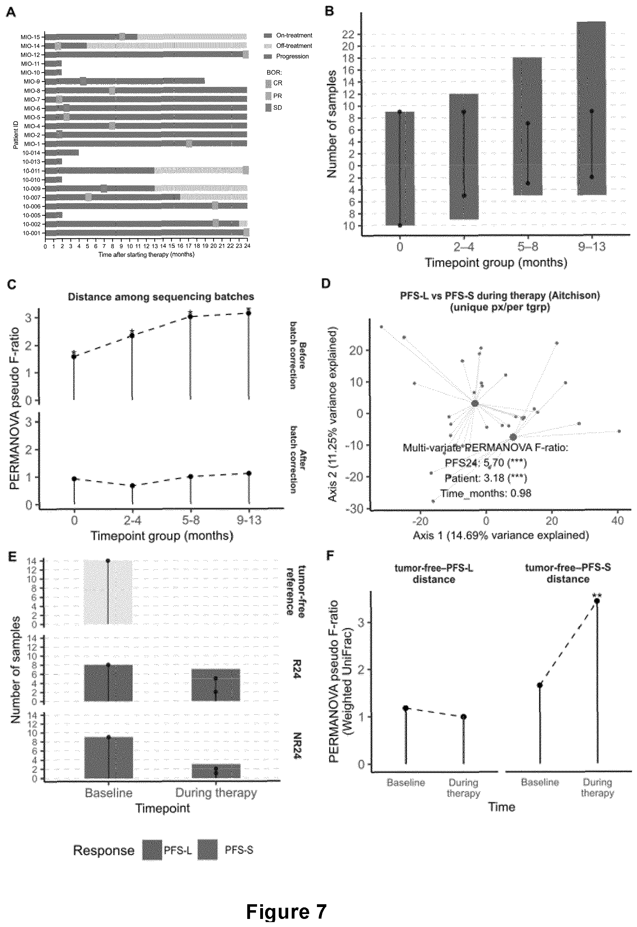

**Figure 7**. (A) Frequency distribution of melanoma Rand nR samples across time in aggregated months (0, 2-4, 5-8, 9-13 months of therapy). Inset line segments (black) indicate number of samples from non-redundant patients. **(B)** Aitchison gut composition difference between the two studies before (top) and after batch correction (bottom, p-adj>0.05, PERMANOVA). **(C)** Aitchison gut composition difference between R and nR, using equal samples across timepoint groups (n=6) (top), and using only unique patient samples (bottom), compared from t0, t2-4, t5-8, to t9-13. **(D)** PCA plot of R and nR at therapy, using only unique patient samples at t2-4, t5-8, and t9-13. Statistics are from multi-variate PERMANOVA (distance~Response+Patient+Timepoint) at therapy. **(E)** Frequency distribution of tumor-free reference, melanoma-R, and melanoma-nR samples sequenced in one run for tumor-free vs. melanoma comparison. Inset line segments (black) indicate number of samples from non-redundant patients (i.e., samples included at each timepoint group are all non-redundant). **(F)** Weighted UniFrac beta-diversity difference between tumor-free reference and melanoma-R and melanoma-nR groups compared at t0 and tn. Asterisk indicates PERMANOVA p-adj (distance-group) at the given timepoint group; p-adj<0.0001 (****), p-adj<0.001 (***), p-adj<0.01 (**), p-adj<0.05 (*), ns (unannotated).

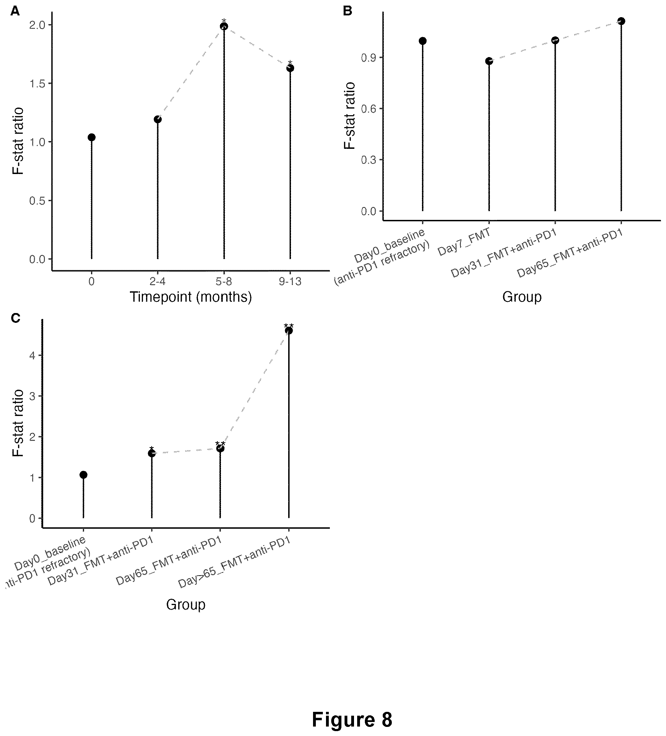

**Figure 8**. (A) Aitchison gut composition computed from Shotgun data (one batch). Plot shows difference between PFS-L and PFS-S (PFS24) melanoma patients across timepoint groups. **(B)** Aitchison gut composition difference between R and nR (RECIST v1.1) FMT recipients from day 0 (pre-FMT), day 7 (post-FMT, no-ICI), day 31 (on-ICI), and day 65 (on-ICI)⁸; **(C)** Aitchison gut composition difference between R and nR (RECIST v1.1) FMT recipients from day 0 (pre-FMT), day 31 (post-FMT + on-ICI), day 65 (on-ICI), and day >65 (on-ICI)¹⁰.

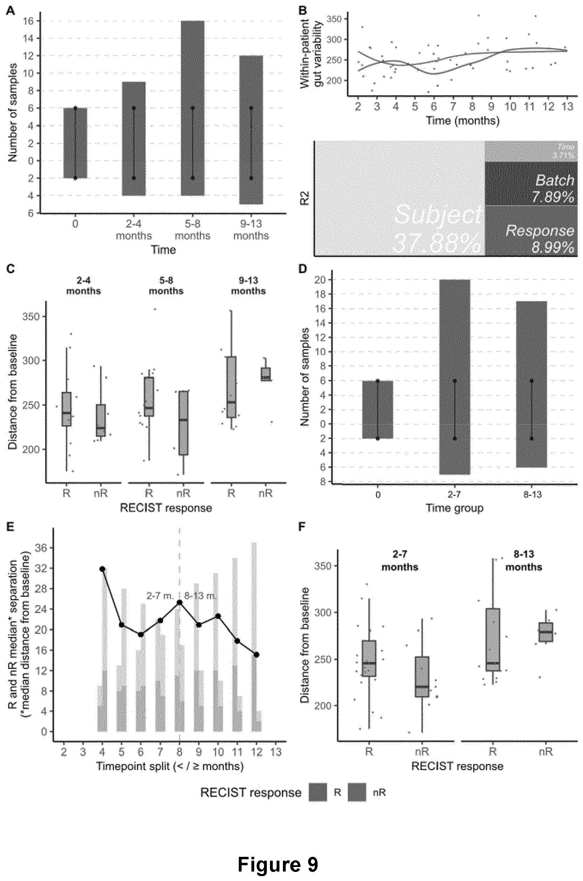

**Figure 9**. (A) Frequency distribution of melanoma Rand nR samples from the eight patients used for longitudinal analysis, across time in aggregated months (0, 2-4, 5-8, 9-13 months of therapy). Inset line segments (black) indicate number of samples from non-redundant patients. **(B)** Loess fit of within-patient gut microbiota change over therapy in R and nR groups based on baseline-normalized distance (top). Top variables explaining gut microbiota variability in all samples, measured by variance explained (R2) from multi-variate PERMANOVA (bottom). **(C-D)** Baseline-normalized distance compared between R and nR groups at timepoint groups t2-4, t5-8, and t9-13 **(C)** and at tearly and tlate **(D),** based on timepoint grouping described. **(E)** Δ median distance from baseline (|R-nR|) at different cut-offs for tearly, from < 4 to < 12 months. Inset barplot indicates sample size of R and nR groups at each cut-off. Dashed line indicates chosen cut-off, where tearly ≤7 months and tlate ≥8 months. **(F)** Frequency distribution of R and nR samples from the eight patients used for longitudinal analysis, in which samples were aggregated into t0, tearly, and tlate. Inset line segments (black) indicate number of samples from non-redundant patients.

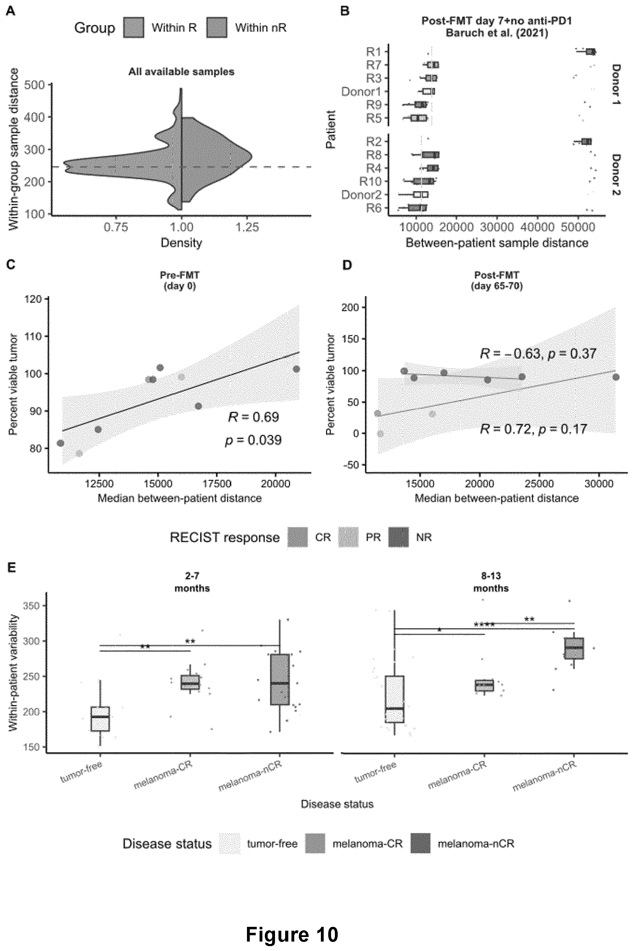

**Figure 10**. (A) Distribution of inter-sample distances within-R and within-nR; dashed blue line indicates cut-off for gut similarity at d=246. **(B)** Ranking of between-patient distances in melanoma patients that received fecal microbiota transplant (FMT). Data reflect gut composition similarity across patients at day 7 post-FMT, prior to immunotherapy. The plot includes between-patient measurements of the two FMT donors in the study, whose respective medians are indicated with a dashed line. **(C)** Spearman correlation of between-patient distance and tumor viability (%) in melanoma patients pre-FMT⁸. X-axis shows the median between-patient distance for each patient at day 0 pre-FMT, whereas y-axis shows the percent viable tumor determined from tumor biopsy pre-FMT. **(D)** Spearman correlation of between-patient distance and tumor viability (%) in melanoma patients post-FMT⁸. X-axis shows the median between-patient distance for each patient at day 65 post-FMT, whereas y-axis shows the percent viable tumor determined from tumor biopsy at day 70 post-FMT. Legend reflects RECIST response at endpoint of study. **(E)** Comparison of baseline-normalized distance in melanoma R and nR from our cohort and against a longitudinal healthy dataset¹⁸ at tearly and date. Gut composition distances are measured by Aitchison unless otherwise stated. P-adj<0.0001 (****), p-adj<0.001 (***), p-adj<0.01 (**), p-adj<0.05 (*), ns (unannotated).

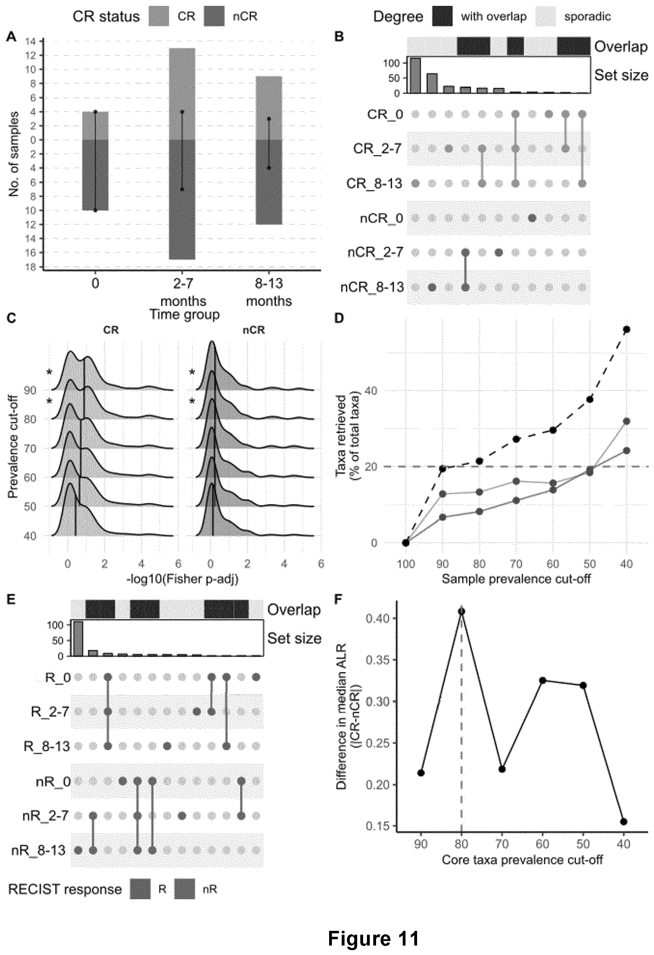

**Figure 11**. (A) Frequency distribution of melanoma CR and nCR samples across time in aggregated months (0, 2-8, 9-13 months of therapy). Inset line segments (black) indicate number of samples from non-redundant patients. **(B)** Differentially abundant taxa overlap at t0, tearly, and tlate in CR and nCR. Connecting dots indicate taxa shared between timepoint groups ("with overlap"), whereas non-connected dots indicate taxa detected only in one timepoint group ("spurious"). **(C)** Distribution of Fisher's exact test p-values (-log10(p-adj)) on taxa retrieved at increasing within-group sample prevalence cut-off, from 40% (not prevalent) to 80% (highly prevalent). Longitudinally consistent "stable" taxa are defined as the union of prevalent taxa at t0, tearly, and tlate within-CR and within-nCR. Density plots are grouped into CR- (left) and nCR- (right) associated based on odds ratio. **(D)** Number of taxa retrieved (% of total) at each prevalence cut-off within CR (blue), within nCR (red), and total (dashed), across timepoint groups. Dashed horizontal red line indicates cut-off of 20% taxa retrieved. **(E)** Stable taxa overlap at t0, tearly, and tlate in responders ("R") and not-responders ("nR"). **(F)** Change in CR-nCR median ALR difference with lower prevalence cut-offs. With overlap (dark gray), spurious (light gray).

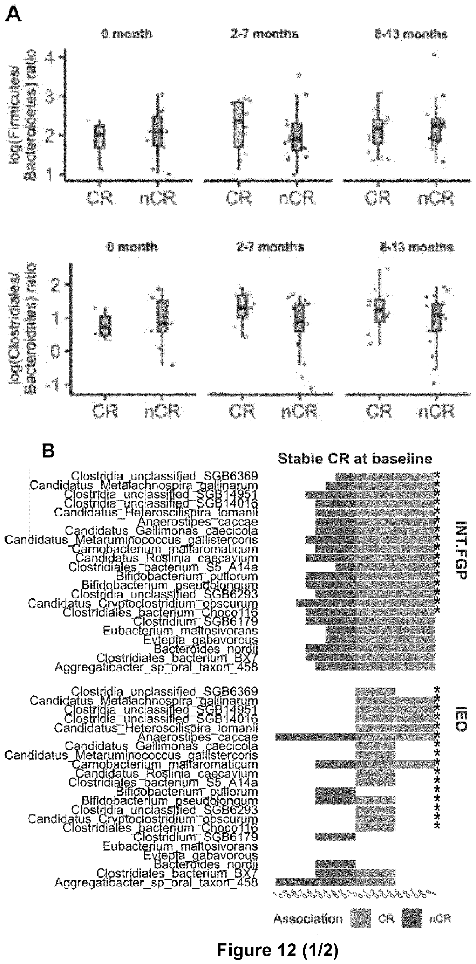

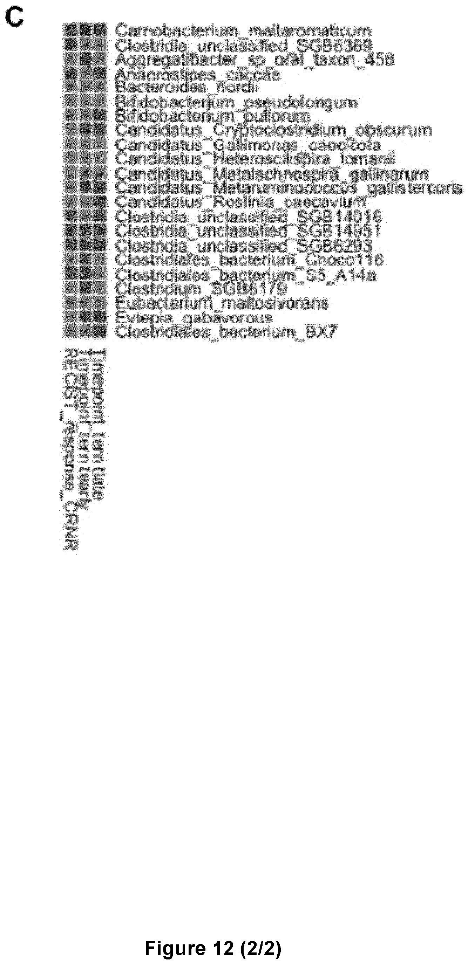

**Figure 12**. (A) Comparison of phylum-level Firmicutes/Bacteroidetes ratio (top), and order-level Clostridiales/Bacteroidales ratio (bottom) between complete responders (CR) and not-complete responders (nCR) at t0, tearly, and date. Ratios are log-transformed for visualization purposes. **(B)** Prevalence barplot of stable CR taxa in CR and nCR groups at baseline (t0). Inset points indicate significance in differential prevalence ranking by Fisher's exact test (-log10(p-adj) ≥ 1.3 (*); ns (.)), scaled by the maximum significance. **(C)** Differential abundance of stable CR taxa by response (with respect to nCR) and timepoint (with respect to baseline), based on Maaslin2 LM coefficient; boxes in ligher gray indicate positive association, whereas boxes in darker gray indicate negative association. All other plots: CR (light gray), nCR (dark gray); p-adj<0.0001 (****), p-adj<0.001 (***), p-adj<0.01 (**), p-adj<0.05 (*), ns (unannotated).

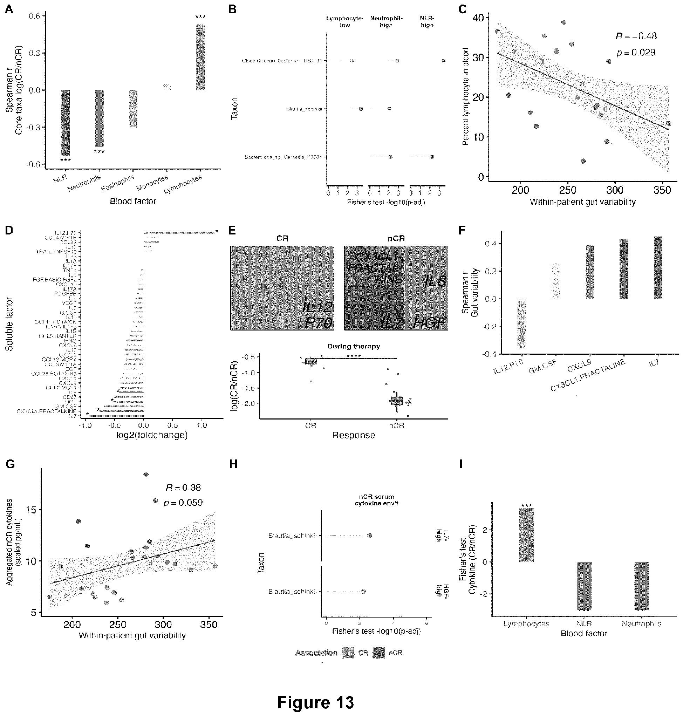

**Figure 13**. (A) Spearman correlation of stable taxa measure (log(CR/nCR)) with lymphocytes (r=0.65), neutrophils (r=-0.53) and NLR (r=-0.61). **(B)** Spearman correlation of within-patient gut variability with peripheral blood lymphocyte (% of total WBC). **(C)** Fisher's test of association between stable nCR gut microbiota taxa (darker gray) and blood environments, "lymphocyte-low, "neutrophil-high", and "NLR-high" during therapy. Taxa are filtered to include only significantly associated taxa at p-adj<0.01; Color indicates taxa belonging to stable nCR denominator (red). **(D)** Serum soluble factors associated with CR or nCR by log2(fold-change), selecting for unique patient samples at aggregated timepoints of 0, 1-4, 5-8, 9-11, and >11 months. Color intensity of bars indicate feature importance based on random forest mean decrease in accuracy (MDA); features in asterisk indicate most important cytokines for CR classification at MDA > 8.5. **(E)** CR- and nCR-associated cytokines scaled by their significance (-log10(Wilcoxon p-adj)) (top), and their corresponding additive log-ratio (ALR) in CR and nCR groups (bottom). **(F)** Fisher's test of association between CR/ nCR cytokine log-ratio and blood environments, "lymphocyte-high", "neutrophil-low", and "NLR". P-adj<0.0001 (****), p-adj<0.001 (***), p-adj<0.01 (**), p-adj<0.05 (*), ns (unannotated). **(G-H)** Spearman correlation of within-patient gut variability with **(G)** individual CR and nCR cytokines and **(H)** aggregated nCR cytokines (scaled pg/mL). **(I)** Fisher's test of association between stable nCR gut microbiota taxa (red) and serum cytokine status, "II,7-high" and "HGF-high" in therapy timepoints. Taxa are filtered to include only significantly associated taxa at p-adj<0.01. Continuous values from cytokine data were split into binary states by the median value (high: >median; low: ≤ median).

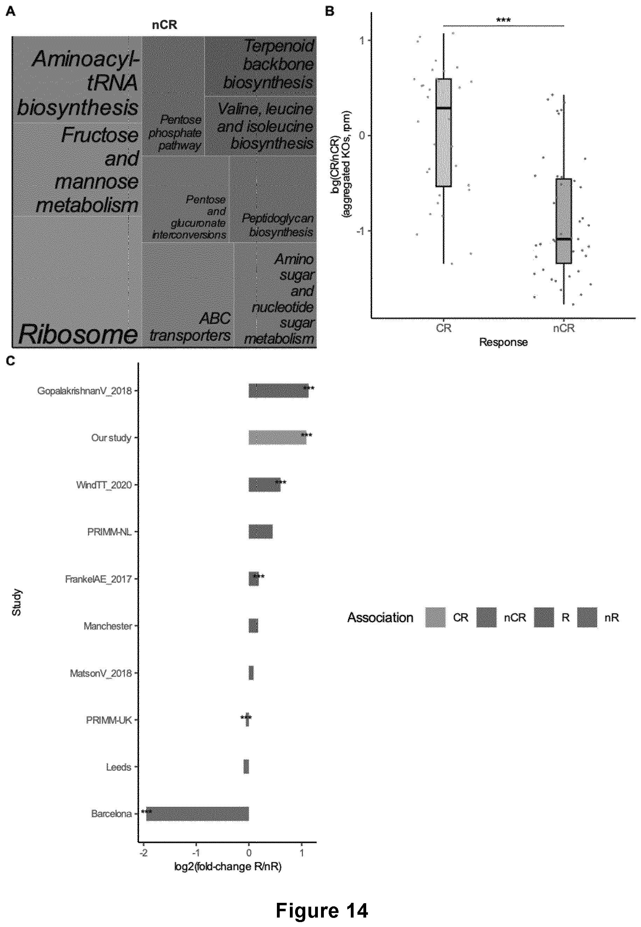

**Figure 14**. (A) Treemap of over-represented pathways in nCR at p-adj < 0.05, based on KOs associated with nCR across all samples (|LM coefficient| > 1.5). Terms in black indicate non-stable pathways. **(B)** Comparison of additive log-ratio (CR/nCR) of aggregated KOs mapping to significantly over-represented pathways (p-adj < 0.05) in CR and nCR groups, compared between CR (left) and nCR (right) pathways. Pathways are further filtered to include only significantly differentially abundant pathways based on aggregated KO abundance (p-adj < 0.1). **(C)** Baseline increase in bacteria-associated flagellin genes (RPK) in R (right) or nR (left) patients across ten melanoma cohorts, including this study that shows increase in CR (right). Statistics shown are from Wilcoxon rank sum test, whereas magnitude and direction are computed from log2 (fold-change) of counts in R/nR for the nine external studies and CR/nCR for our study.

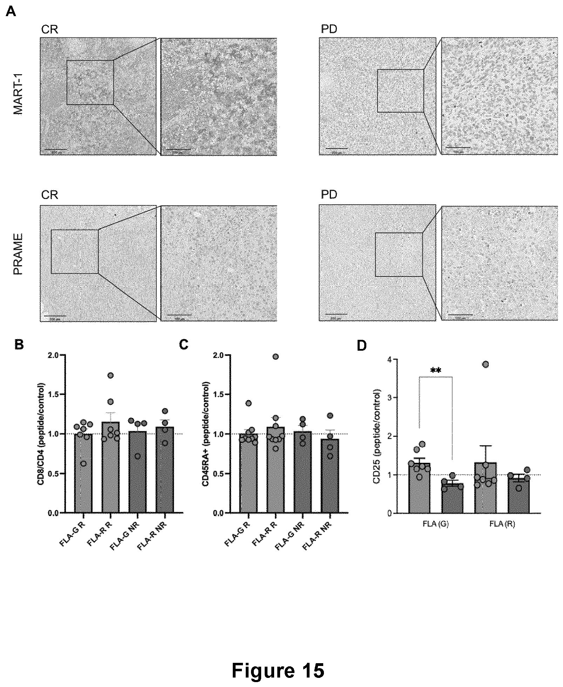

**Figure 15**. (A) Immunohistochemistry staining of representative CR and PD tumor samples using anti-Melan-A (top) and anti-PRAME (bottom) specific antibodies. **(B-D)** Flow cytometry analysis of T cell populations in peripheral blood monocytic cells (PBMC) from patients with melanoma grouped by response to ICI (complete response=4, progressive disease= 2). Bar plots representing the ratio of CD8+/CD4+ T cells in each patient (B), the percentage of na.ve (CD45RA+) and activated CD25+ (C) CD8⁺ T cells.

For flagellin protein is intended a protein forming the filament in a bacterial flagellum.

For flagellin-related gene is intended a gene coding for a protein which does not form the flagellum but which is associated with the activity of the flagellins. Said flagellin-related gene can be present in the genoma of any bacterial organism. Preferably, it is in the genoma of bacteria of Chlostridia class, more preferably of the Lachnospiraceae family, even more preferably of Roseburia genere, in particular Roseburia inulinivorans.

For "wherein said at least one peptide is coded by a flagellin-related gene" is intended that the peptide is a portion or a fragment of a protein which is coded by said gene. In particular, said peptide can be obtained by reducing said protein into fragments using conventional methods, wherein said fragments are at least 9 amino acids long, preferably between 9 and 20 amino acids, more preferably each fragment consists of 9 amino acids.

As used herein the expression "cancer characterized by increased expression of at least one of the following antigens" refers to a cancer having an increased expression of at least one of the mentioned antigens with respect to expression of the same antigen in a subject not affected by cancer.

"Nucleic acid", "nucleotide sequence" and "polynucleotide" are herein used as synonyms.

For "peptide" is intended a chain of amino acids linked by peptide bonds. A peptide typically comprises from 2 to 50 amino acids. According to the present invention, the peptide preferably comprises from 9 to 20 amino acids.

For "peptide variant" is intended a peptide having at least one different amino acid and retaining the ability to bind to the same MHC molecule to which the original peptide binds, for example peptides having one or two amino acids substituted by amino acids belonging to the same group. Peptides according to the invention can be synthetized according to common general knowledge in the field. Peptides coded by the genes herein described can be obtained by expressing the gene using routine techniques.

In an embodiment said peptide is coded by the flagellin-related gene A0A127SWS0 and is selected from the group consisting of peptides comprising or consisting of the following sequences: STEKLSSGY (SEQ ID N.11), RMI,NVTTSA (SEQ ID N.12), ALTEVHSML (SEQ ID N.13), SMLQRMNEL (SEQ ID N.14), and EVHSMLQRM (SEQ ID N.15).

In an embodiment said peptide is coded by the flagellin-related gene C0FQ36 and is selected from the group consisting of peptides comprising or consisting of the following sequences: VLTTVRVPK (SEQ ID N.16), IIINMSNNK (SEQ ID N.17), MKQEDTFVL (SEQ ID N.18), SHIMWLQSM (SEQ ID N.19), FPDMQKFTL (SEQ ID N.20) and KGSKSHIMW (SEQ ID N.21).

In an embodiment said peptide is coded by the flagellin-related gene A0A396AI63 and is selected from the group consisting of peptides comprising or consisting of the following sequences: TLMPCCPAV (SEQ ID N.22), YLNPVIEKA (SEQ ID N.23), YLENSFEEL (SEQ ID N.24), WLLDGQKEI (SEQ ID N.25), QAYNPLMRK (SEQ ID N.26), VFPREVHRF (SEQ ID N.27), EMADRMHEF (SEQ ID N.28), LPQKKNLSA (SEQ ID N.29), YIREKLTEL (SEQ ID N.30), NPLMRKFLI (SEQ ID N.31), RMHEFESEF (SEQ ID N.32), VLSETCAVF (SEQ ID N.33), KLCKLVTAY (SEQ ID N.34), FLREHARFY (SEQ ID N.35), LRWLLDGQK (SEQ ID N.36), ARFYIREKL (SEQ ID N.37), YRNYLNPVI (SEQ ID N.38), SEFQAYNPL (SEQ ID N.39), SEQTENPFL (SEQ ID N.40), NEFNADLLM (SEQ ID N.6), REKLTELFL (SEQ ID N.41) and ESLLVQYQW (SEQ ID N.42).

In an embodiment said peptide is coded by the flagellin-related gene A0A0M6WMV3 and is selected from the group consisting of peptides comprising or consisting of the following sequences: TSDTKQYTY (SEQ ID N.43), GMLNQTTLL (SEQ ID N.44), YIIAVNQTL (SEQ ID N.45), RMSNQQLTV (SEQ ID N.46), AVKAGNMPK (SEQ ID N.47), KTAEEMSQK (SEQ ID N.48), IYQDIDYII (SEQ ID N.49), IIIDYTAAY (SEQ ID N.50), MPKDGAAFI (SEQ ID N.2), MRVTNNMML (SEQ ID N.51), YRTNCKLTF (SEQ ID N.52), VELKTAEEM (SEQ ID N.53), SAQENLQTW (SEQ ID N.54) and KTGTLSYHY (SEQ ID N.55).

In an embodiment said peptide is coded by the flagellin-related gene A0A0M6WD36 and is selected from the group consisting of peptides comprising or consisting of the following sequences: FSDEGKMSY (SEQ ID N.56), KLCDIYSEL (SEQ ID N.57), VLAKAQFAI (SEQ ID N.58), KLDDRIANA (SEQ ID N.59), ISFDEAFHV (SEQ ID N.60), KMSYEDIEL (SEQ ID N. 1), ILRVPSYYK (SEQ ID N.61), KTFQCIADK (SEQ ID N.62), SYYKTFQCI (SEQ ID N.63), RYFANGKSF (SEQ ID N.64), YYMGIEGAF (SEQ ID N.65), YFHVRMETF (SEQ ID N.66), VYFTYRYFM (SEQ ID N.67), RVPSYYKTF (SEQ ID N.68), DVREKSLSV (SEQ ID N.69), MILRVPSYY (SEQ ID N.70), ALCEVCTEY (SEQ ID N.71), YRYFMRAWY (SEQ ID N.72), YRTQAVAIL (SEQ ID N.73), VRMETFDEL (SEQ ID N.74), NEIWYEHIL (SEQ ID N.75), YEDIELPDL (SEQ ID N.76), IEGAFGERL (SEQ ID N.77) and KSGDYNEIW (SEQ ID N.78).

In an embodiment said peptide is coded by the flagellin-related gene D4KYR7 and is selected from the group consisting of peptides comprising or consisting of the following sequences: RLDSGNYNV (SEQ ID N.79), KLLEKYNAL (SEQ ID N.80), KTRKRISRK (SEQ ID N.81) and DAIMRVEAY (SEQ ID N.82).

In an embodiment said peptide is coded by the flagellin-related gene R6VY20 and is selected from the group consisting of peptides comprising or consisting of the following sequences: TSENMTSAY (SEQ ID N.83), STDVPVKEY (SEQ ID N.84), AMTEISEML (SEQ ID N.85), KLLNGEYDL (SEQ ID N.86), VLEVNIPAV (SEQ ID N.87), RMNAQIEGL (SEQ ID N.88), TIKSIPLTK (SEQ ID N.89), DMAEEMTSY (SEQ ID N.90), RPSQVLQLL (SEQ ID N.91), SVRGRLGAF (SEQ ID N.8), EMLQRINEL (SEQ ID N.92), II,AQAGTSM (SEQ ID N.5), GQKLLNGEY (SEQ ID N.93), MRI,DVSGAL (SEQ ID N.94), GENGFEMRL (SEQ ID N.95) and TEFNGQKLL (SEQ ID N.7).

In an embodiment said peptide is coded by the flagellin-related gene A0A351R0D5 and is selected from the group consisting of peptides comprising or consisting of the following sequences: RIRDTDMAK (SEQ ID N.96), KVSSQRSAL (SEQ ID N.97), ILAQAGQSM (SEQ ID N.4) and VKSSLAFSL (SEQ ID N.98).

In an embodiment said peptide is coded by the flagellin-related gene A0A0M6WBR8 and is selected from the group consisting of peptides comprising or consisting of the following sequences: LADFIYQKY (SEQ ID N.99), AVAEII,AMV (SEQ ID N.100), LMSDALIQI (SEQ ID N.101), FVVTALISV (SEQ ID N.102), RMMQDVPKA (SEQ ID N.103), YLAQKIKEA (SEQ ID N.104), GLADFIYQK (SEQ ID N.105), LYQAVAEIL (SEQ ID N.106), LMNSFVDIY (SEQ ID N.107), KSKELTAAF (SEQ ID N.108), RIFSKDSIF (SEQ ID N.109), QQKQLHLIM (SEQ ID N.110), RRMMQDVPK (SEQ ID N.111), GHAQDIFIL (SEQ ID N.112), YEIPLMQAI (SEQ ID N.113), KELTAAFDL (SEQ ID N.114), IEIVENKPL (SEQ ID N.115), GEDYLAQKI (SEQ ID N.116), LIMEWDLQW (SEQ ID N.117), LISVIQVGW (SEQ ID N.118) and KQLHLIMEW (SEQ ID N.119).

In an embodiment said peptide is coded by the flagellin-related gene A0A0M6WYS8 and is selected from the group consisting of peptides comprising or consisting of the following sequences: FTGLQASRY (SEQ ID N.120), ALDDAIAMV (SEQ ID N.121), SIFSSTATV (SEQ ID N.122), ALNETSAIL (SEQ ID N.3), KMQAQIDAL (SEQ ID N.123), SLVLTFSGK (SEQ ID N.124), NMSAVITNK (SEQ ID N.125), TIKQPAATK (SEQ ID N.126), ETSAILQRM (SEQ ID N.127), DMAEEMTNY (SEQ ID N.128), LPQNVLSLL (SEQ ID N.129), LQRMRELSV (SEQ ID N.130), LQQAGVSVL (SEQ ID N.131), SRYGSSASL (SEQ ID N.132), VELSAGTAI (SEQ ID N.133), TEYNSKSLL (SEQ ID N.134) and SSASLVLTF (SEQ ID N.135).

In an embodiment said peptide is coded by the flagellin-related gene R6EJ07 and is selected from the group consisting of peptides comprising or consisting of the following sequences: TSENMTAAY (SEQ ID N.136), AMTEISEML (SEQ ID N.85), KLLNGEFDL (SEQ ID N.137), VLEVNIPAV (SEQ ID N.87), RMNAQIEGL (SEQ ID N.88), DMAEEMTSY (SEQ ID N.90), RPSQVLQLL (SEQ ID N.91), SVRGRLGAF (SEQ ID N.8), EMLQRINEL (SEQ ID N.92), II,AQAGTSM (SEQ ID N.5), GQKLLNGEF (SEQ ID N.138), MRLDVSEAK (SEQ ID N.139) and TEFNGQKLL (SEQ ID N.7).

In an embodiment said peptide is coded by the flagellin-related gene R6VQ94 and is selected from the group consisting of peptides comprising or consisting of the following sequences: ILVMVTVTV (SEQ ID N.140), LQGPFVINV (SEQ ID N.141), MTVNLQGPF (SEQ ID N.142), and FPELKHFTM (SEQ ID N.9).

In an embodiment said peptide is coded by the flagellin-related gene A0A0M6WP36 and is selected from the group consisting of peptides comprising or consisting of the following sequences: GLDALNNLL (SEQ ID N.10) and LTRKKGEAL (SEQ ID N.143).

All combinations of said peptides are included in the invention.

Preferably, the composition of the invention comprises peptides with a length from 9 amino acids to 30 amino acids, preferably from 9 amino acids to 20 amino acids, more preferably of 9 amino acids.

The peptide according to the present invention, or the peptide variant as defined above, may comprise additional portions of amino acids at the N-terminus or C-terminus, which are not necessarily part of the peptide portion that serves as an epitope for the MHC molecules.

However, these additional parts can be important to provide an efficient introduction of the peptide according to the present invention into the cells.

In a further embodiment of the present invention, the peptides are bound to an antibody, or a functional part of the antibody itself, in particular peptides can be inserted into a sequence of an antibody, so as to be specifically carried by the antibody or, for example, can be fused to an antibody, or inserted into an antibody, which is specific for dendritic cells.

Furthermore, the peptides according to the present invention can be further modified or modified to improve the stability and / or the binding with the MHC molecules, in order to obtain a stronger immune response. Methods for optimizing a peptide sequence are well known in the art and include, for example, the introduction of reverse peptide bonds or non-peptide bonds.

In a reverse peptide bond the amino acid residues are not joined by peptide bonds (-CO-NH-), but the peptide bond is reversed. Such retro-inverse peptidomimetic peptides can be made using methods known in the art, such as those described in Meziere et al (1997) J. Immunol. 159, 3230-3237, incorporated herein by reference. This approach involves the creation of pseudopeptides containing changes that involve the skeleton and not the orientation of the side chains. Meziere et al (1997) show that these pseudopeptides are useful for MHC binding and helper T cell responses.

Retro-inverse peptides, which contain NH-CO bonds instead of CO-NH peptide bonds, are much more resistant to proteolysis.

A non-peptide bond can be, for example, one of the following: -CH₂-NH, -CH₂S-, -CH₂CH₂-, - CH=CH-, -COCH₂-, -CH(OH)CH₂- and -CH₂SO-. U.S. Pat. 4,897,445 provides a method for the solid phase synthesis of non-peptide bonds (-CH₂-NH) in polypeptide chains involving the use of polypeptides synthesized by standard procedures and the non-peptide bond synthesized by the reaction of an amino-aldehyde and an amino acid in the presence of NaCNBH₃.

The peptides comprising the sequences described above can be synthesized with further chemical groups present at their amino and/or carboxyl end groups, to improve the stability, bioavailability and/or affinity of the peptides. For example, hydrophobic groups such as the carbobenzyloxy, dansyl or t-butyloxycarbonyl groups can be added to the amino terminals of the peptides.

Similarly, an acetyl group or a 9-fluorenylmethoxycarbonyl group can be placed at the amino terminals of the peptides. Furthermore, the hydrophobic group, the t-butyloxycarbonyl or an amide group can be added to the carboxy-terminus of the peptides.

Furthermore, the peptides according to the present invention can be synthesized in such a way as to alter their steric configuration. For example, the D isomer of one or more of the amino acid residues of the peptide can be used, rather than the usual L isomer. Further, at least one of the amino acid residues of the peptides of the invention can be replaced by one of the well known amino acid residues, not present in nature. Alterations such as these can serve to increase the stability, bioavailability and / or binding action of the peptides of the invention.

Similarly, a peptide or a variant of the peptide according to the present invention can be chemically modified or modified by reacting specific amino acids before or after the synthesis of the peptide. Examples for such modifications are well known in the art and are summarized for example in R. Lundblad, Chemical Reagents for Protein Modification, 3rd ed. CRC Press, 2005, which is incorporated herein by reference.

Chemical modification of amino acids includes, but is not limited to: modification by acylation, amidation, pyridoxylation of lysine, reductive alkylation, trinitrobenzylation of amino groups with 2,4,6-trinitrobenzenesulfonic acid (TNBS), amide modification of carboxylic groups and sulfhydryl modification by oxidation with performic acid of cysteine to cysteic acid, formation of mercurial derivatives, formation of mixed disulfides with other thiol compounds, reaction with maleimide, carboxymethylation with iodoacetic acid or iodoacetamide and carbamylation with cyanate at alkaline pH. In this regard, the methodologies for the chemical modification of proteins are well known to one skilled in the art.

In short, the modification of arginyl residues in proteins is often based on the reaction of vicinal dicarbonyl compounds such as phenylglyoxal, 2,3-butanedione and 1,2-cyclohexanedione to form an adduct. Another example is the reaction of methylglyoxal with arginine residues. Cysteine can be modified without the concomitant modification of other nucleophilic sites such as lysine and histidine.

Consequently, a large number of cysteine modification reagents are available. The websites of companies such as Sigma-Aldrich (www.sigma-aldrich.com) provide information on specific reagents.

The selective reduction of disulfide bonds in proteins is also well known. Disulfide bonds can be formed and oxidized during the heat treatment of biopharmaceutical products. Woodward's K reagent can be used to modify specific glutamic acid residues. N-(3-(dimethylamino)propyl)-N'-ethylcarbodiimide can be used to form intra-molecular connections between a lysine residue and a glutamic acid residue. For example, diethyl pyrocarbonate is a reagent for the modification of histidyl residues in proteins. Histidine can also be modified using 4-hydroxy-2-nonenal. The reaction of lysine residues and other α-amino groups is, for example, useful in the binding of peptides to surfaces or in the cross-linking of proteins / peptides. Lysine is the attachment site of poly (ethylene) glycol and the major modification site in protein glycosylation. The methionine residues in proteins can be modified for example with iodoacetamide, bromoethylamine and chloramine T.

Tetranitromethane and N-acetylimidazole can be used for the modification of tyrosyl residues. Cross-linking through the formation of di-tyrosine can be accomplished with hydrogen peroxide/copper ions.

N-bromosuccinimide, 2-hydroxy-5-nitrobenzyl bromide or 3-bromo-3-methyl-2-(2-nitrophenylmercapto)-3H-indole (BPNS-skatole) have been used in some tryptophan modification studies.

Successful modification of therapeutic proteins and peptides with PEG is often associated with a prolongation of the circulatory half-life, while cross-linking of proteins with glutaraldehyde, polyethylene glycol diacrylate and formaldehyde is used for the preparation of hydrogels.

Chemical modification of allergens for immunotherapy is often achieved by carbamylation with potassium cyanate.

In general, peptides and variants (at least those containing peptide bonds between amino acid residues) can be synthesized by the Fmoc-polyamide mode of solid phase peptide synthesis, as disclosed by Lukas et al. (Solid-phase peptide synthesis under continuous flow conditions Proc. Natl Acad Sci USA, May 1981; 78 (5): 2791-2795). Temporary protection of the N-amino group is provided by the 9-fluorenylmethyloxycarbonyl (Fmoc) group.

In another aspect, the invention provides at least one polynucleotide coding for at least one of the peptides disclosed herein. The skilled person can design such polynucleotide according to the common general knowledge regarding nucleic acids coding for peptides.

Generally, expression vectors are plasmids which are used to introduce a desired nucleic acid sequence, such as a gene, into a target cell, resulting in the transcription and translation of the protein encoded by the nucleic acid sequence, i.e. the chimeric antigen receptor, the antibody or the binding molecule. Therefore, the expression vector in general comprises regulatory sequences, such as promoter and enhancer regions, as well as a polyadenylation site in order to direct efficient transcription of the nucleic acid sequence on the expression vector. The expression vector may further comprise additional necessary or useful regions, such as a selectable marker for selection in eukaryotic or prokaryotic cells, a purification tag for the purification of the resulting protein, a multiple cloning site or an origin of replication.

Usually, the expression vector may be a viral or a non- viral vector. In general, various kinds of viral vectors, such as retroviral vectors, e.g. lentiviral or adenoviral vectors, or plasmids may be used.

In another aspect, the invention provides, compositions, e.g., pharmaceutical compositions, which include a pharmaceutically acceptable carrier, excipient or stabilizer, and at least one of the peptides described herein. In one embodiment, the composition, e.g., the pharmaceutical composition, includes a combination of the peptide(s) and one or more agents, e.g., a therapeutic agent, as described herein. The composition can also be a vaccine against a cancer. For vaccine against a cancer is intended that administration of the composition to a subject stimulates immunity against a cancer. The cancer can be any cancer as herein defined.

The peptide or the pharmaceutical composition can be administered to the subject in various embodiments in a formulation comprising suitable carriers, excipients, and other agents to provide improved transfer, delivery, tolerance, and the like, and suitable for an intravenous or subcutaneous injection, or for intramuscular or oral delivery.

The content of the peptide in the pharmaceutical composition is not limited as far as it is useful for treatment or prevention of a cancer. The choice of the carrier may depend upon the route of administration and concentration of the active agent(s) and the carrier may be in the form of a 21ecerning21d composition or an aqueous solution. Generally, an appropriate amount of a pharmaceutically acceptable salt is used in the carrier to render the composition isotonic. Examples of the carrier include but are not limited to saline, Ringer's solution and dextrose solution. Preferably, acceptable excipients, carriers, or stabilizers are non-toxic at the dosages and concentrations employed, including buffers such as citrate, phosphate, and other organic acids; salt-forming counter- ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol). Suitable carriers and their formulations are described in greater detail in Remington's Pharmaceutical Sciences, 17th ed., 1985, Mack Publishing Co. The composition may also contain at least one further active compound, such as an anti-cancer agent. Preferred anti-cancer agents include a chemotherapeutic agent, a targeted drug and an immunotherapeutic agent, such as an immune checkpoint modulator. Preferably, said further active compound is an immune checkpoint inhibitor (ICI). Said ICI can be selected from an antibody anti-PD-1, an antibody anti-PD-L1 and an antibody anti-CTLA-4.

Preferably, the peptide is included in an effective amount. The term "effective amount" refers to an amount sufficient to induce a detectable therapeutic response in the subject to which the pharmaceutical composition is to be administered.

The peptide or the composition according to the invention can be administered to the subject using any acceptable device or mechanism.

According to the invention, "subject" means a human subject or human patient.

For "melanoma" or "melanoma cancer" it is intended a skin cancer that develops from the pigment-producing cells known as melanocytes. Melanoma can be of any type.

The invention will be now illustrated by the following examples.

### EXAMPLES

### Example 1

### Methods

### Sample collection.

Melanoma patients (n=23) were followed at baseline and over the course of anti-PD1 immunotherapy and collected fecal (Canvax Biotech, ES) and blood samples for at least one timepoint. Select fecal samples were processed for 16S amplicon (n=94) and shotgun metagenomic sequencing (n=65), whereas blood samples were processed as whole blood for white blood cell count (n=58) and as serum for soluble factor quantification (n=40).

For the 16S sequencing, 19 patients have baseline samples, 17 have therapy samples, and 13 have baseline and at least 1 therapy sample. Of the 13, 8 had samples both at 1-7 months (early therapy) and 8-13 months (late therapy); these 8 patients were used for the 16S longitudinal analysis.

For the shotgun sequencing, 14 patients have baseline samples, 11 have samples at 1-7 months (early therapy) and 7 have samples at 8-13 months (late therapy). These samples were used for stable taxa and functional analyses.

### 16S amplicon sequencing.

Fecal samples were processed for 16S sequencing to compare the microbiota structure and composition at baseline (t0) (i.e., before therapy) and at each cycle of therapy (t1, t2, t3... tn). Briefly, DNA was extracted from feces of melanoma patients and healthy donors using the Dneasy PowerSoil Pro kit (Qiagen), after which the V3-V4 region of 16S was amplified. Libraries were prepared following the 16S sequencing library preparation protocol (Illumina), and sequenced by a 2x250 bp paired end chemistry on a MiSeq platform.

### Sequence processing.

For each 16S sequencing run, data were filtered and denoised using the DADA2¹⁹ plug-in in qiime2 (qiime2-2018.11)²⁰ where parameters were set to trim sequences at the 5' region by the length of the primer, to generate a counts table of amplicon sequence variants (ASVs) for that dataset.

Phylogenetic tree reconstruction for downstream diversity analyses was done with the q2-fragment-insertion plug-in²¹, using SILVA 128 database²² as reference sequence. For taxonomy assignment the q2-feature-classifier plug-in was used. Briefly, full-length reference sequences from the SILVA 132 database were downloaded from the SILVA resources page for qiime (https://www.arb-silva.de/download/archive/qiime), after which the sequences were used to train a Naive-Bayes classifier using the fit-classifier-naïve-bayes function²³. The trained classifier was run on the representative sequences output of DADA2 using the classify-skleam function to generate taxonomic assignments for each ASV.

All downstream analyses were performed in R, after exporting the taxonomy table, ASV counts table, phylogenetic tree, and metadata in R and converting into a phyloseq object²⁴.

### Core diversity from rarefied data.

All downstream analyses were performed in R, after exporting the taxonomy table, ASV counts table, phylogenetic tree, and metadata in R and converting into a phyloseq object. Alpha and beta diversity analyses were performed using the vegan package²⁵, using counts rarefied by the lowest sequence depth within a given dataset. For alpha-diversity analysis by standardized Faith's phylogenetic diversity, the ses.pd function from the picante package was used²⁶. For computing higher level taxa ratios, raw counts were first aggregated to that level before rarefaction. Group differences based on alpha-diversity and counts ratio were computed by Wilcoxon rank sum test²⁷; beta-diversity differences were computed from the distance matrices by PERMANOVA²⁸, checking for balance in dispersion by PERMDISP²⁵. All reported significant p-values by PERMANOVA were checked to have non-significant dispersion.

### Batch correction.

For batch correction, raw counts tables were first transformed by centered-log-ratio (CLR) following the mixOmics workflow for pre-processing microbiota data²⁹, and batch effect correction was applied using COMBAT with non-parametric setting³⁰. The overall composition of the samples was projected into a PCA after computing the Aitchison distance on the transformed abundance, testing group differences on the abundance table by PERMANOVA while checking for balance in dispersion by PERMDISP.

For the melanoma versus tumor-free and baseline versus immunotherapy comparisons, batch correction was performed more conservatively: first, by mean-centering CLR-transformed raw counts from each sequence batch within a group; this ensures that correction is performed by batch while preventing within-batch between-group differences from being corrected; then, by merging the separately batch-corrected groups together as one dataset. Composition was visualized as described above.

### Distance-based comparisons.

For handling distance data the usedist package³¹ was used: dist_subset for subsetting distances, dist_get for retrieving specific group-wise distances, dist_groups for sorting within- and between-group distances.

### Within-patient gut distance.

To normalize for patient variability in the longitudinal analysis of 16S data, within-patient gut distance was used as measure, computing the Euclidean distance between a baseline and a timepoint sample from taxonomic abundance data (CLR-transformed and batch-corrected), and doing boxplot group comparisons on this distance, using non-parametric Wilcoxon rank sum as statistical test.

### Between-patient gut distance.

As measure of gut similarity, patients were ranked by between-patient gut distance: first, the Euclidean distance from taxonomic abundance data (CLR-transformed and batch-corrected) was computed, then, distances between-patient distances was retrieved, extracting between-group distances after using patient ID metadata in the dist_groups function from usedist.

### Shotgun metagenomic sequencing.

DNA extracts from fecal samples of select melanoma samples from our study were subjected to metagenomic sequencing, where libraries were prepared using the Illumina DNA Prep Kit according to manufacturer's protocol. Libraries were multiplexed using dual indexing and sequencing was performed with a 300-bp paired-end chemistry, using the Illumina NovaSeq6000 platform according to manufacturer's protocol.

### Sequence pre-processing.

Shotgun metagenomic sequencing was performed at the NGS Core Facility at University of Trento. The quality of all sequenced metagenomes was controlled using the preprocessing pipeline implemented in https://github.com/SegataLab/preprocessing, which consists of three main stages: (1) initial quality control by removing low-quality reads (quality score <Q20), fragmented short reads (<75 bp) and reads with more than two ambiguous nucleotides; (2) contaminant DNA removal using Bowtie 2³² and the sensitive local parameter, removing both the phiX174 Illumina spike-in and human-associated reads (hg19); and (3) sorting and splitting for the creation of standard forward, reverse and unpaired reads output files for each metagenome.

### Metagenomic analyses.

Metagenomic sequence data from our study as well as from previously published baseline melanoma cohorts¹¹ were run through the biobakery 3 pipeline³³, which leverages a set of 99,200 high-quality and fully annotated reference microbial genomes spanning 16,800 species and the 87.3 million UniRef90³⁴ functional annotations available in UniProt as of January 2019. Taxonomic profiling of taxa composition of all metagenomic samples was performed with MetaPhlAn v4.0.3³⁵ using default parameters and CHOCOPhlAnSGB v202103 as database. Functional potential analysis of the metagenomic samples was performed with HUMAnN v3.6³³ using default parameters.

### Stable taxa analysis.

Within CR and nCR groups, prevalent taxa were identified as taxa having a non-zero abundance in > 80% of samples. This was determined within each timepoint group, t0, tearly (< 8 months), and tlate (≥ 8 months). Cut-off at 80% was determined as the minimum prevalence at which the median differential prevalence score is significantly greater than that at the starting cut-off of 40% prevalence (i.e., not highly prevalent) (Figure 10C). To account for having two studies in the analysis, prevalent taxa identification was performed as follows: first, putting together both studies, and second, subsetting only for INT-FGP samples. The union of taxa identified within-group with each dataset was taken as the prevalent taxa set for that group.

To utilize longitudinality of our data, prevalent taxa were further filtered for longitudinal consistency. "Stable" taxa were thus determined as prevalent taxa detected in all three timepoint groups for CR (t0, tearly, and tlate), and in at least two timepoint groups nCR (t0 and tearly or t0 and tlate).

### Taxonomic differential ranking.

To determine significantly differentially abundant taxa, Wilcoxon rank sum test was performed on taxonomic abundance data from metaphlan (relative abundance), with p-value adjustment by FDR. To determine the group with which a feature was associated, the corresponding log2 fold-change was also computed, by adding an offset of 0.01 to the abundances, getting the mean relative abundance of each feature across samples within a group, and, using the foldchange package, getting the fold-change (gtools)³⁶ of the group means and converting it to log-ratio (foldchange2logratio).

Differential prevalence was performed as follows. First, from the taxonomy counts table, abundances > 0 were all converted to 1 to signify presence. A contingency table was built by counting the presences and absences at a binary category being tested, after which Fisher's exact test²⁰ was performed on the table. P-adj were used to determine significantly differentially prevalent taxa, and the calculated odds ratio (OR) for each feature was used to determine direction of association.

Differential ranking was performed on the hits by sorting the features by the +log10(p-adj) of each feature, determining the group of association by the sign of either the log2 fold-change (for differential abundance) or Fisher's OR (for differential prevalence). The hits within a group were percentile-ranked using dplyr::ntile³⁸ with n=100, and the hits that fall in the 75^{th} percentile or more were considered as top ranking taxa. Differential abundance and prevalence indices of stable taxa were measured by taking the size of stable taxa and dividing against the size of top-ranking hits.

### Additive log-ratio.

To account for compositionality of taxonomic data, group comparisons on metagenomic (taxonomic and gene family) abundance data were performed by first subsetting the abundance table (relative abundance for taxonomy and RPK for gene family) to the features of interest (e.g., features that were determined to define each group of comparison), sum-aggregating abundances across features, taking the ratio of the values between the two groups, and log-transforming the sum³⁹. For response group comparisons, values pertaining to the CR group were always used as numerator.

### Differential abundance on functional data.

To determine significant terms associated with a group, differential abundance was performed using Maaslin2⁴⁰. First, gene family abundances from Humann were re-grouped from gene family terms (Uniprot90 IDs) to KEGG orthologs (KOs). Group-associated KOs were determined by converting abundances in RPK to rpm, linearly modelling log-transformed relative abundances against response status of samples at t0, tearly, and tlate. Significance for differential abundance was set at p-adj < 0.05.

### Over-representation analysis of pathways.

Next, over-representation analysis was performed to examine higher level pathways within the CR and nCR groups and pathways. For each group of comparison, KOs with |LM coefficient| > 1.5 were taken as input, filtering for those taxa associated with the given group by the sign of LM coefficient (eg., +LM values only for comparison group; -LM values only for reference group). Over-representation of pathways was tested using the enricher function from clusterProfiler in R⁴¹, using the pathway-KO mapping file from https://github.com/picrust/picrust2/blob/master/picrust2/default_files/pathway_mapfiles/KEGG _pathways_to_KO.tsv and KO-pathway names from https://github.com/biobakery/humann/blob/master/humann/data/misc/map_kegg-pwy_name.txt.gz. Significantly over-represented pathways present across timepoints were defined as "stable".

### Flagellin-related analyses.

To determine flagellin-related terms from the gene family abundance table, a list was generated from the UniProt website (https://www.uniprot.org/), searching for "(taxonomy_id:2) AND flagellin" within the UniRef database and matching for UniRef90 hits as was used for the gene family data. With this list, we retrieve 1,563 features present in our dataset and 4,241 features in the multi-cohort baseline melanoma data.

To determine Lachnospiraceae-related terms the bacterial flagellin list was subset to those whose Genus designation in the 'Organism' column falls in Lachnospiraceae (n=59). With this list, we retrieve 391 features present in the multi-cohort baseline melanoma data.

In our dataset, group comparisons for abundance were performed by Wilcoxon rank sum test, applying p-value adjustment with FDR and visualizing as boxplots by log10-transforming the abundances. To perform flagellin differential testing on the multi-cohort and our dataset, abundances were sum-aggregated before performing group comparisons by Wilcoxon rank sum test. Log2 fold-change was computed by getting the median of the sum-aggregate abundance across samples within a group, and, using the gtools package³⁶, getting the fold-change (foldchange) of the group medians and converting it to log-ratio (foldchange2logratio).

### Candidate complete response flagellin markers.

To shortlist response-related flagellin markers from our longitudinal data, log2 fold-change was computed by getting the median abundance of each flagellin across samples within CR and nCR, and, using the gtools package³⁶, getting the fold-change (foldchange) of the group medians and converting it to log-ratio (foldchange2logratio). The top 14 flagellin markers with the highest log2 fold-change were identified as candidate CR flagellin markers.

### Epitope prediction and tumor antigen matching.

Candidate flagellin proteins determined from metagenomic data were tested for tumor mimicking potential following the methods described in Ragone et al., 2022⁴². Briefly, protein sequences of our candidate flagellins were queried, NetMHCstabpan 4.1⁴³ was used to shortlist 9-mer peptides with predicting strong-binding (SB) affinity (<100 nM) to MHC class I HLA alleles. BLAST homology search was performed on these SB peptides against a pre-determined list of tumor-associated antigens (n=271), after which homologous sequences were queried for epitope prediction using NetMHCstabpan 4.1. Among the homologous sequences, those peptides with matching HLA allele affinity to any of the TAAs were selected for structural prediction.

Molsoft Mol Browser (version 3.8-7d) (Molsoft LLC, San Diego, CA) was used for epitope modelling and molecular docking and conformation calculations for the shortlisted peptides and matching TAAs.

### Immunohistochemistry.

Slides (5 µm) from formalin-fixed paraffin embedded (FFPE) samples were processed for deparaffinization and rehydration. Heat-induced antigen retrieval (citrate buffer pH 6 for MART-1 or 9 for PRAME, Thermofisher) was followed by incubation in 3% H₂O₂ to inactivate endogenous peroxidase. Slides were incubated overnight at 4 °C with rabbit anti-human MART-1 (1:400) and rabbit anti-human PRAME (EPR20330, 1:400) in blocking solution (3% BSA, 5% goat serum, 0.1% Triton in PBS). After incubation with HRP-secondary antibody (RT, 30 min), the staining was developed using Aminoethyl Carbazole (AEC) + High Sensitivity Substrate Chromogen (Dako, Santa Clara, CA, USA) and slides counterstained with hematoxylin. Mounted slides were acquired using Aperio ScanScope whole slide scanner (Leica Biosystems Inc).

### Bead-based multiplexed ELISA.

Multiplexed ELISA serum was performed on a Luminex 200 platform (Luminex Inc.,) using custom kits of pre-mixed antibody-coated beads (R&D System Inc., MN), which included the following analytes: CCL11_eotaxin, CCL13_MCP4, CCL17_TARC, CCL2_MCP1, CCL22_MDC, CCL26_EOTAXIN3, CCL5 RANTES, CCL8 MCP2, CD25_IL2Ra, CX3CL1_FRACTALKINE, CXCL1_GROa, CXCL10_IP10, CXCL11_ITAC1, CXCL13 BLC BCA1, CXCL4 PF4, CXCL5 ENA78, CXCL6_GCP2, EGF, IFNg, GMCSF, HGF, IL10, IL1ra_IL1F3, IL7, IL8_CXCL8, TRAIL, VEGFA, IL1b_IL1F2, IL5, IL6, IL-17F, IL22, IL23, TNFa, LXSAHM-01, CCL3_MIP1a, CCL4_MIP1b, FGFbasic_FGF2, GCSF, ILla II,1F1, IL2, IL4, IL-12 p70, IL13, IL15, IL-17/IL17A, CXCL12_SDF1, TGFB1, TGFB3. The assay was run based on manufacturer recommendations. Briefly, 50ml of samples together with kit standards were added to each well in duplicate and incubated with the diluted Microparticle Cocktail at 4C, overnight, on a shaker at 850rpm. Unbound soluble molecules were removed by washing the plate. The Biotin-Antibody Cocktail specific to the analytes of interest was added to each well for 1h at RT. After washing again, the Streptavidin-Phycoeriythrin conjugated was added for 30 minutes at RT. After the final washing steps, the microparticles are resuspended in kit buffer and read on a Luminex 200 platform. The outputs (pg/mL) were visualized and statistically analyzed in R upon centering and scaling using the scale function in R (SD from mean pg/mL)⁴⁴. Data were visualized together with sample annotations using the ComplexHeatmap package⁴⁵. Group comparisons were visualized as boxplots using the ggpubr package and statistically analyzed by applying the non-parametric Wilcoxon signed-rank test on the values, where significance is set at p-value < 0.05.

### Analysis of blood markers.

White blood cell counts (% of total WBC) and soluble factor data (mean-centered and scaled by SD of pg/mL) were analyzed, making group comparison on the quantities by Wilcoxon rank sum test. For the soluble factor data, important features were determined by the mean decrease in accuracy computed from random forest regression using randomForest::randomForest⁴⁶ with mtry=6 and ntree=1000. Regression was performed on data that was controlled for even patient and response representation across timepoints, selecting for unique patients in timepoints of 0, 1-4, 5-8, 9-11, and >11 months.

### Cross-data feature association.

To make associations between metagenomic features and systemic data, Fisher's exact test was used. Binary states of metagenomic features (present: >0, absent: 0 abundance) and systemic features (high: >median; low: ≤median) were transformed into a contingency table, with the odds ratio computed by Fisher's exact test.

For cross-data correlations, Spearman r coefficients were computed with rstatix::cor_test⁴⁷.

### Cross-cohort feature set enrichment analysis.

Enrichment analysis was performed by Fisher's exact test. Briefly, contingency tables were computed; binary categories for feature states were generated, by prevalence (prevalent: |OR|>1; not-prevalent: |OR|<1) for the taxonomic data and abundance (abundant: |fold-change|>1.1; not-abundant: |fold-change|<1) for the flagellin abundance data, and presence of the term in the query feature set (present: TRUE; absent: FALSE). Enrichment magnitude was determined by Fisher's exact test OR; unadjusted p-values were reported (*p<0.05).

### Statistical and analytical parameters.

All processed data were analyzed in R version 4.1.1. Statistical tests were set at a significance level of p-adj<0.05 unless otherwise stated. Multiple comparisons adjustment was performed by FDR. Statistics were computed using the rstatix package⁴⁷; p-values were adjusted for multiple comparisons by FDR using stats::p.adjust; where random sampling was performed, a seed of 1 was used (base::set.seed)⁴⁴.

### Plot visualizations.

Plots were visualized with ggplot2⁴⁸ except for heatmaps and UpSet plots, which were generated using ComplexHeatmap⁴⁵, and ridgeplots, which were generated with ggridges::geom_ridgeline⁴⁹. Statistics were annotated to figures with ggpubr::stat_pvalue_manual⁵⁰. Figures were assembled in R using grid. arrange and arrangeGrob functions from gridExtra⁵¹.

### Results

To delineate gut changes related to host response and identify gut and host factors involved, patients with advanced melanoma (n=23) were enrolled at two Italian hospitals between January 2018 and October 2022 and, along with clinical information, fecal and blood samples were collected at baseline and during single-agent anti-PD-1 treatment (Figure 1A). The objective response rate (ORR) was 56% and the disease control rate (DCR) was 74%. Cohort characteristics are30ecarized in **Table 1** and **Figure 7** and detailed in the Methods.

### Gut microbiota differences between response groups emerge during ICI therapy.

We leveraged our unique longitudinal dataset to understand if gut differences between response groups change in magnitude from baseline to ICI treatment. 16S sequencing data were generated from fecal samples collected over a 13-month long period from the start of ICI therapy (n=94, **Figure 7B** ) and taxonomic counts were transformed and batch-corrected to effectively reduce study differences at all timepoints (see Methods, **Figure 7C****).** After binning timepoints into groups with balanced number of samples (0, 2-4, 5-8, and 9-13 months), we compared the beta-diversity of the gut microbiota at each timepoint in patients stratified based on progression-free survival at twenty-four months (PFS24), measuring group differences by significance as well as magnitude (PERMANOVA pseudo-F-ratio)²⁸. We observed an increasing difference in gut diversity across therapy between patients with a PFS ≥24 months (PFS-L) and with melanoma responsive to ICI - R and PR based on the best objective response category that the patient achieved at any point following baseline (BOR) - and those with a PFS <24 months (PFS-S) and a melanoma non-responsive to the therapy (SD and PD, **Table 1** and **Figure 7A****).** This difference was not seen at baseline, was peaking in magnitude at 5-8 months and consistently significant from 5-8 to 9-13 months (Figures 1B-C). Similar trends held after controlling for subject or timepoint imbalance **(****Figure 7D****).**

To further support this finding and address inter-sequencing batch variability, we generated metagenomic data in a single run of shotgun sequencing from a subset of samples (n=65), similarly observing significant differences between PFS-L and PFS-S groups at 5-8 and 9-13 months of therapy but not at baseline **(****Figure 8A****).** In accordance, re-analysis of publicly available longitudinal metagenomic data from two independent studies whose recipients received anti-PD-1 treatment in combination with FMT^{8,10} revealed increasing differences across therapy between patients with melanoma responsive or non-responsive to ICI **(**Figure 8B-C).

Next, to determine whether the increasing group differences over therapy were primarily due to patients with melanoma responsive or non-responsive to ICI, we utilized 16S data generated in a single sequencing run from patients (n=17) and tumor-free subjects (n=14), measuring the distance of each response group relative to tumor-free subjects **(****Figure 7E****).** While at baseline the tumor-free group was equidistant to PFS-L and PFS-S groups - allowing its use as non-melanoma baseline reference - during therapy PFS-S, but not PFS-L, was significantly different from baseline (Figure 1D and **Figure 7F****).** We conclude that differences in gut microbiota diversity progressively increase between response groups during therapy and, although we cannot rule out other potential sources of variation, we propose that an increasingly diverging microbiota in patients with melanoma non-responsive to ICI is a main contributor.

### Gut variability over therapy teases out differences among responders.

A major challenge in analyzing longitudinal microbiome data is disentangling biologically relevant temporal changes from technical and individual variability. Aiming to extrapolate treatment-related features and seeing instead individual differences as a major contributor to variance in our longitudinal 16S dataset (37,88% gut microbiota variability in all samples explained by the subject, Figures 9A-B), first we controlled for inter-subject variability by measuring changes in microbiota composition in each patient with 16S data consistently available from baseline up to 13 months from the start of therapy (n=8, see Methods). However, within-patient longitudinal changes did not reveal significant treatment-related differences, even when binning timepoints differently **(**Figures 9C-F). Therefore, we stratified patients based on relative gut microbiota dissimilarity, instead, by measuring the distance of each sample at any timepoint from those of other patients (see Methods). Using the median distance across patients as cut-off for gut dissimilarity (d=246, **Figure 10A****),** we observed that patients with worse clinical outcomes had more distinctive microbiomes, with two patients with tumors in partial remission (PR, orange) despite a good survival (PFS-L) and two others who had stable diseases (SD, red) with a worse survival (PFS-S) all laying above the cut-off **(****Figure 2A****).** On the other hand, all four patients with melanoma completely responsive to ICI (CR, light blue) and PFS-L had in common the lowest dissimilarity from all other samples (p<0.05 Fisher's exact test). Thus, despite other factors may contribute to microbial variation and response to therapy, measuring relative gut distance teases out gut-based differences among patients that closely reflect disease-related factors.

We validated this approach on an external dataset of patients with melanoma that, initially refractory to ICI therapy, were treated with anti-PD-1 following FMT from donors who responded to ICI (donors=2, recipients=10)⁸. In agreement with our results, after three cycles of anti-PD-1 therapy the two patients that did not benefit from the treatment (NR, red) ranked higher than the three in remission - who received FMT from the same donor - with the only patient in complete remission ranking the lowest (CR, **Figure 2B****).** We did not see a response-associated ranking at pre-immunotherapy, instead **(****Figure 10B****).** Of note, even though assessing tumor viability in patients with a refractory disease has implicit limitations, we observed that between-patients gut variability positively correlated with tumor viability in recipients at pre-FMT (whereas post-FMT was donor-dependent, **Figures 10C** and **10D****,** respectively).

Next, considering that extreme response categories are the most objective and, therefore, are expected to be the most reliable indicators of differential biology, we investigated whether the consistency between gut and clinical status reflected real biological differences in patients with melanoma completely responsive to ICI (CR) against all the others (nCR), especially in light of the low gut dissimilarity shared by CR. First, comparing within-patient gut variability **(****Figure 2C****),** we observed significant differences between CR and nCR groups at late timepoints (8-13 months, **Figure 2D** **upper panel),** which could be explained by CR carrying more conserved taxa from baseline through treatment (Jaccard index, **Figure 2D** **bottom panel).** We validated these findings by applying the longitudinal measure on an external, publicly available longitudinal dataset of tumor-free subjects (n=9)¹⁸, observing a progressive increase in gut microbiota variability from tumor-free subjects to CR and then nCR at 8-13 months **(****Figure 10E****),** supporting that the variability of CR gut microbiota is more akin to the gut dynamic of tumor-free subjects than of nCR. In summary, while demonstrating that patients with melanoma undergoing ICI therapy can be effectively stratified based on the longitudinal analysis of their gut microbiota composition, we define low gut variability as a distinct feature of CR, which could be related to a more-effective, tumor-clearing response upon ICI treatment.

**Complete responder gut microbiota comprises a stable, Clostridia-dominated community.** Having found the gut microbiota of patients with CR melanoma distinctly less variable during ICI therapy, next we sought to identify temporally robust features related to response. We employed shotgun sequencing and metagenomic analysis on samples collected at baseline (n=14) and during therapy (n=51, **Figure 11A** ), considered within-response group sample prevalence as criterion for filtering (i.e., present in the majority of samples in a response group) and determined 80% of the samples as a cut-off lenient enough to retrieve a good number of taxa (e.g., ≥ 20% of all taxa combined) while sufficiently stringent to enrich for biologically relevant features (e.g., significantly associated with a response group, Figures 11C-D). Whereas most differentially abundant taxa were sporadic (i.e. present only at one timepoint) both in CR and nCR **(****Figure 11B****),** our prevalence approach determined stable (i.e. present at every timepoint) taxa in CR (n=25) and a few in nCR (n=7 after relaxing the prevalence criteria to at least two timepoints, **Figure 3A** and **Table 2).** We did not retrieve as many stable taxa (n=7) when grouping responder melanoma based on BOR (i.e. CR and PR vs SD and PD, **Figure 11E****),** instead, suggesting a more compositionally defined stable microbiota in CR.

When assessing the strength of association with response, we found that more stable CR taxa ranked highly by prevalence than by differential abundance (46% vs 27%, **Figure 3B****),** which is commonly used to retrieve disease-relevant taxa. Nevertheless, the top 3 stable CR in the differential abundance ranking were also among the top differentially prevalent taxa, namely *Clostridia unclassified* SGB6369, *Clostridia unclassified* SGB14951, and *Anaerostipes caccae.* CR and nCR were distinguished at 8-13 months of therapy by stable taxa **(****Figure 3C** **bottom),** particularly those retrieved at 80% prevalence **(****Figure 11F****),** and patients bearing a less variable gut had a higher proportion of stable CR taxa **(****Figure 3D****).**

Taxonomically, stable CR taxa were dominated by Clostridia (Bacillota phylum, previously Firmicutes, 77%), whereas stable nCR taxa were split between Bacteroidales (Bacteroidetes phylum, 60%) and Clostridiales (40%, **Figure 3C** **top).** However, CR and nCR were not distinguishable based on the proportion of stable taxa at phylum (Firmicutes/Bacteroidetes) or order level (Clostridiales/Bacteroidales) **(****Figure 12A****),** suggesting that species-level definition has a better discriminating power than higher level taxa. Individually, the majority of the stable CR taxa (20 out of 25) were longitudinally consistent, showing significant difference in prevalence both at baseline **(****Figure 12B****)** and during therapy **(****Figure 3E****)** across the two cohorts and, in some cases (11 out of 25), demonstrating an increase by relative abundance from baseline to late timepoints **(****Figure 12C****).**

Importantly, the definition of stable taxa implied their presence in the gut microbiota of CR since baseline. This allowed us to validate the general clinical relevance of stable CR taxa in a larger and diverse baseline dataset taken from nine external melanoma cohorts (total n=281), namely four from Europe, two from the UK and three from the USA¹¹. While previous analysis found limited reproducibility of microbiome-based signatures across these cohorts¹¹, our results demonstrated enrichment of stable CR taxa in patients with melanoma responsive to ICI (score of >2) in four of the tested studies. However, the most robust way to apply our stable CR taxa was the comparison between CR (n=29) and PD (n=118) across all nine datasets of patients with melanoma **(****Figure 3F****).**

### Stable taxa in complete responders associate with blood markers.

Interactions between the gut microbiome and host are bi-directional: while the host state can induce changes in the gut¹², features of the intestinal ecosystem can directly influence the systemic immunological state¹⁶. We extended the characterization of gut-stable patients with complete responsive melanoma to systemic level by investigating potential markers of immunomodulation. When we examined patients with paired fecal and blood samples, at baseline and on treatment (n=8), and analyzed the relationship between gut microbiota and white blood cell counts over time, we found significantly higher lymphocytes and lower neutrophils and neutrophil-to-lymphocyte ratio (NLR) during therapy in CR compared to nCR **(****Figure 4A** and **Figure 13A****),** in line with previous reports⁵⁴. The systemic state also reflected on the gut microbiota, where these blood cell markers significantly associated with stable CR taxa, such as *Clostridia spp.* And *Anaerostipes caccae* **(****Figure 4B****),** and blood lymphocytes anticorrelated with gut variability **(****Figure 13B****).** Conversely, low lymphocytes, high neutrophils and high NLR were correlated with a few stable nCR taxa **(****Figure 13C****).**

These immune cell profiles were complemented by the quantification of soluble inflammatory molecules (n=41) from serum samples (n=40), both at baseline and during therapy **(****Figure 13D****).** First, we identified five cytokines as the most important in classifying CR status: IL-12p70, which associated with CR, and CX3CL1/FRACTALKINE, IL-7, IL-8, and HGF, which associated with nCR **(****Figure 4C****).** These CR and nCR cytokines distinguished between response groups during therapy **(****Figure 13E** **bottom panel)** and were positively correlated with lymphocyte and inversely with neutrophils **(****Figure 13I**). While individually CR- and nCR-associated cytokines were not significantly correlated to within-patient gut variability **(****Figure 13F****),** pooled levels of nCR-associated cytokine showed a strong trend for a moderate positive correlation **(****Figure 13G****),** which may suggest a more overt inflammatory state in patients bearing a more variable gut microbiota. With respect to the gut microbiota, CR-associated cytokines correlated mostly with stable CR taxa **(****Figure 4D****),** while nCR-associated cytokines were correlated with only one stable nCR taxon, namely *Blautia schinkii* **(****Figure 13H****),** in line with a more variable nCR gut microbiota. In all, we show that longitudinally defined gut microbiota features associated with low variability and complete response to ICI correlate with systemic markers of immune response, most notable of which are low NLR and high IL12 p70 levels.

**CR gut microbiome is functionally distinct and stably carries metabolic and flagellin-associated genes.**

Precise mechanisms underlying gut microbiota modulation of response to cancer immunotherapy are only starting to be unraveled^{52,55}. Among potential gut effectors, preclinical evidence supports the involvement of immunomodulatory bacterial metabolites, direct stimulation of anti-tumor T-cell responses, and molecular mimicry between shared bacterial and tumor epitopes⁵⁶.

Having demonstrated compositional and taxonomic differences between the gut microbiota of CR and nCR, we sought to gain better insight into genes and pathways that were maintained in these communities by examining our longitudinal metagenomic data (n=65) at the functional level (see Methods). We re-grouped gene family abundances into KEGG orthologs (KOs) and mapped them to higher level pathways to determine over-represented functions. Among the top pathways associated with a complete response were flagellar assembly and bacterial chemotaxis as well as starch and sucrose metabolism **(****Figure 5A****),** which were consistently significant from baseline through early and late therapy timepoints. By contrast, pathways associated with nCR were more variable across time **(****Figure 14A****).** In addition, we observed some KO terms significantly associated with CR at 8-13 months **(****Figure 5B****),** which included enzymes involved in fatty acid metabolism (acetyl-CoA carboxylase), butanoate metabolism (4-hydroxybutyrate dehydrogenase) and membrane transport (MFS transporter) **(Table 3).** We conclude that patients with melanoma achieving CR carry a subset of stable metagenomic functions that distinguish them from nCR **(****Figure 14B****).**

Bacterial flagellins are well-known modulators of both innate and adaptive immunity⁵⁷⁻⁵⁹ and, since we found them among the top CR-associated stable functions, next we tested their involvement in the gut microbiota-mediated response to ICI. We saw significantly more abundant flagellin-related proteins (n=1,563 from UniRef90, see Methods) in CR, from baseline through therapy **(****Figure 5C****).** Similar trends emerged when considering the fold-change of aggregated flagellin genes in patients with melanoma responsive or non-responsive to ICI at baseline, both in our cohort and in most of the nine external cohorts we used for validation **(****Figure 14C****).** Notably, top CR-associated flagellin genes retrieved from our dataset were mostly annotated to Lachnospiraceae taxa (especially to butyrate-producer *Roseburia inulinivorans,.* **Table 4** and **Table 5),** which have been shown to play a synergistic role in host immune tolerance⁶⁰ and anti-tumor immunity⁶¹. Thus, we restricted our investigation to Lachnospiraceae-associated flagellin terms (n=391, UniRef90), obtaining significant gene set enrichment in patients with melanoma responsive to ICI in eight out of nine external cohorts we used for validation **(****Figure 5D****).** Overall, these studies demonstrate the general clinical relevance of longitudinally-defined microbiota features, which overcome limitations of previously tested microbiome-based signatures¹¹ and effectively stratify patients also across baseline cohorts.

**Reactivity toward flagellin-related peptides in patients with complete response.**

Considering that gut-residing Lachnospiraceae can be a rich source of tumor-mimicking epitopes⁴², next we searched our shortlist of top CR-associated flagellin proteins for peptides predicted to bind to HLA class I receptors (HLA-I), then matched them against a database of validated tumor-associated antigens (TAAs, n=271, see Methods). Out of the 14 CR-associated flagellin-related proteins tested, 13 encoded for 9-mer peptides with predicted affinity to HLA-I **(Table 6,** see Methods for details). Among those, three showed strong HLA-1 affinity (<100 nM) and sequence homology to four TAAs. Strikingly, three of these matching TAAs were known to associate with melanoma, namely Preferentially Expressed Antigen in Melanoma (PRAME), a melanoma-associated antigen expressed in 87% of metastatic and 83.2% of primary melanomas⁶³, Melanoma Antigen Recognized by T Cells 1 (MART-1/Melan-A), one of the oldest identified tumor antigens found in most melanomas⁶⁴⁻⁶⁵, and secerning 1 (SCRN1), a protein involved in MMP2/9 exocytosis⁶⁶⁻⁶⁷ and overexpressed in amelanotic melanoma⁶⁸. Structural prediction demonstrated high homology between the three bacterial peptides and their respective TAA in terms of conformation and predicted HLA and T cell receptor (TCR) contact points **(****Figure 6A****).** Furthermore, analysis of tumor lesions at baseline from CR (n=2) and nCR (PR n=7 and PD n=2) in our cohorts by immunohistochemistry showed preferential expression of Melan-A (p=0.033, Fisher's exact test) and PRAME (p=0.018, Fisher's exact test) In CR as compared to nCR. In particular, while CR expressed both, none of the tested antigens were detected on both PD samples **(****Figure 15A****).**

Finally, we validated experimentally the immunogenicity of flagellin-related peptides derived from the CR gut microbiota to demonstrate if they could activate a CD8-mediated immune response preferentially in patients with melanoma responsive to ICI. Specifically, we pulsed peripheral blood monocytic cells (PBMC) isolated from patients with melanoma undergoing ICI therapy (n=11) with two mix of purified peptides - namely FLA-R and FLA-G, see **Table 7** - derived from the top 14 CR-associated flagellin-related proteins (listed in **Table 6)** and we assessed CD8⁺ T cell specific reactivity using flow cytometry. Compared with patients with non-responding melanoma (n=4), CD8⁺ T cells of complete responders (n=7) demonstrated greater activation following peptide stimulation, as measured by CD25 upregulation **(****Figure 15D****)** and increased proportion of IFNγ-secreting CD8⁺ T cells in 5/7 and 6/7 of CR tested with FLA-G and FLA-R mix, respectively (peptide mix/control ratio 1.3±0.2 vs 0,6±0.1 FLA-G and 1.4± 0.2 vs 0.3±0.2 FLA-R, Figures 6B-C). While providing evidence of their immunogenicity, the differential reactivity proves that patients with melanoma responsive to ICI can specifically mount a CD8⁺ T cell-mediated response directed against tumor-mimicking, flagellin-related peptides.

### Discussion

Amidst the body of work that associates the gut microbiota to response of melanoma to ICI, to precisely identifying response-modulating features remains a challenge. Hurdles in consolidating studies include biological heterogeneity in clinical responses and the lack of data on the gut microbiota during therapy, when response modulation takes place. We address these problems by leveraging a unique longitudinal dataset of patients with melanoma followed for over one year from the start of ICI treatment, analyzing fecal and blood samples to gain novel insights into the ICI-exposed gut and the host immune state.

We determine that response group's differences increase during ICI, both in our and in other external datasets. In particular, gut diversity exhibits different dynamics relative to baseline in patients with better or worse outcome (PFS-L or PFS-S, respectively). A previous study on patients with melanoma reported that microbiota distinguishes response groups the most at around one year of treatment⁷. Here, we complement this finding showing that, following gut diversity in real-time, the microbiota in PFS-L group is more baseline-like over time, whereas in PFS-S is increasingly more variable, with significant response differences observed until 13 months from the start of therapy. While most gut microbiota studies on ICI response rely on baseline data, determining response-associated taxa at late timepoints can help confirming their true biological relevance.

Second, we demonstrate that measuring relative gut diversity distance can tease out gut differences within patients with melanoma responsive to ICI treatment. In particular, we find that CR microbiota is less variable over time and, also, more similar to each other over the course of immunotherapy. Although it cannot be used as a biomarker of response *a priori,* measuring gut variability may be developed into a perspective tool to support decision-making, either in the neoadjuvant (i.e. to determine the optimal timing for surgery) or in the adjuvant (i.e. where it could guide therapy interruption) setting of ICI therapy.

Of note, our comparison of FMT recipients pre- and during-ICI shows that gut similarity aligns with response only in the immunotherapy setting, emphasizing the role of ICI in making these gut differences emerge. However, given that positive immune response and tumor relief happen concomitantly in the course of therapy, we cannot rule out that changes observed in the gut during ICI may be due either to an overall improvement in health among responders or to other sources of variation (i.e. diet, psychological and life style factors) and clarifying causative versus confounding factors is untenable with observational data alone in such a small cohort. Nevertheless, the fact that CR bear some basal microbial peculiarities that emerge during immunotherapy supports the hypothesis that a common pressure is acting on CR and nCR that shapes differently the host environments, reinforcing differences between their gut microbiota. Notably, we find a degree of consistency among CR in terms of gut and host systemic state, which warrant us to evaluate their relation to response in larger cohorts and in future mechanistic studies. Taxonomically, our data reveal that Clostridia, which have been previously associated with response to ICI^{3,61,69-70} and a successful FMT⁷¹⁻⁷², are prevalent within CR and longitudinally consistent. These stable taxa enable to distinguish CR and nCR at late therapy timepoints (8-13 months) and are linked to distinct blood biomarkers and an overall lower-inflammatory state, except for II,-12p70. Notably, IL-12p70 is produced by dendritic cells, among others, both upon antigenic stimulation (where it is implicated in helper T-cell differentiation)⁷³ and in response to the IFNg released by anti-PD-1 activated T cells; as such, it has been associated to severe immune related adverse events (irAEs) and enhancement of anti-PD-1 response, respectively. The observation of a combination of cytokines that distinguishes CR since early timepoints supports a model where gut microbiota changes develop from pre-existing immunological differences, again prompting further research to address how this bi-directional dialogue is established and evolves.

In terms of function, we find that the CR gut metagenome stably carries genes for starch metabolism and fatty acid biosynthesis. Short-chain fatty acids (SCFAs), particularly that from Clostridia, have widely reported beneficial effects, including intestinal epithelial homeostasis⁷⁴⁻⁷⁵ and immune functions⁷⁶⁻⁸⁰. Despite the role of SCFAs in host immunity and therapy response appears ambiguous⁸¹⁻⁸², with concurrent immune-activating (driven by IFN-g and IL-12)⁸³⁻⁸⁴ and suppressive (mediated by Treg)⁸⁴ effects, a higher intake of dietary fibers - which are metabolic precursors of SCFAs - has been shown to favorably impact clinical outcome⁶⁹, and has prompted diet intervention trials (NCT04645680).

On the other hand, our functional data reveal a previously unappreciated role for flagellins in the microbiota-host crosstalk during ICI. We detect flagellin genes as significantly increased in the CR gut since baseline rather than incidentally emerging from lifestyle and diet changes during therapy, both in our patients as well as in different international cohorts. Interestingly, a large portion of the flagellins we retrieved in CR gut are carried by Lachnospiraceae, which reportedly exhibit a peculiar weak-agonist activation of TLR5 immunity⁶⁰, allowing the expression of a "silent", host-tolerated repertoire of antigens with broad tumor-mimicry potential. A recent study demonstrated the capacity of commensals in mediating a host-tolerated immunogenic response⁸⁵ and, here, we provide proof of a distinctive pre-ICI immunity directed against flagellin-derived peptides in CR, suggesting that these patients are predisposed to respond better to ICI treatment. The role of tumor antigens in ICI response is an emerging paradigm. Recent studies have shown the success of ICI on MMR-deficient (dMMR) patients that is linked, at least in part, to a higher immune "visibility" of their tumors¹¹⁻¹⁶. These results led to trials combining immunotherapy with a personalized cancer vaccine that encodes for patient-specific tumor neoantigens (NCT03897881)⁵⁴. The immunomodulatory effect of one flagellin has been also demonstrated⁵⁸ and its safety and efficacy as live biotherapy is being clinically tested on patients with cancer, alone (NCT03934827) or in combination with immunotherapy (NCT03637803). We propose that the immune response elicited by ICI therapy shapes a "host niche" favourable for gut taxa that synergistically support immune cell function and tumor recognition. Patients that carry such a beneficial gut are thus disposed to respond well to ICI and addressing how to harness such a pre-existing anti-tumor repertoire - which we demonstrate to exist - or to generate it in case of resistance to ICI may improve our ability to cure patients with melanoma and, potentially, other tumors.

### Tables

**Table 1. Breakdown of patient's characteristics. BMI body mass index; BOR best overall response (RECIST 1.1); CR complete response; PR partial response; SD stable disease; PD disease progression; OS overall survival; PFS progression free survival; DRR durable response rate (CR/PR); DCR disease control rate (CR/PR/SD).**

| **Patient's Characteristics** | | **n (tot= 23)** | **%** |
|---|---|---|---|
| **Age, years (median)** | | 54 (33-78) | |
| **Sex** | | | |
| | Male | 17 | 74% |
| | Female | 6 | 26% |
| **BMI, kg/m² (range)** | | 26.6 (23.2-33.6) | |

| **BOR (median OS and PFS, months)** | | | |
|---|---|---|---|
| | CR | 7 (108, 50) | 30% |
| | PR | 6 (104, 36) | 26% |
| | SD | 4 (53, 21) | 18% |
| | PD | 6 (25, 2) | 26% |

| **ECOG performance score** | | | |
|---|---|---|---|
| | 0-1 | 18 | 79% |
| | 2 | 3 | 13% |
| | NA | 2 | 8% |

| **Tumor stage at study entry (AJCC 8^{th} Edition)** | | | |
|---|---|---|---|
| | Unresectable stage III (M0) | 3 | 13% |
| | Skin, soft tissue ± nonregional nodes (M1a) | 1 | 4% |
| | Distant metastasis to lung (M1b) | 2 | 8% |
| | Metastases to visceral non-CNS (M1C) | 12 | 53% |
| | Metastases CNS (M1d) | 4 | 18% |
| | NA | 1 | 4% |

| **BRAF mutated** | | | |
|---|---|---|---|
| | Yes | 9 | 39% |
| | No | 12 | 53% |
| | NA | 2 | 8% |

| **Previous adjuvant therapy** | | | |
|---|---|---|---|
| | Vemurafenib | 1 | 4% |
| | Tafinlar | 1 | 4% |
| | Ipilumab | 1 | 4% |
| | Target therapy | 2 | 8% |
| **ORR** | | | 56% |
| **DCR** | | | 74% |

| **Adverse Event** | | | |
|---|---|---|---|
| | G1-G2 | 10 | 43% |
| | G3 | 1 | 4% |
| | NA | 1 | 4% |

**Table 2. Stable CR/ stable nCR taxa definition. Stable CR is defined as prevalent taxa in CR (>80% of samples) across t0, tearly, and date; stable nCR is defined as prevalent taxa in nCR across at least two timepoints.**

| **Group** | **Longitudinal overlap** | | | **List of taxa** |
|---|---|---|---|---|
| | t0 | t_2-7 months | t_8-13 months | |
| stable CR (n=25) | x | x | x | *Evtepia gabavorous* SGB15120 |
| | | | | Clostridium SGB6179 |
| | | | | *Anaerostipes caccae* SGB4529 |
| | | | | *Bacteroides nordii* SGB1858 |
| | | | | Clostridiales bacterium Choco116 SGB15149 |
| | | | | Clostridiales bacterium BX7 SGB72833 |
| | | | | Clostridia unclassified SGB14951 |
| | | | | *Candidatus Metaruminococcus gallistercoris* SGB14870 |
| | | | | *Candidatus Cryptoclostridium obscurum* SGB61016 |
| | | | | *Bifidobacterium pseudolongum* SGB17279 |
| | | | | Veillonellaceae SGB5809 group |
| | | | | Clostridia unclassified SGB6369 |
| | | | | Clostridia unclassified SGB14016 |
| | | | | Clostridia unclassified SGB6293 |
| | | | | *Eubacterium maltosivorans* SGB4078 |
| | | | | *Carnobacterium maltaromaticum* SGB7902 |
| | | | | *Candidatus Metalachnospira gallinarum* SGB5181 |
| | | | | *Candidatus Roslinia caecavium* SGB4165 |
| | | | | *Candidatus Gallimonas caecicola* SGB14041 |
| | | | | *Candidatus Heteroscilispira lomanii* SGB63278 |
| | | | | *Bifidobacterium pullorum* SGB17264 |
| | | | | Firmicutes SGB47515 |
| | | | | Bacteroidales unclassified SGB2173 |
| | | | | *Aggregatibacter sp oral taxon* 458 SGB9733 |
| | | | | Clostridiales bacterium S5_A14a SGB3977 |
| stable nCR (n=7) | x | x | x | *Alistipes sp AF17_16* SGB2326 |
| | | | | Clostridiaceae bacterium NSJ_31 SGB14839 |
| | | x | x | *Blautia schinkii* SGB4825 |
| | | | | Candidatus Saccharibacteria unclassified SGB19893 |
| | | | | Bacteroidaceae SGB1808 |
| | | | | *Bacteroides sp Marseille P3684* SGB1429 |
| | | | | *Alistipes sp An66* SGB2306 |

**Table 3. Significant KOs associated with CR at late therapy timepoints (p-adj < 0.05).**

| n | KO ID | Annotation | Associated group | LM coefficient | p-adj |
|---|---|---|---|---|---|
| 1 | K08168 | MFS transporter | CR | 3.00 | 0.00188 |
| 2 | K11263 | acetyl-CoA/propionyl-CoA carboxylase | CR | 3.20 | 0.00188 |
| 3 | K18120 | 4-hydroxybutyrate dehydrogenase | CR | 2.43 | 0.00188 |
| 4 | K09705 | hypothetical protein | CR | 3.06 | 0.00197 |

**Table 4. List of bacteria-associated flagellin gene families (UniRef90) in our dataset.**

| **UniRef90** | **log2FC** | **Entry** | **Organism** |
|---|---|---|---|
| **A0A0M6WP36** | 19,58 | A0A0M6WP36 | Roseburia inulinivorans |
| **C0FQ31** | 19,56 | C0FQ31 | Roseburia inulinivorans DSM 16841 |
| **A0A127SWS0** | 19,27 | A0A127SWS0 | uncultured bacterium |
| **C0FQ36** | 19,09 | C0FQ36 | Roseburia inulinivorans DSM 16841 |
| **A0A396AI63** | 19,09 | A0A396AI63 | Roseburia inulinivorans |
| **A0A0M6WMV3** | 19,09 | A0A0M6WMV 3 | Roseburia inulinivorans |
| **A0A0M6WD36** | 18,69 | A0A0M6WD3 6 | Roseburia inulinivorans |
| **D4KYR7** | 18,66 | D4KYR7 | Roseburia intestinalis XB6B4 |
| **R6VY20** | 18,62 | R6VY20 | Clostridium sp. CAG:91 |
| **A0A351R0D5** | 18,57 | A0A351R0D5 | Roseburia sp |
| **A0A0M6WBR8** | 18,49 | A0A0M6WBR 8 | Roseburia inulinivorans |
| **A0A0M6WYS8** | 18,34 | A0A0M6WYS 8 | Roseburia inulinivorans |
| **R6EJ07** | 18,26 | R6EJ07 | Firmicutes bacterium CAG:65 |
| **R6VQ94** | 18,17 | R6VQ94 | Clostridium sp. CAG:91 |
| **G2SZK1** | 18,11 | G2SZK1 | Roseburia hominis (strain DSM 16839 / JCM 17582 / NCIMB 14029 / A2-183) |
| **C7G5T8** | 18,10 | C7G5T8 | Roseburia intestinalis L1-82 |
| **ASZ847** | 18,06 | A5Z847 | Eubacterium ventriosum ATCC 27560 |
| **A0A174K7I7** | 18,02 | A0A174K7I7 | Dorea longicatena |
| **A0A1Q6SAN9** | 17,85 | A0A1Q6SAN9 | Roseburia intestinalis |
| **D4KYS1** | 17,83 | D4KYS1 | Roseburia intestinalis XB6B4 |
| **D4KW84** | 17,81 | D4KW84 | Roseburia intestinalis XB6B4 |
| **R6EJJ6** | 17,72 | R6EJJ6 | Firmicutes bacterium CAG:65 |
| **R6EC52** | 17,69 | R6EC52 | Firmicutes bacterium CAG:65 |
| **A0A395V8C5** | 17,63 | A0A395V8C5 | Roseburia hominis |
| **R6EJJ3** | 17,45 | R6EJJ3 | Firmicutes bacterium CAG:65 |
| **R5SPI8** | 17,40 | R5SPI8 | Clostridium sp. CAG:75 |
| **R5T8V3** | 17,40 | R5T8V3 | Clostridium sp. CAG:75 |
| **D4RW24** | 17,35 | D4RW24 | Butyrivibrio crossotus DSM 2876 |
| **RST617** | 17,34 | R5T617 | Clostridium sp. CAG:75 |
| **A0A373K755** | 17,30 | A0A373K755 | Clostridium sp. AF46-9NS |
| **R6X2U9** | 17,28 | R6X2U9 | Clostridium sp. CAG:91 |
| **G2T3F2** | 17,22 | G2T3F2 | Roseburia hominis (strain DSM 16839 / JCM 17582 / NCIMB 14029 / A2-183) |
| **G2SZJ5** | 17,19 | G2SZJ5 | Roseburia hominis (strain DSM 16839 / JCM 17582 / NCIMB 14029 / A2-183) |
| **D4KYS2** | 17,18 | D4KYS2 | Roseburia intestinalis XB6B4 |
| **R6NV68** | 17,13 | R6NV68 | Roseburia sp. CAG:45 |
| **R5T127** | 17,11 | R5T127 | Clostridium sp. CAG:75 |
| **A0A395V4R6** | 17,11 | A0A395V4R6 | Roseburia hominis |
| **C4Z282** | 17,06 | C4Z282 | Lachnospira eligens (strain ATCC 27750 / DSM 3376 / VPI C15-48 / C15-B4) (Eubacterium eligens) |
| **A0A395VCZ3** | 17,04 | A0A395VCZ3 | Roseburia hominis |
| **A0A174BIC3** | 16,84 | A0A174BIC3 | Faecalibacterium prausnitzii |
| **R5SNW4** | 16,83 | R5SNW4 | Clostridium sp. CAG:75 |
| **A0A395VAJ9** | 16,82 | A0A395VAJ9 | Roseburia hominis |
| **A0A174ZTA0** | 16,80 | A0A174ZTA0 | Lachnospira eligens |
| **A0A396ABL6** | 16,80 | A0A396ABL6 | Roseburia inulinivorans |
| **A0A3E4X9M7** | 16,77 | A0A3E4X9M7 | [Eubacterium] rectale |
| **R6NRZ0** | 16,76 | R6NRZ0 | Roseburia sp. CAG:45 |
| **R5SHJ2** | 16,65 | R5SHJ2 | Clostridium sp. CAG:75 |
| **RSE9Z9** | 16,57 | R5E9Z9 | Eubacterium sp. CAG:86 |
| **R7C6Q7** | 16,52 | R7C6Q7 | Clostridium sp. CAG:62 |
| **A0A3D5WGL2** | 16,52 | A0A3D5WGL2 | Lachnospiraceae bacterium |
| **D7GSU0** | 16,49 | D7GSU0 | butyrate-producing bacterium SS3/4 |
| **D7GSR3** | 16,44 | D7GSR3 | butyrate-producing bacterium SS3/4 |
| **D7GSR2** | 16,42 | D7GSR2 | butyrate-producing bacterium SS3/4 |
| **A0A173WCI3** | 16,40 | A0A173WCI3 | Roseburia inulinivorans |
| **A0A1C5P5U9** | 16,39 | A0A1C5P5U9 | uncultured Blautia sp |
| **R6EJS1** | 16,37 | R6EJS1 | Firmicutes bacterium CAG:65 |
| **R5WFA2** | 16,15 | R5WFA2 | Clostridium sp. CAG:167 |
| **R9M386** | 16,13 | R9M386 | Oscillibacter sp. 1-3 |
| **A0A174N4P2** | 16,06 | A0A174N4P2 | Lachnospira pectinoschiza |
| **D7GST1** | 15,89 | D7GST1 | butyrate-producing bacterium SS3/4 |
| **R6P646** | 15,88 | R6P646 | Roseburia sp. CAG:45 |
| **A0A174LQE7** | 15,88 | A0A174LQE7 | Lachnospira pectinoschiza |
| **A0A174YXY8** | 15,82 | A0A174YXY8 | Lachnospira eligens |
| **D7GSS4** | 15,78 | D7GSS4 | butyrate-producing bacterium SS3/4 |
| **R7CCH1** | 15,77 | R7CCH1 | Clostridium sp. CAG:62 |
| **D7GSS8** | 15,71 | D7GSS8 | butyrate-producing bacterium SS3/4 |
| **A0A1C5WBB9** | 15,60 | A0A1C5WBB9 | uncultured Ruminococcus sp |
| **A0A1C5KUA4** | 15,45 | A0A1C5KUA4 | uncultured Clostridium sp |
| **D4RY26** | 15,41 | D4RY26 | Butyrivibrio crossotus DSM 2876 |
| **J5GJA7** | 15,41 | J5GJA7 | Streptococcus oralis SK304 |
| **R6NM46** | 15,36 | R6NM46 | Roseburia sp. CAG:45 |
| **A0A1C5RKN2** | 15,33 | A0A1C5RKN2 | uncultured Clostridium sp |
| **R7NFE3** | 15,18 | R7NFE3 | Eubacterium sp. CAG:76 |
| **A0A174YYK7** | 15,12 | A0A174YYK7 | Lachnospira eligens |
| **RSE067** | 15,08 | R5E067 | Eubacterium sp. CAG:86 |
| **A2V703** | 15,01 | A2V703 | Escherichia coli |
| **R5DRV5** | 14,91 | R5DRV5 | Eubacterium sp. CAG:86 |
| **A0A0K4GI28** | 14,79 | A0A0K4GI28 | Escherichia coli |
| **A0A174MPU4** | 14,77 | A0A174MPU4 | Lachnospira pectinoschiza |
| **A0A376JIF4** | 14,53 | A0A376JIF4 | Escherichia coli |
| **RSDRV9** | 14,52 | R5DRV9 | Eubacterium sp. CAG:86 |
| **R6NJG6** | 14,40 | R6NJG6 | Roseburia sp. CAG:45 |
| **A0A285PW02** | 14,07 | A0A285PW02 | Anaerobutyricum hallii |
| **C0FTT4** | 13,86 | C0FTT4 | Roseburia inulinivorans DSM 16841 |
| **A0A2X1MZ58** | 12,08 | A0A2X1MZ58 | Escherichia coli |
| **R6VWT3** | 4,43 | R6VWT3 | Clostridium sp. CAG:91 |
| **A0A174DCE4** | 4,42 | A0A174DCE4 | [Eubacterium] rectale |
| **A0A174I7J5** | 4,20 | A0A174I7J5 | [Eubacterium] rectale |
| **C4ZAM7** | 3,94 | C4ZAM7 | Agathobacter rectalis (strain ATCC 33656 / DSM 3377 / JCM 17463 / KCTC 5835 / VPI 0990) (Eubacterium rectale) |
| **C4Z9R8** | 3,75 | C4Z9R8 | Agathobacter rectalis (strain ATCC 33656 / DSM 3377 / JCM 17463 / KCTC 5835 / VPI 0990) (Eubacterium rectale) |
| **A0A2U2EKC6** | 3,67 | A0A2U2EKC6 | [Eubacterium] rectale |
| **R6EL64** | 3,19 | R6EL64 | Firmicutes bacterium CAG:65 |
| **A0A395UYP9** | 3,08 | A0A395UYP9 | [Eubacterium] rectale |
| **A0A2U2MF49** | 3,08 | A0A2U2MF49 | Streptococcus thermophilus |
| **R6EHH0** | 2,91 | R6EHH0 | Firmicutes bacterium CAG:65 |
| **A0A396FMF8** | 2,90 | A0A396FMF8 | [Eubacterium] rectale |
| **A0A0M6WBY2** | 2,86 | A0A0M6WBY 2 | Roseburia inulinivorans |
| **A0A174Q4T5** | 2,22 | A0A174Q4T5 | Dorea longicatena |
| **B7ART7** | 2,14 | B7ART7 | [Bacteroides] pectinophilus ATCC 43243 |
| **A0A396FGM9** | 2,03 | A0A396FGM9 | [Eubacterium] rectale |
| **A0A396FKV9** | 2,02 | A0A396FKV9 | [Eubacterium] rectale |
| **R6ESB9** | 1,99 | R6ESB9 | Firmicutes bacterium CAG:65 |
| **RSTD65** | 1,72 | R5TD65 | Clostridium sp. CAG:75 |
| **R6BIK6** | 1,61 | R6BIK6 | Firmicutes bacterium CAG:56 |
| **C7GHZ7** | 1,53 | C7GHZ7 | Roseburia intestinalis L1-82 |
| **A0A174AGF2** | 1,48 | A0A174AGF2 | Coprococcus comes |
| **R5SHJ0** | 1,33 | R5SHJ0 | Clostridium sp. CAG:75 |
| **P0AEM4** | 1,32 | P0AEM4 | Escherichia coli (strain K12) |
| **A0A174YHS0** | 1,31 | A0A174YHS0 | Lachnospira eligens |
| **A0A3E5AME9** | 1,28 | A0A3E5AME9 | [Eubacterium] rectale |

**Table 5. Top CR-associated flagellin gene families.**

| n | **Bacterial flagellin marker** | **log2FC** | **Percentile rank** | **Organism** |
|---|---|---|---|---|
| 1 | UniRef90_A0A0M6WP36 | 19.58 | 100 | *Roseburia inulinivorans* |
| 2 | UniRef90_C0FQ31 | 19.56 | 99 | *Roseburia inulinivorans* DSM 16841 |
| 3 | UniRef90_A0A127SWS0 | 19.26 | 98 | uncultured bacterium |
| 4 | UniRef90_C0FQ36 | 19.093 | 97 | *Roseburia inulinivorans* DSM 16841 |
| 5 | UniRef90_A0A396AI63 | 19.092 | 96 | *Roseburia inulinivorans* |
| 6 | UniRef90_A0A0M6WMV3 | 19.087 | 95 | *Roseburia inulinivorans* |
| 7 | UniRef90_A0A0M6WD36 | 18.69 | 94 | *Roseburia inulinivorans* |
| 8 | UniRef90_D4KYR7 | 18.66 | 93 | *Roseburia intestinalis* XB6B4 |
| 9 | UniRef90_R6VY20 | 18.62 | 92 | *Clostridium sp.* CAG:91 |
| 10 | UniRef90_A0A351R0DS | 18.57 | 91 | *Roseburia sp.* |
| 11 | UniRef90_A0A0M6WBR8 | 18.49 | 90 | *Roseburia inulinivorans* |
| 12 | UniRef90_A0A0M6WYS8 | 18.34 | 89 | *Roseburia inulinivorans* |
| 13 | UniRef90_R6EJ07 | 18.25 | 88 | Firmicutes bacterium CAG:65 |
| 14 | UniRef90_R6VQ94 | 18.17 | 87 | *Clostridium sp.* CAG:91 |

**Table 6. List of predicted epitopes for top CR-associated flagellin-related gene families.**

| **UniRef90** | **log2FC** | **Posi tion** | **Peptide** | **MHC** | **Aff (nM)** | **Stron g Bindi ng status** | **Affini ty final** | **Tumor Antigen match** |
|---|---|---|---|---|---|---|---|---|
| A0A0M6WMV3 | 19,0872451 | 264 | MPKDGAAFI (SEQ ID N.2) | HLA-B*07:02 | 9313 | SB | 93.13 | Melan-A/MART -1 |
| A0A0M6WD36 | 18,6908699 | 130 | KMSYEDIEL (SEQ ID N.1) | HLA-A*02:01 | 7169 | SB | 71.69 | secemin 1 |
| A0A0M6WP36 | 19,5804199 | 63 | GLDALNNLL (SEQ IDN.10) | HLA-A*02:01 | 5179 | SB | 51.79 | PRAME |
| A0A0M6WP36 | 19,5804199 | 63 | GLDALNNLL (SEQ IDN.10) | HLA-A*02:01 | 5179 | SB | 51.79 | PAP |
| A0A0M6WBR8 | 18,4868315 | 317 | GEDYLAQKI (SEQ ID N.116) | HLA-B*40:01 | 8071 | SB | NA | no |
| A0A0M6WBR8 | 18,4868315 | 360 | LYQAVAEIL (SEQ ID N.106) | HLA-A*24:02 | 7788 | SB | NA | no |
| A0A0M6WBR8 | 18,4868315 | 190 | GHAQDIFIL (SEQ ID N.112) | HLA-B*39:01 | 7472 | SB | NA | no |
| A0A0M6WBR8 | 18,4868315 | 229 | GLADFIYQK (SEQ ID N.105) | HLA-A*03:01 | 6289 | SB | NA | no |
| A0A0M6WBR8 | 18,4868315 | 334 | IEIVENKPL (SEQ ID N.115) | HLA-B*40:01 | 5009 | SB | NA | no |
| A0A0M6WBR8 | 18,4868315 | 3 | QQKQLHLIM (SEQ ID N.110) | HLA-B*15:01 | 4011 | SB | NA | no |
| A0A0M6WBR8 | 18,4868315 | 320 | YLAQKIKEA (SEQ ID N.104) | HLA-A*02:01 | 3406 | SB | NA | no |
| A0A0M6WBR8 | 18,4868315 | 5 | KQLHLIMEW (SEQ ID N.119) | HLA-B*58:01 | 3285 | SB | NA | no |
| A0A0M6WBR8 | 18,4868315 | 230 | LADFIYQKY (SEQ ID N.99) | HLA-A*01:01 | 3148 | SB | NA | no |
| A0A0M6WBR8 | 18,4868315 | 130 | LISVIQVGW (SEQ ID N.118) | HLA-B*58:01 | 2754 | SB | NA | no |
| A0A0M6WBR8 | 18,4868315 | 160 | RIFSKDSIF (SEQ ID N.109) | HLA-B*15:01 | 2709 | SB | NA | no |
| A0A0M6WBR8 | 18,4868315 | 47 | KSKELTAAF (SEQ ID N.108) | HLA-B*15:01 | 2132 | SB | NA | no |
| A0A0M6WBR8 | 18,4868315 | 49 | KELTAAFDL (SEQ ID N.114) | HLA-B*40:01 | 1881 | SB | NA | no |
| A0A0M6WBR8 | 18,4868315 | 279 | RMMQDVPK A (SEQ ID N.103) | HLA-A*02:01 | 1821 | SB | NA | no |
| A0A0M6WBR8 | 18,4868315 | 75 | LMNSFVDIY (SEQ ID N.107) | HLA-B*15:01 | 1626 | SB | NA | no |
| A0A0M6WBR8 | 18,4868315 | 199 | YEIPLMQAI (SEQ ID N.113) | HLA-B*40:01 | 1308 | SB | NA | no |
| A0A0M6WBR8 | 18,4868315 | 278 | RRMMQDVP K (SEQ ID N.111) | HLA-B*27:05 | 1302 | SB | NA | no |
| A0A0M6WBR8 | 18,4868315 | 125 | FVVTALISV (SEQ ID N.102) | HLA-A*02:01 | 1142 | SB | NA | no |
| A0A0M6WBR8 | 18,4868315 | 9 | LIMEWDLQW (SEQ ID N.117) | HLA-B*58:01 | 1052 | SB | NA | no |
| A0A0M6WBR8 | 18,4868315 | 105 | LMSDALIQI (SEQ ID N.101) | HLA-A*02:01 | 820 | SB | NA | no |
| A0A0M6WBR8 | 18,4868315 | 363 | AVAEILAMV (SEQ ID N.100) | HLA-A*02:01 | 789 | SB | NA | no |
| A0A0M6WD36 | 18,6908699 | 125 | FSDEGKMSY (SEQ ID N.56) | HLA-A*01:01 | 514 | SB | NA | no |
| A0A0M6WD36 | 18,6908699 | 78 | KLCDIYSEL (SEQ ID N.57) | HLA-A*02:01 | 2422 | SB | NA | no |
| A0A0M6WD36 | 18,6908699 | 289 | VLAKAQFAI (SEQ ID N.58) | HLA-A*02:01 | 3387 | SB | NA | no |
| A0A0M6WD36 | 18,6908699 | 319 | KLDDRIANA (SEQ ID N.59) | HLA-A*02:01 | 4453 | SB | NA | no |
| A0A0M6WD36 | 18,6908699 | 348 | ISFDEAFHV (SEQ ID N.60) | HLA-A*02:01 | 7072 | SB | NA | no |
| A0A0M6WD36 | 18,6908699 | 2 | ILRVPSYYK (SEQ ID N.61) | HLA-A*03:01 | 1154 | SB | NA | no |
| A0A0M6WD36 | 18,6908699 | 10 | KTFQCIADK (SEQ ID N.62) | HLA-A*03:01 | 2857 | SB | NA | no |
| A0A0M6WD36 | 18,6908699 | 7 | SYYKTFQCI (SEQ ID N.63) | HLA-A*24:02 | 1409 | SB | NA | no |
| A0A0M6WD36 | 18,6908699 | 310 | RYFANGKSF (SEQ ID N.64) | HLA-A*24:02 | 2389 | SB | NA | no |
| A0A0M6WD36 | 18,6908699 | 38 | YYMGIEGAF (SEQ ID N.65) | HLA-A*24:02 | 3145 | SB | NA | no |
| A0A0M6WD36 | 18,6908699 | 216 | YFHVRMETF (SEQ ID N.66) | HLA-A*24:02 | 3931 | SB | NA | no |
| A0A0M6WD36 | 18,6908699 | 273 | VYFTYRYFM (SEQ ID N.67) | HLA-A*24:02 | 4091 | SB | NA | no |
| A0A0M6WD36 | 18,6908699 | 4 | RVPSYYKTF (SEQ ID N.68) | HLA-A*24:02 | 8292 | SB | NA | no |
| A0A0M6WD36 | 18,6908699 | 107 | DVREKSLSV (SEQ ID N.69) | HLA-B*08:01 | 8064 | SB | NA | no |
| A0A0M6WD36 | 18,6908699 | 1 | MILRVPSYY (SEQ ID N.70) | HLA-B* 15:01 | 9059 | SB | NA | no |
| A0A0M6WD36 | 18,6908699 | 90 | ALCEVCTEY (SEQ ID N.71) | HLA-B* 15:01 | 9566 | SB | NA | no |
| A0A0M6WD36 | 18,6908699 | 277 | YRYFMRAW Y (SEQ ID N.72) | HLA-B*27:05 | 3174 | SB | NA | no |
| A0A0M6WD36 | 18,6908699 | 170 | YRTQAVAIL (SEQ ID N.73) | HLA-B*39:01 | 1877 | SB | NA | no |
| A0A0M6WD36 | 18,6908699 | 219 | VRMETFDEL (SEQ ID N.74) | HLA-B*39:01 | 3977 | SB | NA | no |
| A0A0M6WD36 | 18,6908699 | 264 | NEIWYEHIL (SEQ ID N.75) | HLA-B*40:01 | 1954 | SB | NA | no |
| A0A0M6WD36 | 18,6908699 | 133 | YEDIELPDL (SEQ ID N.76) | HLA-B*40:01 | 8528 | SB | NA | no |
| A0A0M6WD36 | 18,6908699 | 42 | IEGAFGERL (SEQ ID N.77) | HLA-B*40:01 | 8801 | SB | NA | no |
| A0A0M6WD36 | 18,6908699 | 259 | KSGDYNEIW (SEQ ID N.78) | HLA-B*58:01 | 687 | SB | NA | no |
| A0A0M6WMV3 | 19,0872451 | 191 | TSDTKQYTY (SEQ ID N.43) | HLA-A*01:01 | 1498 | SB | NA | no |
| A0A0M6WMV3 | 19,0872451 | 507 | GMLNQTTLL (SEQ ID N.44) | HLA-A*02:01 | 3240 | SB | NA | no |
| A0A0M6WMV3 | 19,0872451 | 345 | YIIAVNQTL (SEQ ID N.45) | HLA-A*02:01 | 3924 | SB | NA | no |
| A0A0M6WMV3 | 19,0872451 | 465 | RMSNQQLTV (SEQ ID N.46) | HLA-A*02:01 | 6559 | SB | NA | no |
| A0A0M6WMV3 | 19,0872451 | 258 | AVKAGNMP K (SEQ ID N.47) | HLA-A*03:01 | 3571 | SB | NA | no |
| A0A0M6WMV3 | 19,0872451 | 180 | KTAEEMSQK (SEQ ID N.48) | HLA-A*03:01 | 4778 | SB | NA | no |
| A0A0M6WMV3 | 19,0872451 | 339 | IYQDIDYII (SEQ ID N.49) | HLA-A*24:02 | 1456 | SB | NA | no |
| A0A0M6WMV3 | 19,0872451 | 488 | IIIDYTAAY (SEQ ID N.50) | HLA-A*26:01 | 3745 | SB | NA | no |
| A0A0M6WMV3 | 19,0872451 | 488 | IIIDYTAAY (SEQ ID N.50) | HLA-B* 15:01 | 1626 | SB | NA | no |
| A0A0M6WMV3 | 19,0872451 | 1 | MRVTNNMM L (SEQ ID N.51) | HLA-B*27:05 | 5858 | SB | NA | no |
| A0A0M6WMV3 | 19,0872451 | 139 | YRTNCKLTF (SEQ ID N.52) | HLA-B*27:05 | 7368 | SB | NA | no |
| A0A0M6WMV3 | 19,0872451 | 1 | MRVTNNMM L (SEQ ID N.51) | HLA-B*39:01 | 2465 | SB | NA | no |
| A0A0M6WMV3 | 19,0872451 | 177 | VELKTAEEM (SEQ ID N.53) | HLA-B*40:01 | 9926 | SB | NA | no |
| A0A0M6WMV3 | 19,0872451 | 404 | SAQENLQTW (SEQ ID N.54) | HLA-B*58:01 | 1945 | SB | NA | no |
| A0A0M6WMV3 | 19,0872451 | 224 | KTGTLSYHY (SEQ ID N.55) | HLA-B*58:01 | 6810 | SB | NA | no |
| A0A0M6WYS8 | 18,3445463 | 240 | FTGLQASRY (SEQ ID N. 120) | HLA-A*01:01 | 6039 | SB | NA | no |
| A0A0M6WYS8 | 18,3445463 | 410 | ALDDAIAMV (SEQ ID N.121) | HLA-A*02:01 | 620 | SB | NA | no |
| A0A0M6WYS8 | 18,3445463 | 320 | SIFSSTATV (SEQ ID N. 122) | HLA-A*02:01 | 3822 | SB | NA | no |
| A0A0M6WYS8 | 18,3445463 | 79 | ALNETSAIL (SEQ ID N.3) | HLA-A*02:01 | 6747 | SB | 67.47 | no |
| A0A0M6WYS8 | 18,3445463 | 51 | KMQAQIDAL (SEQ ID N.123) | HLA-A*02:01 | 7225 | SB | NA | no |
| A0A0M6WYS8 | 18,3445463 | 253 | SLVLTFSGK (SEQ ID N. 124) | HLA-A*03:01 | 5695 | SB | NA | no |
| A0A0M6WYS8 | 18,3445463 | 6 | NMSAVITNK (SEQ ID N. 125) | HLA-A*03:01 | 6444 | SB | NA | no |
| A0A0M6WYS8 | 18,3445463 | 168 | TIKQPAATK (SEQ ID N. 126) | HLA-A*03:01 | 9553 | SB | NA | no |
| A0A0M6WYS8 | 18,3445463 | 82 | ETSAILQRM (SEQ ID N. 127) | HLA-A*26:01 | 2882 | SB | NA | no |
| A0A0M6WYS8 | 18,3445463 | 457 | DMAEEMTN Y (SEQ ID N.128) | HLA-A*26:01 | 8659 | SB | NA | no |
| A0A0M6WYS8 | 18,3445463 | 485 | LPQNVLSLL (SEQ ID N. 129) | HLA-B*07:02 | 5410 | SB | NA | no |
| A0A0M6WYS8 | 18,3445463 | 87 | LQRMRELSV (SEQ ID N.130) | HLA-B*08:01 | 3136 | SB | NA | no |
| A0A0M6WYS8 | 18,3445463 | 471 | LQQAGVSVL (SEQ ID N.131) | HLA-B* 15:01 | 2543 | SB | NA | no |
| A0A0M6WYS8 | 18,3445463 | 250 | SSASLVLTF (SEQ ID N.135) | HLA-B* 15:01 | 3064 | SB | NA | no |
| A0A0M6WYS8 | 18,3445463 | 246 | SRYGSSASL (SEQ ID N. 132) | HLA-B*39:01 | 3669 | SB | NA | no |
| A0A0M6WYS8 | 18,3445463 | 305 | VELSAGTAI (SEQ ID N.133) | HLA-B*40:01 | 2275 | SB | NA | no |
| A0A0M6WYS8 | 18,3445463 | 128 | TEYNSKSLL (SEQ ID N. 134) | HLA-B*40:01 | 3400 | SB | NA | no |
| A0A0M6WYS8 | 18,3445463 | 250 | SSASLVLTF (SEQ ID N.135) | HLA-B*58:01 | 1215 | SB | NA | no |
| A0A127SWS0 | 19,2651367 | 77 | STEKLSSGY (SEQ ID N.11) | HLA-A*01:01 | 3878 | SB | NA | no |
| A0A127SWS0 | 19,2651367 | 65 | RMLNVTTSA (SEQ ID N.12) | HLA-A*02:01 | 4128 | SB | NA | no |
| A0A127SWS0 | 19,2651367 | 130 | ALTEVHSML (SEQ ID N.13) | HLA-A*02:01 | 6759 | SB | NA | no |
| A0A127SWS0 | 19,2651367 | 136 | SMLQRMNEL (SEQ IDN.14) | HLA-A*02:01 | 8385 | SB | NA | no |
| A0A127SWS0 | 19,2651367 | 133 | EVHSMLQRM (SEQ ID N.15) | HLA-A*26:01 | 4276 | SB | NA | no |
| A0A127SWS0 | 19,2651367 | 136 | SMLQRMNEL (SEQ ID N.14) | HLA-B*08:01 | 3430 | SB | NA | no |
| A0A351R0D5 | 18,5681348 | 131 | RIRDTDMAK (SEQ ID N.96) | HLA-A*03:01 | 5560 | SB | NA | no |
| A0A351R0D5 | 18,5681348 | 96 | KVSSQRSAL (SEQ ID N.97) | HLA-B*07:02 | 2328 | SB | NA | no |
| A0A351R0D5 | 18,5681348 | 149 | ILAQAGQSM (SEQ ID N.4) | HLA-B* 15:01 | 3257 | SB | 32.57 | no |
| A0A351R0D5 | 18,5681348 | 149 | ILAQAGQSM (SEQ ID N.4) | HLA-B* 15:01 | 3257 | SB | 32.57 | no |
| A0A351R0D5 | 18,5681348 | 36 | VKSSLAFSL (SEQ ID N.98) | HLA-B*39:01 | 9091 | SB | NA | no |
| A0A396AI63 | 19,092699 | 117 | TLMPCCPAV (SEQ ID N.22) | HLA-A*02:01 | 462 | SB | NA | no |
| A0A396AI63 | 19,092699 | 255 | YLNPVIEKA (SEQ ID N.23) | HLA-A*02:01 | 1746 | SB | NA | no |
| A0A396AI63 | 19,092699 | 386 | YLENSFEEL (SEQ ID N.24) | HLA-A*02:01 | 3714 | SB | NA | no |
| A0A396AI63 | 19,092699 | 352 | WLLDGQKEI (SEQ ID N.25) | HLA-A*02:01 | 4562 | SB | NA | no |
| A0A396AI63 | 19,092699 | 302 | QAYNPLMRK (SEQ ID N.26) | HLA-A*03:01 | 2645 | SB | NA | no |
| A0A396AI63 | 19,092699 | 102 | VFPREVHRF (SEQ ID N.27) | HLA-A*24:02 | 1929 | SB | NA | no |
| A0A396AI63 | 19,092699 | 289 | EMADRMHEF (SEQ ID N.28) | HLA-A*26:01 | 2276 | SB | NA | no |
| A0A396AI63 | 19,092699 | 49 | LPQKKNLSA (SEQ ID N.29) | HLA-B*07:02 | 8816 | SB | NA | no |
| A0A396AI63 | 19,092699 | 161 | YIREKLTEL (SEQ ID N.30) | HLA-B*08:01 | 1133 | SB | NA | no |
| A0A396AI63 | 19,092699 | 305 | NPLMRKFLI (SEQ ID N.31) | HLA-B*08:01 | 4896 | SB | NA | no |
| A0A396AI63 | 19,092699 | 293 | RMHEFESEF (SEQ ID N.32) | HLA-B* 15:01 | 991 | SB | NA | no |
| A0A396AI63 | 19,092699 | 95 | VLSETCAVF (SEQ ID N.33) | HLA-B* 15:01 | 1311 | SB | NA | no |
| A0A396AI63 | 19,092699 | 83 | KLCKLVTAY (SEQ ID N.34) | HLA-B* 15:01 | 3621 | SB | NA | no |
| A0A396AI63 | 19,092699 | 153 | FLREHARFY (SEQ ID N.35) | HLA-B* 15:01 | 3982 | SB | NA | no |
| A0A396AI63 | 19,092699 | 350 | LRWLLDGQK (SEQ ID N.36) | HLA-B*27:05 | 3497 | SB | NA | no |
| A0A396AI63 | 19,092699 | 158 | ARFYIREKL (SEQ ID N.37) | HLA-B*27:05 | 5458 | SB | NA | no |
| A0A396AI63 | 19,092699 | 252 | YRNYLNPVI (SEQ ID N.38) | HLA-B*39:01 | 3813 | SB | NA | no |
| A0A396AI63 | 19,092699 | 299 | SEFQAYNPL (SEQ ID N.39) | HLA-B*40:01 | 482 | SB | NA | no |
| A0A396AI63 | 19,092699 | 146 | SEQTENPFL (SEQ ID N.40) | HLA-B*40:01 | 3809 | SB | NA | no |
| A0A396AI63 | 19,092699 | 314 | NEFNADLLM (SEQ ID N.6) | HLA-B*40:01 | 5577 | SB | 55.77 | no |
| A0A396AI63 | 19,092699 | 163 | REKLTELFL (SEQ ID N.41) | HLA-B*40:01 | 6818 | SB | NA | no |
| A0A396AI63 | 19,092699 | 328 | ESLLVQYQW (SEQ ID N.42) | HLA-B*58:01 | 1172 | SB | NA | no |
| C0FQ36 | 19,0939629 | 95 | VLTTVRVPK (SEQ ID N.16) | HLA-A*03:01 | 3795 | SB | NA | no |
| C0FQ36 | 19,0939629 | 116 | IIINMSNNK (SEQ ID N.17) | HLA-A*03:01 | 9565 | SB | NA | no |
| C0FQ36 | 19,0939629 | 88 | MKQEDTFVL (SEQ ID N.18) | HLA-B*39:01 | 1172 | SB | NA | no |
| C0FQ36 | 19,0939629 | 46 | SHIMWLQSM (SEQ IDN.19) | HLA-B*39:01 | 2117 | SB | NA | no |
| C0FQ36 | 19,0939629 | 28 | FPDMQKFTL (SEQ ID N.20) | HLA-B*39:01 | 6076 | SB | NA | no |
| C0FQ36 | 19,0939629 | 42 | KGSKSHIMW (SEQ ID N.21) | HLA-B*58:01 | 1701 | SB | NA | no |
| D4KYR7 | 18,6596352 | 90 | RLDSGNYNV (SEQ ID N.79) | HLA-A*02:01 | 869 | SB | NA | no |
| D4KYR7 | 18,6596352 | 106 | KLLEKYNAL (SEQ ID N.80) | HLA-A*02:01 | 1579 | SB | NA | no |
| D4KYR7 | 18,6596352 | 10 | KTRKRISRK (SEQ ID N.81) | HLA-A*03:01 | 6237 | SB | NA | no |
| D4KYR7 | 18,6596352 | 21 | DAIMRVEAY (SEQ ID N.82) | HLA-A*26:01 | 9418 | SB | NA | no |
| R6EJ07 | 18,2552288 | 318 | TSENMTAAY (SEQ ID N.136) | HLA-A*01:01 | 1534 | SB | NA | no |
| R6EJ07 | 18,2552288 | 79 | AMTEISEML (SEQ ID N.85) | HLA-A*02:01 | 1972 | SB | NA | no |
| R6EJ07 | 18,2552288 | 134 | KLLNGEFDL (SEQ ID N. 13 7) | HLA-A*02:01 | 1982 | SB | NA | no |
| R6EJ07 | 18,2552288 | 254 | VLEVNIPAV (SEQ ID N.87) | HLA-A*02:01 | 4757 | SB | NA | no |
| R6EJ07 | 18,2552288 | 51 | RMNAQIEGL (SEQ ID N.88) | HLA-A*02:01 | 8596 | SB | NA | no |
| R6EJ07 | 18,2552288 | 333 | DMAEEMTSY (SEQ ID N.90) | HLA-A*26:01 | 5239 | SB | NA | no |
| R6EJ07 | 18,2552288 | 361 | RPSQVLQLL (SEQ ID N.91) | HLA-B*07:02 | 1805 | SB | NA | no |
| R6EJ07 | 18,2552288 | 296 | SVRGRLGAF (SEQ ID N.8) | HLA-B*07:02 | 7258 | SB | 66.44 | no |
| R6EJ07 | 18,2552288 | 296 | SVRGRLGAF (SEQ ID N.8) | HLA-B*07:02 | 7258 | SB | 72.58 | no |
| R6EJ07 | 18,2552288 | 85 | EMLQRINEL (SEQ ID N.92) | HLA-B*08:01 | 2754 | SB | NA | no |
| R6EJ07 | 18,2552288 | 346 | ILAQAGTSM (SEQ ID N.5) | HLA-B*15:01 | 2443 | SB | 24.43 | no |
| R6EJ07 | 18,2552288 | 346 | ILAQAGTSM (SEQ ID N.5) | HLA-B*15:01 | 2443 | SB | 24.43 | no |
| R6EJ07 | 18,2552288 | 346 | ILAQAGTSM (SEQ ID N.5) | HLA-B*15:01 | 2443 | SB | 24.43 | no |
| R6EJ07 | 18,2552288 | 132 | GQKLLNGEF (SEQ ID N.138) | HLA-B*15:01 | 2795 | SB | NA | no |
| R6EJ07 | 18,2552288 | 296 | SVRGRLGAF (SEQ ID N.8) | HLA-B* 15:01 | 6644 | SB | 66.44 | no |
| R6EJ07 | 18,2552288 | 296 | SVRGRLGAF (SEQ ID N.8) | HLA-B*15:01 | 6644 | SB | 72.58 | no |
| R6EJ07 | 18,2552288 | 217 | MRLDVSEAK (SEQ ID N.139) | HLA-B*27:05 | 6843 | SB | NA | no |
| R6EJ07 | 18,2552288 | 128 | TEFNGQKLL (SEQ ID N.7) | HLA-B*40:01 | 2494 | SB | 24.94 | no |
| R6EJ07 | 18,2552288 | 128 | TEFNGQKLL (SEQ ID N.7) | HLA-B*40:01 | 2494 | SB | 24.94 | no |
| R6VQ94 | 18,1660438 | 93 | ILVMVTVTV (SEQ ID N. 140) | HLA-A*02:01 | 2911 | SB | NA | no |
| R6VQ94 | 18,1660438 | 112 | LQGPFVINV (SEQ ID N.141) | HLA-A*02:01 | 3534 | SB | NA | no |
| R6VQ94 | 18,1660438 | 108 | MTVNLQGPF (SEQ ID N. 42) | HLA-A*26:01 | 3843 | SB | NA | no |
| R6VQ94 | 18,1660438 | 28 | FPELKHFTM (SEQ ID N.9) | HLA-B*07:02 | 6459 | SB | 41.6 | no |
| R6VQ94 | 18,1660438 | 28 | FPELKHFTM (SEQ ID N.9) | HLA-B*07:02 | 6459 | SB | 41.6 | no |
| R6VQ94 | 18,1660438 | 28 | FPELKHFTM (SEQ ID N.9) | HLA-B*08:01 | 4160 | SB | 41.6 | no |
| R6VQ94 | 18,1660438 | 28 | FPELKHFTM (SEQ ID N.9) | HLA-B*08:01 | 4160 | SB | 41.6 | no |
| R6VY20 | 18,6173584 | 322 | TSENMTSAY (SEQ ID N.83) | HLA-A*01:01 | 1807 | SB | NA | no |
| R6VY20 | 18,6173584 | 157 | STDVPVKEY (SEQ ID N.84) | HLA-A*01:01 | 5591 | SB | NA | no |
| R6VY20 | 18,6173584 | 79 | AMTEISEML (SEQ ID N.85) | HLA-A*02:01 | 1972 | SB | NA | no |
| R6VY20 | 18,6173584 | 134 | KLLNGEYDL (SEQ ID N.86) | HLA-A*02:01 | 3434 | SB | NA | no |
| R6VY20 | 18,6173584 | 258 | VLEVNIPAV (SEQ ID N.87) | HLA-A*02:01 | 4757 | SB | NA | no |
| R6VY20 | 18,6173584 | 51 | RMNAQIEGL (SEQ ID N.88) | HLA-A*02:01 | 8596 | SB | NA | no |
| R6VY20 | 18,6173584 | 166 | TIKSIPLTK (SEQ ID N.89) | HLA-A*03:01 | 4969 | SB | NA | no |
| R6VY20 | 18,6173584 | 337 | DMAEEMTSY (SEQ ID N.90) | HLA-A*26:01 | 5239 | SB | NA | no |
| R6VY20 | 18,6173584 | 365 | RPSQVLQLL (SEQ ID N.91) | HLA-B*07:02 | 1805 | SB | NA | no |
| R6VY20 | 18,6173584 | 300 | SVRGRLGAF (SEQ ID N.8) | HLA-B*07:02 | 7258 | SB | 66.44 | no |
| R6VY20 | 18,6173584 | 300 | SVRGRLGAF (SEQ ID N.8) | HLA-B*07:02 | 7258 | SB | 72.58 | no |
| R6VY20 | 18,6173584 | 85 | EMLQRINEL (SEQ ID N.92) | HLA-B*08:01 | 2754 | SB | NA | no |
| R6VY20 | 18,6173584 | 350 | ILAQAGTSM (SEQ ID N.5) | HLA-B*15:01 | 2443 | SB | 24.43 | no |
| R6VY20 | 18,6173584 | 350 | ILAQAGTSM (SEQ ID N.5) | HLA-B*15:01 | 2443 | SB | 24.43 | no |
| R6VY20 | 18,6173584 | 350 | ILAQAGTSM (SEQ ID N.5) | HLA-B*15:01 | 2443 | SB | 24.43 | no |
| R6VY20 | 18,6173584 | 132 | GQKLLNGEY (SEQ ID N.93) | HLA-B*15:01 | 2444 | SB | NA | no |
| R6VY20 | 18,6173584 | 300 | SVRGRLGAF (SEQ ID N.8) | HLA-B*15:01 | 6644 | SB | 66.44 | no |
| R6VY20 | 18,6173584 | 300 | SVRGRLGAF (SEQ ID N.8) | HLA-B*15:01 | 6644 | SB | 72.58 | no |
| R6VY20 | 18,6173584 | 217 | MRLDVSGAL (SEQ ID N.94) | HLA-B*27:05 | 7684 | SB | NA | no |
| R6VY20 | 18,6173584 | 217 | MRLDVSGAL (SEQ ID N.94) | HLA-B*39:01 | 1697 | SB | NA | no |
| R6VY20 | 18,6173584 | 211 | GENGFEMRL (SEQ ID N.95) | HLA-B*40:01 | 1876 | SB | NA | no |
| R6VY20 | 18,6173584 | 128 | TEFNGQKLL (SEQ ID N.7) | HLA-B*40:01 | 2494 | SB | 24.94 | no |
| R6VY20 | 18,6173584 | 128 | TEFNGQKLL (SEQ ID N.7) | HLA-B*40:01 | 2494 | SB | 24.94 | no |
| A0A0M6WP36 | 19,5804199 | 4 | LTRKKGEAL (SEQ ID N. 143) | HLA-B*08:01 | 8039 | SB | NA | no |

**Table 7. List of peptides used in the FLA-G and FLA-R mixtures.**

| **Gene name** | **Peptide** | **HLA** | **Affinity** | **FLA-R/G** |
|---|---|---|---|---|
| tr_A0A0M6WD36_A | KMSYEDIEL (SEQ ID N.1) | HLA-A*02:01 | 71,69 | **FLA-G** |
| tr_A0A0M6WMV3_A | MPKDGAAFI (SEQ ID N.2) | HLA-B*07:02 | 93,13 | **FLA-G** |
| tr_A0A0M6WYS8_A | ALNETSAIL (SEQ ID N.3) | HLA-A*02:01 | 67,47 | **FLA-R** |
| tr_A0A351R0D5_A | ILAQAGQSM (SEQ ID N.4) | HLA-B*15:01 | 32,57 | **FLA-R** |
| tr_R6EJ07_R6EJ0 | ILAQAGTSM (SEQ ID N.5) | HLA-B*15:01 | 24,43 | **FLA-R** |
| tr_R6VY20_R6VY2 | ILAQAGTSM (SEQ ID N.5) | HLA-B*15:01 | 24,43 | |
| tr_A0A396AI63_A | NEFNADLLM (SEQ ID N.6) | HLA-B*40:01 | 55,77 | **FLA-R** |
| tr_R6EJ07_R6EJ0 | TEFNGQKLL (SEQ ID N.7) | HLA-B*40:01 | 24,94 | **FLA-R** |
| tr_R6VY20_R6VY2 | TEFNGQKLL (SEQ ID N.7) | HLA-B*40:01 | 24,94 | |
| tr_R6EJ07_R6EJ0/ tr_R6VY20_R6VY2 | SVRGRLGAF (SEQ ID N.8) | HLA-B*15:01 | 66,44 | **FLA-R** |
| tr_R6EJ07_R6EJ0/ tr_R6VY20_R6VY2 | SVRGRLGAF (SEQ ID N.8) | HLA-B*07:02 | 72,58 | **FLA-R** |
| tr_R6VQ94_R6VQ9 | FPELKHFTM (SEQ ID N.9) | HLA-B*08:01 | 41,60 | **FLA-R** |
| tr_R6VQ94_R6VQ9 | FPELKHFTM (SEQ ID N.9) | HLA-B*08:01 | 41,60 | **FLA-R** |
| tr_A0A351R0D5_A | ILAQAGQSM (SEQ ID N.4) | HLA-B*15:01 | 32,57 | **FLA-R** |
| tr_R6EJ07_R6EJ0/ tr_R6VY20_R6VY2 | ILAQAGTSM (SEQ ID N.5) | HLA-B*15:01 | 24,43 | |
| A0A0M6WP36 | GLDALNNLL (SEQ IDN.10) | HLA-A*02:01 | 51,79 | **FLA-G** |
| A0A0M6WP36 | GLDALNNLL (SEQ IDN.10) | HLA-A*02:01 | 51,79 | **FLA-G** |

### References

1. Luke, J. J., Flaherty, K. T., Ribas, A. & Long, G. V. Targeted agents and immunotherapies: optimizing outcomes in melanoma. Nature Reviews Clinical Oncology 2017 14:8 14, 463-482 (2017).
2. Robert, C. et al. Nivolumab in Previously Untreated Melanoma without BRAF Mutation . New England Journal of Medicine 372, 320-330 (2015).
3. Gopalakrishnan, V. et al. Gut microbiome modulates response to anti-PD-1 immunotherapy in melanoma patients. Science (1979) 359, 97-103 (2018).
4. Matson, V. et al. The commensal microbiome is associated with anti-PD-1 efficacy in metastatic melanoma patients. Science (1979) 359, 104-108 (2018).
5. Routy, B. et al. Gut microbiome influences efficacy of PD-1-based immunotherapy against epithelial tumors. Science (1979) 359, 91-97 (2018).
6. Frankel, A. E. et al. Metagenomic Shotgun Sequencing and Unbiased Metabolomic Profiling Identify Specific Human Gut Microbiota and Metabolites Associated with Immune Checkpoint Therapy Efficacy in Melanoma Patients. Neoplasia 19, 848-855 (2017).
7. McCulloch, J. A. et al. Intestinal microbiota signatures of clinical response and immune-related adverse events in melanoma patients treated with anti-PD-1. Nature Medicine 2022 28:3 28, 545-556 (2022).
8. Baruch, E. N. et al. Fecal microbiota transplant promotes response in immunotherapy-refractory melanoma patients. Science (1979) 371, 602-609 (2021).
9. Routy, B. et al. Fecal microbiota transplantation plus anti-PD-1 immunotherapy in advanced melanoma: a phase I trial. Nature Medicine 2023 29:8 29, 2121-2132 (2023).
10. Davar, D. et al. Fecal microbiota transplant overcomes resistance to anti-PD-1 therapy in melanoma patients. Science (1979) 371, 595-602 (2021).
11. Lee, K. A. et al. Cross-cohort gut microbiome associations with immune checkpoint inhibitor response in advanced melanoma. Nature Medicine 2022 28:3 28, 535-544 (2022).
12. Le, D. T. et al. Mismatch repair deficiency predicts response of solid tumors to PD-1 blockade. Science (1979) 357, 409-413 (2017).
13. Mandal, S. et al. Analysis of composition of microbiomes: a novel method for studying microbial composition. Microb Ecol Health Dis 26, (2015).
14. Cercek, A. et al. PD-1 Blockade in Mismatch Repair-Deficient, Locally Advanced Rectal Cancer. N Engl J Med 386, 2363-2376 (2022).
15. Diehl, A. et al. Relationships between lymphocyte counts and treatment-related toxicities and clinical responses in patients with solid tumors treated with PD-1 checkpoint inhibitors. Oncotarget 8, 114268-114280 (2017).
16. Lee, Y. J. et al. Peripheral lymphocyte count as a surrogate marker of immune checkpoint inhibitor therapy outcomes in patients with non-small-cell lung cancer. Sci Rep 12, (2022).
17. Bauman, J. et al. 798 Safety, tolerability, and immunogenicity of Mrna-4157 in combination with pembrolizumab in subjects with unresectable solid tumors (KEYNOTE-603): an update. J Immunother Cancer 8, A477.1-A477 (2020).
18. Halfvarson, J. et al. Dynamics of the human gut microbiome in inflammatory bowel disease. Nature Microbiology 2017 2:5 2, 1-7 (2017).
19. Callahan, B. J. et al. DADA2: High resolution sample inference from Illumina amplicon data. Nat Methods 13, 581-583 (2016).
20. Bolyen, E. et al. Reproducible, interactive, scalable and extensible microbiome data science using QIIME 2. Nat Biotechnol 37, 852-857 (2019).
21. Janssen, S. et al. Phylogenetic Placement of Exact Amplicon Sequences Improves Associations with Clinical Information. MSystems 3, (2018).
22. Quast, C. et al. The SILVA ribosomal RNA gene database project: improved data processing and web-based tools. Nucleic Acids Res 41, D590-D596 (2013).
23. Bokulich, N.A., Kaehler, B.D., Rideout, J.R. et al. Optimizing taxonomic classification of marker-gene amplicon sequences with QIIME 2's q2-feature-classifier plugin. Microbiome 6, 90 (2018). https://doi.ora/10.1186/s40168-018-0470-z
24. phyloseq: An R Package for Reproducible Interactive Analysis and Graphics of Microbiome Census Data | PLOS ONE. https://journals.plos.org/plosone/article?id=10.1371/journal.pone.0061217.
25. Oksanen, J. et al. The vegan Package. (2009).
26. Kembel, S. An introduction to the picante package. R Proj 1-16 (2010).
27. Wilcoxon, F. Individual comparisons by ranking methods. (Springer, 1992).
28. Anderson, M. J. Permutational Multivariate Analysis of Variance (PERMANOVA). In Wiley StatsRef: Statistics Reference Online 1-15 (John Wiley & Sons, Ltd, 2017). Doi:10.1002/9781118445112.stat07841.
29. Rohart, F., Gautier, B., Singh, A. & Lê Cao, K.-A. mixOmics: An R package for 'omics feature selection and multiple data integration. PloS Comput Biol 13, (2017).
30. Johnson, W. E., Li, C. & Rabinovic, A. Adjusting batch effects in microarray expression data using empirical Bayes methods. Biostatistics 8, 118-127 (2007).
31. Bittinger, K. & Bittinger, M. K. Package 'usedist'. (2022).
32. Langmead, B. & Salzberg, S. L. Fast gapped-read alignment with Bowtie 2. Nature methods 9, 357-359 (2012).
33. Beghini, F. et al. Integrating taxonomic, functional, and strain-level profiling of diverse microbial communities with bioBakery 3. Elife 10, e65088 (2021).
34. Suzek, B. E. et al. UniRef clusters: a comprehensive and scalable alternative for improving sequence similarity searches. Bioinformatics 31, 926-932 (2015).
35. Blanco-Miguez, A. et al. Extending and improving metagenomic taxonomic profiling with uncharacterized species using MetaPhlAn 4. Nat Biotechnol 1-12 (2023) doi:10.1038/s41587-023-01688-w.
36. Warnes, G. R., Bolker, B., Lumley, T. & Warnes, M. G. R. Package 'gtools'. R Package version 3, (2015).
37. Fisher, R. A. Statistical methods for research workers. (Springer, 1992).
38. Wickham, H., Francois, R., Henry, L. & Müller, K. dplyr. In useR! Conference (2014).
39. Lin, H., Peddada, S.D. Analysis of microbial compositions: a review of normalization and differential abundance analysis. Npj Biofilms Microbiomes 6, 60 (2020). https://doi.org/10.1038/s41522-020-00160-w
40. Mallick, H. et al. Multivariable association discovery in population-scale meta-omics studies. PloS computational biology 17, e1009442 (2021).
41. Wu, T. et al. clusterProfiler 4.0: A universal enrichment tool for interpreting omics data. The Innovation 2, 100141 (2021).
42. Ragone, C. et al. Molecular mimicry between tumor associated antigens and microbiota-derived epitopes. J Transl Med 20, 316 (2022).
43. Rasmussen, M. et al. Pan-specific prediction of peptide-MHC class I complex stability, a correlate of T cell immunogenicity. The Journal of Immunology 197, 1517-1524 (2016).
44. R Core Team. R: A language and environment for statistical computing. R Foundation for Statistical Computing, Vienna, Austria. http://www. R-project. Org/ (2023).
45. Gu, Z. Complex heatmap visualization. Imeta 1, e43 (2022).
46. RcolorBrewer, S. & Liaw, M. A. Package 'randomforest'. University of California, Berkeley: Berkeley, CA, USA (2018).
47. Kassambara, A. rstatix: Pipe-friendly framework for basic statistical tests. (2020).
48. Wickham H (2016). Ggplot2: Elegant Graphics for Data Analysis. Springer-Verlag New York. ISBN 978-3-319-24277-4, https://ggplot2.tidyverse.org.
49. Aldahmani, S., Zoubeidi, T. & Aldahmani, M. S. Package 'GGRidge'. (2021).
50. Kassambara, A. & Kassambara, M. A. Package 'ggpubr'. Rpackage version 0.1 6, (2020).
51. Auguie, B., Antonov, A. & Auguie, M. B. Package 'gridExtra'. Miscellaneous Functions for "Grid" Graphics (2017).
52. Choi, Y. et al. Immune checkpoint blockade induces gut microbiota translocation that augments extraintestinal antitumor immunity. Sci Immunol 8, (2023).
53. Andrews, M. C., Reuben, A., Gopalakrishnan, V. & Wargo, J. A. Concepts Collide: Genomic, immune, and microbial influences on the tumor microenvironment and response to cancer therapy. Front Immunol 9, 355224 (2018).
54. Capone, M. et al. Baseline neutrophil-to-lymphocyte ratio (NLR) and derived NLR could predict overall survival in patients with advanced melanoma treated with nivolumab. J Immunother Cancer 6, 1-7 (2018).
55. Bender, M. J. et al. Dietary tryptophan metabolite released by intratumoral Lactobacillus reuteri facilitates immune checkpoint inhibitor treatment. Cell 186, 1846-1862.e26 (2023).
56. Davar, D. & Zarour, H. M. Facts and Hopes for Gut Microbiota Interventions in Cancer Immunotherapy. Clinical Cancer Research 28, 4370-4384 (2022).
57. Hajam, I. A., Dar, P. A., Shahnawaz, I., Jaume, J. C. & Lee, J. H. Bacterial flagellin-a potent immunomodulatory agent. Experimental & Molecular Medicine 2017 49:9 49, e373-e373 (2017).
58. Lauté-Caly, D. L. et al. The flagellin of candidate live biotherapeutic Enterococcus gallinarum MRx0518 is a potent immunostimulant. Sci Rep 9, (2019).
59. Zheng, J. H. et al. Two-step enhanced cancer immunotherapy with engineered Salmonella typhimurium secreting heterologous flagellin. Sci TranslMed 9, (2017).
60. Clasen, S. J. et al. Silent recognition of flagellins from human gut commensal bacteria by Toll-like receptor 5. Sci Immunol 8, (2023).
61. Montalban-Arques, A. et al. Commensal Clostridiales strains mediate effective anti-cancer immune response against solid tumors. Cell Host Microbe 29, 1573-1588.e7 (2021).
62. Ragone, C. et al. Molecular mimicry between tumor associated antigens and microbiota-derived epitopes. J TranslMed 20, 1-18 (2022).
63. Lezcano, C., Jungbluth, A. A., Nehal, K. S., Hollmann, T. J. & Busam, K. J. PRAME Expression in Melanocytic Tumors. American Journal of Surgical Pathology 42, 1456-1465 (2018).
64. Kawakami, Y. et al. Cloning of the gene coding for a shared human melanoma antigen recognized by autologous T cells infiltrating into tumor. Proc Natl Acad Sci U S A 91, 3515-3519 (1994).
65. Coulie, P. G. et al. A new gene coding for a differentiation antigen recognized by autologous cytolytic T lymphocytes on HLA-A2 melanomas. Journal of Experimental Medicine 180, 35-42 (1994).
66. Lin, S. et al. Secernin-1 Contributes to Colon Cancer Progression through Enhancing Matrix Metalloproteinase-2/9 Exocytosis. Dis Markers 2015, (2015).
67. Schnaeker, E. M. et al. Microtubule-Dependent Matrix Metalloproteinase-2/Matrix Metalloproteinase-9 ExocytosisPrerequisite in Human Melanoma Cell Invasion. Cancer Res 64, 8924-8931 (2004).
68. Militaru, I. V., Rus, A. A., Munteanu, C. V. A., Manica, G. & Petrescu, S. M. New panel of biomarkers to discriminate between amelanotic and melanotic metastatic melanoma. Front Oncol 12, 1061832 (2023).
69. Spencer, C. N. et al. Dietary fiber and probiotics influence the gut microbiome and melanoma immunotherapy response. Science 374, 1632-1640 (2021).
70. Simpson, R. C. et al. Diet-driven microbial ecology underpins associations between cancer immunotherapy outcomes and the gut microbiome. Nature Medicine 2022 28:11 28, 2344-2352 (2022).
71. laniro, G. et al. Variability of strain engraftment and predictability of microbiome composition after fecal microbiota transplantation across different diseases. Nature Medicine 2022 28:9 28, 1913-1923 (2022).
72. Kazemian, N. et al. The trans-kingdom battle between donor and recipient gut microbiome influences fecal microbiota transplantation outcome. Scientific Reports 2020 10:1 10, 1-10 (2020).
73. Trinchieri, G. Interleukin-12 and the regulation of innate resistance and adaptive immunity. Nature Reviews Immunology 2003 3:2 3, 133-146 (2003).
74. Kaiko, G. E. et al. The Colonic Crypt Protects Stem Cells from Microbiota-Derived Metabolites. Cell 165, 1708-1720 (2016).
75. Kelly, C. R. et al. Update on Fecal Microbiota Transplantation 2015: Indications, Methodologies, Mechanisms, and Outlook. Gastroenterology 149, 223-237 (2015).
76. Zitvogel, L., Daillère, R., Roberti, M. P., Routy, B. & Kroemer, G. Anticancer effects of the microbiome and its products. Nature Reviews Microbiology 2017 15:8 15, 465-478 (2017).
77. Rooks, M. G. & Garrett, W. S. Gut microbiota, metabolites and host immunity. Nature Reviews Immunology 2016 16:6 16, 341-352 (2016).
78. He, Y. et al. Gut microbial metabolites facilitate anticancer therapy efficacy by modulating cytotoxic CD8+ T cell immunity. Cell Metab 33, 988-1000.e7 (2021).
79. Luu, M. et al. Regulation of the effector function of CD8+ T cells by gut microbiota-derived metabolite butyrate. Scientific Reports 2018 8:1 8, 1-10 (2018).
80. Luu, M. et al. Microbial short-chain fatty acids modulate CD8+ T cell responses and improve adoptive immunotherapy for cancer. Nature Communications 2021 12:1 12, 1-12 (2021).
81. Coutzac, C. et al. Systemic short chain fatty acids limit antitumor effect of CTLA-4 blockade in hosts with cancer. Nature Communications 2020 11:1 11, 1-13 (2020).
82. Nomura, M. et al. Association of Short-Chain Fatty Acids in the Gut Microbiome With Clinical Response to Treatment With Nivolumab or Pembrolizumab in Patients With Solid Cancer Tumors. JAMA Netw Open 3, e202895-e202895 (2020).
83. Danne, C. & Sokol, H. Butyrate, a new microbiota-dependent player in CD8+ T cells immunity and cancer therapy? Cell Rep Med 2, (2021).
84. Furusawa, Y. et al. Commensal microbe-derived butyrate induces the differentiation of colonic regulatory T cells. Nature 2013 504:7480 504, 446-450 (2013).
85. Chen, Y. E. et al. Engineered skin bacteria induce antitumor T cell responses against melanoma. Science (1979) 380, 203-210 (2023).

## Claims

1. A composition comprising at least one peptide for use for the prevention and/or treatment of a cancer, wherein said at least one peptide is coded by at least one flagellin-related gene selected from the group consisting of A0A0M6WP36, C0FQ31, A0A127SWS0, C0FQ36, A0A396AI63, A0A0M6WMV3, A0A0M6WD36, D4KYR7, R6VY20, A0A351R0D5, A0A0M6WBR8, A0A0M6WYS8, R6EJ07, R6VQ94, G2SZK1, C7G5T8, A5Z847, A0A174K7I7, A0A1Q6SAN9, D4KYS1, D4KW84, R6EJJ6, R6EC52, A0A395V8C5, R6EJJ3, R5SPI8, R5T8V3, D4RW24, R5T617, A0A373K755, R6X2U9, G2T3F2, G2SZJ5, D4KYS2, R6NV68, R5T127, A0A395V4R6, C4Z282, A0A395VCZ3, A0A174BIC3, R5SNW4, A0A395VAJ9, A0A174ZTA0, A0A396ABL6, A0A3E4X9M7, R6NRZ0, R5SHJ2, R5E9Z9, R7C6Q7, A0A3D5WGL2, D7GSU0, D7GSR3, D7GSR2, A0A173WCI3, A0A1C5P5U9, R6EJS1, R5WFA2, R9M386, A0A174N4P2, D7GST1, R6P646, A0A174LQE7, A0A174YXY8, D7GSS4, R7CCH1, D7GSS8, A0A1C5WBB9, A0A1C5KUA4, D4RY26, J5GJA7, R6NM46, A0A1C5RKN2, R7NFE3, A0A174YYK7, R5E067, A2V703, R5DRV5, A0A0K4GI28, A0A174MPU4, A0A376JIF4, R5DRV9, R6NJG6, A0A285PW02, C0FTT4, A0A2X1MZ58, R6VWT3, A0A174DCE4, A0A174I7J5, C4ZAM7, C4Z9R8, A0A2U2EKC6, R6EL64, A0A395UYP9, A0A2U2MF49, R6EHH0, A0A396FMF8, A0A0M6WBY2, A0A174Q4T5, B7ART7, A0A396FGM9, A0A396FKV9, R6ESB9, R5TD65, R6BIK6, C7GHZ7, A0A174AGF2, R5SHJ0, P0AEM4, A0A174YHS0 and A0A3E5AME9, wherein said peptide is at least 9 amino acids long, preferably it is between 9 and 20 amino acids, more preferably it consists of 9 amino acids.

2. The composition for the use according to claim 1 wherein said flagellin-related gene is from a bacteria of Chlostridia class, more preferably of the Lachnospiraceae family, even more preferably of Roseburia genere, in particular Roseburia inulinivorans, and/or wherein said peptide is coded by at least one flagellin-related gene selected from the group consisting of: A0A0M6WP36, C0FQ31, A0A127SWS0, C0FQ36, A0A396AI63, A0A0M6WMV3, A0A0M6WD36, D4KYR7, R6VY20, A0A351R0D5, A0A0M6WBR8, A0A0M6WYS8, R6EJ07 and R6VQ94, preferably selected from the group consisting of A0A0M6WD36, A0A0M6WMV3, A0A0M6WYS8, A0A351R0D5, R6EJ07, R6VY20, A0A396AI63, A0A0M6WP36 and R6VQ94.

3. A composition comprising at least one peptide coded by a gene selected from the group consisting of A0A0M6WP36, A0A127SWS0, C0FQ36, A0A396AI63, A0A0M6WMV3, A0A0M6WD36, D4KYR7, R6VY20, A0A351R0D5, A0A0M6WBR8, A0A0M6WYS8, R6EJ07, and R6VQ94, wherein said peptide is coded by the flagellin-related gene A0A0M6WP36 and is selected from the group consisting of peptides comprising or consisting of the following sequences: GLDALNNLL (SEQ ID N.10) and LTRKKGEAL (SEQ ID N.143) and/or said peptide is coded by the flagellin-related gene A0A127SWS0 and is selected from the group consisting of peptides comprising or consisting of the following sequences: STEKLSSGY (SEQ ID N.11), RMLNVTTSA (SEQ ID N.12), ALTEVHSML (SEQ ID N.13), SMLQRMNEL (SEQ ID N.14), and EVHSMLQRM (SEQ ID N.15), and/or said peptide is coded by the flagellin-related gene C0FQ36 and is selected from the group consisting of peptides comprising or consisting of the following sequences: VLTTVRVPK (SEQ ID N.16), IIINMSNNK (SEQ ID N.17), MKQEDTFVL (SEQ ID N.18), SHIMWLQSM (SEQ ID N.19), FPDMQKFTL (SEQ ID N.20) and KGSKSHIMW (SEQ ID N.21) and/or said peptide is coded by the flagellin-related gene A0A396AI63 and is selected from the group consisting of peptides comprising or consisting of the following sequences: TLMPCCPAV (SEQ ID N.22), YLNPVIEKA (SEQ ID N.23), YLENSFEEL (SEQ ID N.24), WLLDGQKEI (SEQ ID N.25), QAYNPLMRK (SEQ ID N.26), VFPREVHRF (SEQ ID N.27), EMADRMHEF (SEQ ID N.28), LPQKKNLSA (SEQ ID N.29), YIREKLTEL (SEQ ID N.30), NPLMRKFLI (SEQ ID N.31), RMHEFESEF (SEQ ID N.32), VLSETCAVF (SEQ ID N.33), KLCKLVTAY (SEQ ID N.34), FLREHARFY (SEQ ID N.35), LRWLLDGQK (SEQ ID N.36), ARFYIREKL (SEQ ID N.37), YRNYLNPVI (SEQ ID N.38), SEFQAYNPL (SEQ ID N.39), SEQTENPFL (SEQ ID N.40), NEFNADLLM (SEQ ID N.6), REKLTELFL (SEQ ID N.41) and ESLLVQYQW (SEQ ID N.42) and/or said peptide is coded by the flagellin-related gene A0A0M6WMV3 and is selected from the group consisting of peptides comprising or consisting of the following sequences: TSDTKQYTY (SEQ ID N.43), GMLNQTTLL (SEQ ID N.44), YIIAVNQTL (SEQ ID N.45), RMSNQQLTV (SEQ ID N.46), AVKAGNMPK (SEQ ID N.47), KTAEEMSQK (SEQ ID N.48), IYQDIDYII (SEQ ID N.49), IIIDYTAAY (SEQ ID N.50), MPKDGAAFI (SEQ ID N.2), MRVTNNMML (SEQ ID N.51), YRTNCKLTF (SEQ ID N.52), VELKTAEEM (SEQ ID N.53), SAQENLQTW (SEQ ID N.54) and KTGTLSYHY (SEQ ID N.55) and/or said peptide is coded by the flagellin-related gene A0A0M6WD36 and is selected from the group consisting of peptides comprising or consisting of the following sequences: FSDEGKMSY (SEQ ID N.56), KLCDIYSEL (SEQ ID N.57), VLAKAQFAI (SEQ ID N.58), KLDDRIANA (SEQ ID N.59), ISFDEAFHV (SEQ ID N.60), KMSYEDIEL (SEQ ID N.1), II,RVPSYYK (SEQ ID N.61), KTFQCIADK (SEQ ID N.62), SYYKTFQCI (SEQ ID N.63), RYFANGKSF (SEQ ID N.64), YYMGIEGAF (SEQ ID N.65), YFHVRMETF (SEQ ID N.66), VYFTYRYFM (SEQ ID N.67), RVPSYYKTF (SEQ ID N.68), DVREKSLSV (SEQ ID N.69), MILRVPSYY (SEQ ID N.70), ALCEVCTEY (SEQ ID N.71), YRYFMRAWY (SEQ ID N.72), YRTQAVAII, (SEQ ID N.73), VRMETFDEL (SEQ ID N.74), NEIWYEHIL (SEQ ID N.75), YEDIELPDL (SEQ ID N.76), IEGAFGERL (SEQ ID N.77) and KSGDYNEIW (SEQ ID N.78) and/or said peptide is coded by the flagellin-related gene D4KYR7 and is selected from the group consisting of peptides comprising or consisting of the following sequences: RLDSGNYNV (SEQ ID N.79), KLLEKYNAL (SEQ ID N.80), KTRKRISRK (SEQ ID N.81) and DAIMRVEAY (SEQ ID N.82) and/or said peptide is coded by the flagellin-related gene R6VY20 and is selected from the group consisting of peptides comprising or consisting of the following sequences: TSENMTSAY (SEQ ID N.83), STDVPVKEY (SEQ ID N.84), AMTEISEML (SEQ ID N.85), KLLNGEYDL (SEQ ID N.86), VLEVNIPAV (SEQ ID N.87), RMNAQIEGL (SEQ ID N.88), TIKSIPLTK (SEQ ID N.89), DMAEEMTSY (SEQ ID N.90), RPSQVLQLL (SEQ ID N.91), SVRGRLGAF (SEQ ID N.8), EMLQRINEL (SEQ ID N.92), ILAQAGTSM (SEQ ID N.5), GQKLLNGEY (SEQ ID N.93), MRLDVSGAL (SEQ ID N.94), GENGFEMRL (SEQ ID N.95) and TEFNGQKLL (SEQ ID N.7) and/or said peptide is coded by the flagellin-related gene A0A351R0D5 and is selected from the group consisting of peptides comprising or consisting of the following sequences: RIRDTDMAK (SEQ ID N.96), KVSSQRSAL (SEQ ID N.97), ILAQAGQSM (SEQ ID N.4) and VKSSLAFSL (SEQ ID N.98) and/or said peptide is coded by the flagellin-related gene A0A0M6WBR8 and is selected from the group consisting of peptides comprising or consisting of the following sequences: LADFIYQKY (SEQ ID N.99), AVAEII,AMV (SEQ ID N.100), LMSDALIQI (SEQ ID N.101), FVVTALISV (SEQ ID N.102), RMMQDVPKA (SEQ ID N.103), YLAQKIKEA (SEQ ID N.104), GLADFIYQK (SEQ ID N.105), LYQAVAEII, (SEQ ID N.106), LMNSFVDIY (SEQ ID N.107), KSKELTAAF (SEQ ID N.108), RIFSKDSIF (SEQ ID N.109), QQKQLHLIM (SEQ ID N.110), RRMMQDVPK (SEQ ID N.111), GHAQDIFIL (SEQ ID N.112), YEIPLMQAI (SEQ ID N.113), KELTAAFDL (SEQ ID N.114), IEIVENKPL (SEQ ID N.115), GEDYLAQKI (SEQ ID N.116), LIMEWDLQW (SEQ ID N.117), LISVIQVGW (SEQ ID N.118) and KQLHLIMEW (SEQ ID N.119) and/or said peptide is coded by the flagellin-related gene A0A0M6WYS8 and is selected from the group consisting of peptides comprising or consisting of the following sequences: FTGLQASRY (SEQ ID N.120), ALDDAIAMV (SEQ ID N.121), SIFSSTATV (SEQ ID N.122), ALNETSAIL (SEQ ID N.3), KMQAQIDAL (SEQ ID N.123), SLVLTFSGK (SEQ ID N.124), NMSAVITNK (SEQ ID N.125), TIKQPAATK (SEQ ID N.126), ETSAILQRM (SEQ ID N.127), DMAEEMTNY (SEQ ID N.128), LPQNVLSLL (SEQ ID N.129), LQRMRELSV (SEQ ID N.130), LQQAGVSVL (SEQ ID N.131), SRYGSSASL (SEQ ID N.132), VELSAGTAI (SEQ ID N.133), TEYNSKSLL(SEQ ID N.134) and SSASLVLTF (SEQ ID N.135) and/or said peptide is coded by the flagellin-related gene R6EJ07 and is selected from the group consisting of peptides comprising or consisting of the following sequences: TSENMTAAY (SEQ ID N.136), AMTEISEML (SEQ ID N.85), KLLNGEFDL (SEQ ID N.137), VLEVNIPAV (SEQ ID N.87), RMNAQIEGL (SEQ ID N.88), DMAEEMTSY (SEQ ID N.90), RPSQVLQLL (SEQ ID N.91), SVRGRLGAF (SEQ ID N.8), EMLQRINEL (SEQ ID N.92), ILAQAGTSM (SEQ ID N.5), GQKLLNGEF (SEQ ID N.138), MRLDVSEAK (SEQ ID N.139) and TEFNGQKLL (SEQ ID N.7) and/or said peptide is coded by the flagellin-related gene R6VQ94 and is selected from the group consisting of peptides comprising or consisting of the following sequences: ILVMVTVTV (SEQ ID N.140), LQGPFVINV (SEQ ID N.141), MTVNLQGPF (SEQ ID N.142), and FPELKHFTM (SEQ ID N.9), or variants thereof.

4. The composition according to claim 3 wherein said at least one peptide comprises or consists of a sequence selected from the group consisting of:
ALNETSAIL (SEQ ID N.3),
ILAQAGQSM (SEQ ID N.4),
ILAQAGTSM (SEQ ID N.5),
NEFNADLLM (SEQ ID N.6),
TEFNGQKLL (SEQ ID N.7),
SVRGRLGAF (SEQ ID N.8),
FPELKHFTM (SEQ ID N.9),
KMSYEDIEL (SEQ ID N.1),
MPKDGAAFI (SEQ ID N.2) and
GLDALNNLL (SEQ ID N.10),
or variants thereof,
preferably in combination with at least one peptide comprising or consisting of a sequence of any of SEQ ID Ns 1-143.

5. The composition according to claim 3 or 4 wherein it comprises at least two, three, four, five, six, seven, eight, nine or ten peptides selected from the group consisting of peptides comprising or consisting of a sequence of SEQ ID Ns. 1, 2, 3, 4, 5, 6, 7, 8, 9, or 10, preferably said composition comprising a peptide of SEQ ID N.3, a peptide of SEQ ID N.4, a peptide of SEQ ID N.5, a peptide of SEQ ID N.6, a peptide of SEQ ID N.7, a peptide of SEQ ID N.8 and a peptide of SEQ ID N.9 or said composition comprising a peptide of SEQ ID N.1, a peptide of SEQ ID N.2 and a peptide of SEQ ID N.10.

6. A polynucleotide coding for a peptide of any one of claims 3-5 or an expression vector comprising said polynucleotide.

7. A pharmaceutical composition or a vaccine or an immunogenic composition comprising the composition of any one of claims 3-5 or the polynucleotide or the expression vector of claim 6 and a pharmaceutically acceptable vehicle or excipient and optionally an adjuvant, preferably said pharmaceutical composition further comprising an anti-cancer therapeutic agent, preferably an immune checkpoint inhibitor.

8. The composition according to any one of claims 3-5 or the polynucleotide or the expression vector of claim 6 or the pharmaceutical composition, vaccine or immunogenic composition of claim 7 for use as a medicament.

9. The composition according to any one of claims 3-5 or the polynucleotide or the expression vector of claim 6 or the pharmaceutical composition, vaccine or immunogenic composition of claim 7 for use for the prevention and/or treatment of a cancer and/or for use as a vaccine against a cancer.

10. The composition or the polynucleotide or the expression vector or the pharmaceutical composition, vaccine or immunogenic composition for the use according to claim 9, wherein the cancer is **characterized by** increased expression of at least one of the following antigens: MAGE-A1, N-ras, CEA, alpha-actinin-4, NY-ESO-1/LAGE-2, MAGE-A3, N-ras, PAP, Preferentially Expressed Antigen in Melanoma (PRAME), Melanoma Antigen Recognized by T Cells 1 (MART-1/Melan-A), and secerning 1 (SCRN1).

11. The composition or the polynucleotide or the expression vector or the pharmaceutical composition, vaccine or immunogenic composition for the use according to any one of claims 9 or 10 wherein the cancer is selected from the group consisting of melanoma, lung cancers/# bladder cancers, esophageal and head and neck cancers, glioblastomas, sarcomas, colorectal cancers, gastric cancers, skin cancers, cutaneous squamous cell carcinomas, thyroid cancers, undifferentiated pleomorphic sarcoma/myxofibrosarcoma, non-small cell lung cancer, small cell lung cancer, multiple myeloma, cerebral cancers, renal cancers, leukemias, lymphomas, hematopoietic and lymphoid cancers, diffuse large B-cell lymphoma, hepatocellular carcinomas, breast cancers, pancreatic cancers, and gynecology cancers.

12. The composition or the polynucleotide or the expression vector or the pharmaceutical composition, vaccine or immunogenic composition for the use according to any one of claims 8-11, wherein said composition or polynucleotide or vector or pharmaceutical composition, vaccine or immunogenic composition is used in combination with a further anti-cancer therapeutic agent, preferably an immune checkpoint inhibitor#such as an antibody anti-PD-1, an antibody anti-PD-L1 and/or an antibody anti-CTLA-4.

13. The composition of any one of claims 3-5 or the polynucleotide or the expression vector of claim 6, or the pharmaceutical composition, vaccine or immunogenic composition of claim 7, for use for increasing effectiveness of an immune checkpoint inhibitor wherein said composition or polynucleotide or expression vector or vaccine is administered to a subject in need thereof before, simultaneously or after said immune checkpoint inhibitor.

14. The composition or the polynucleotide or the expression vector or the pharmaceutical composition, vaccine or immunogenic composition for the use according to any one of claims 7-13, wherein said composition or polynucleotide or vector or pharmaceutical composition, vaccine or immunogenic composition is used in combination with a further composition comprising at least one peptide comprising or consisting of a sequence of any one of SEQ ID Ns 1-143, or variants thereof, preferably said further composition comprising at least one, two, three, four, five, six, seven, eight, nine or ten peptides selected from the group consisting of peptides comprising or consisting of a sequence of SEQ ID Ns. 1, 2, 3, 4, 5, 6, 7, 8, 9, or 10.

15. A dietary supplement or replacement comprising at least one peptide comprising or consisting of a sequence of any of SEQ ID Ns. 1-143 or variants thereof, preferably at least one peptide comprising or consisting of a sequence of SEQ ID Ns. 1, 2, 3, 4, 5, 6, 7, 8, 9, or 10, preferably for use in the treatment and/or prevention of a cancer and/or for use as a vaccine against a cancer, preferably said cancer being **characterized by** increased expression of at least one of the following antigens: MAGE-A1, N-ras, CEA, alpha-actinin-4, NY-ESO-1/LAGE-2, MAGE-A3, N-ras, PAP, Preferentially Expressed Antigen in Melanoma (PRAME), Melanoma Antigen Recognized by T Cells 1 (MART-1/Melan-A), and secernin 1 (SCRN1), more preferably said cancer being selected from the group consisting of melanoma, lung cancers, bladder cancers, esophageal and head and neck cancers, glioblastoma, and sarcomas, colorectal cancers, gastric cancers, skin cancers, cutaneous squamous cell carcinomas, thyroid cancers, undifferentiated pleomorphic sarcoma/myxofibrosarcoma, non-small cell lung cancer, small cell lung cancer, multiple myeloma, cerebral cancers, renal cancers, leukemias, lymphomas, hematopoietic and lymphoid cancers, diffuse large B-cell lymphoma, hepatocellular carcinomas, breast cancers, pancreatic cancers, and gynecology cancers.
